# EUROPÄISCHE PATENTSCHRIFT

(11) **EP 1 390 356 B1**
(45) Veröffentlichungstag und Bekanntmachung des Hinweises auf die Patenterteilung: **31.05.2006**
(21) Anmeldenummer: 02716830.1
(22) Anmeldetag: 09.03.2002
(51) Int. Cl.: C07D 261/04, C07D 413/10, C07D 413/14, A61K 31/42, A61K 31/44, A61K 31/445, A01N 43/80

(54) **ARYLISOXAZOLIN-DERIVATIVE, VERFAHREN ZU IHRER HERSTELLUNG UND IHRE VERWENDUNG ALS SCHÄDLINGSBEKÄMPFUNGSMITTEL**
ARYLISOXAZOLINE DERIVATIVES, METHOD FOR PRODUCTION AND USE THEREOF AS PESTICIDES
DERIVE D'ARYLISOXAZOLINE, LEUR PROCEDE DE PRODUCTION ET LEUR UTILISATION COMME PESTICIDES

(30) Priorität: 23.03.2001 DE 10114597
(43) Veröffentlichungstag der Anmeldung: 25.02.2004
(73) Patentinhaber: Bayer CropScience GmbH, 65929 Frankfurt/Main (DE)
(72) Erfinder: BRAUN, Ralf, 76857 Ramberg (DE); ORT, Oswald, 61479 Glashütten (DE); HAACK, Karl-Josef, 65527 Niedernhausen (DE); ECKHARDT, Matthias, 88400 Biberach (DE); HEMPEL, Waltraud, 65835 Liederbach (DE); THÖNESSEN, Maria-Theresia, 55262 Heidesheim (DE)
(74) Vertreter: Lutze, Oliver
(86) Internationale Anmeldenummer: PCT/EP2002/002619
(87) Internationale Veröffentlichungsnummer: WO 2002/076956

(56) Entgegenhaltungen:
- EP-A- 0 174 685
- EP-A- 0 611 760
- WO-A-93/24470
- WO-A-95/04726
- WO-A-96/22283

## Beschreibung

Arylisoxazolin-Derivate, Verfahren zu ihrer Herstellung und ihre Verwendung als Schädlingsbekämpfungsmittel

Die Erfindung betrifft Arylisvxazolin-Derivate; Verfahren zu ihrer Herstellung, diese enthaltende Mittel und ihre Verwendung zur Bekämpfung von tierischen Schädlingen, insbesondere Arthropoden, wie Insekten und Acarina, und Helminthen.

Bestimmte 1,3-Oxazoline, 1,3-Thiazoline, Pyrroline und Imidazoline eignen sich auf Grund ihrer biologischen Aktivität zur Bekämpfung von tierischen Schädlingen (siehe z.B. WO-A-93/24470, WO-A-95/04726 und WO-A-96/22283). In der EP-A 0 174 685 und EP-A 0 611 760 sind isoxazolinderivate mit herbizider bzw. fungizider Wirkung beschrieben.

Wegen der vielfältigen Anforderungen an moderne Schädlingsbekämpfungsmittel, beispielsweise was Wirkhöhe, Wirkdauer, Wirkspektrum, Anwendungsspektrum, Toxizität, Kombination mit anderen Wirkstoffen, Kombination mit Formulierungsmitteln oder Synthese angeht, und wegen des möglichen Auftretens von Resistenzen kann die Entwicklung solcher Stoffe jedoch nie als abgeschlossen betrachtet werden, und es besteht beständig ein hoher Bedarf an neuen Verbindungen, die zumindest in Teilaspekten Vorteile gegenüber den bekannten aufweisen.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe sowie weitere nicht explizit genannte Aufgaben, die aus den hierin diskutierten Zusammenhängen ableitbar oder erschließbar sind durch Arylisoxazolin-Derivate der Formel (I), 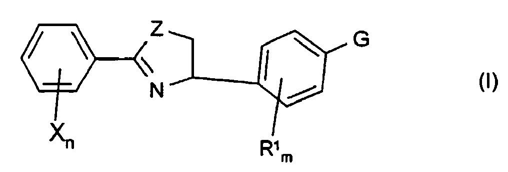 worin die Symbole und Indizes folgende Bedeutungen haben:
- X: ist gleich oder verschieden
a) Halogen, Cyano, Nitro;
b) (C₁-C₄)-Alkyl, (C₁-C₄)-Alkoxy, (C₁-C₄)-Alkylthio, (C₁-C₄)-Alkylsulfinyl, wobei die Reste der Gruppe b gegebenenfalls durch einen oder mehrere, vorzugsweise einen, zwei oder drei, Reste aus der Gruppe Halogen substituiert sind;
- R¹: ist gleich oder verschieden Halogen, (C₁-C₄)Haloalkyl, (C₁-C₄)Alkyl, (C₁-C₄)Alkoxy, (C₁-C₄)Haloalkoxy oder Cyano;
- m: ist 0, 1, 2, 3 oder 4;
- n: ist 1, 2, 3, 4 oder 5;
- Z: ist Sauerstoff, Schwefel, CH₂ oder NR²;
- R²: ist CN, (C₁-C₄)-Alkoxy-(C₁-C₄)-alkyl, CHO, (C₁-C₆)-Alkylcarbonyl, (C₁-C₆)-Alkoxycarbonyl oder(CW)NR³R⁴;
- R³, R⁴: sind gleich oder verschieden H, (C₁-C₆)-Alkyl;
- W: ist O oder S;
- G: ist 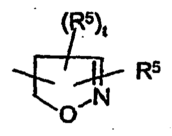
- t: ist 0,1,2 oder 3;
- R⁵: ist gleich oder verschieden
a) Halogen, CN, NO₂
b) eine geradkettige oder verzweigte Alkylgruppe mit 1 bis 12 C-Atomen, wobei eine oder mehrere (CH₂)-Gruppen gegebenenfalls durch -O-, -S(O)_{-0,1,2,} -NH-, -NR⁶-, -CO-, -CS-, -CH=CH-, -C≡C-, Aryl-diyl, Heterocyclyl-diyl, (C₃-C₈)-Cycloalkandiyl oder (C₃-C₈)-Cycloalkendiyl ersetzt sind, mit der Maßgabe, daß Chalkogene nicht benachbart sein dürfen, wobei einzelne Wasserstoffatome gegebenenfalls durch Halogen ersetzt sind;
c) im Falle von zwei α-ständigen Resten R⁵ auch (=Y) wobei Y (=O), (=S), (=NOR⁶) oder (=CR₂⁶) bedeutet;
mit der Maßgabe, daß der oder die Reste R⁵ zusammen nicht mehr als ein fünf- oder mehrgliedriges Ringsystem enthalten;
- R⁶: ist (C₁-C₄)-Alkyl, Phenyl oder Benzyl;
- Aryl: ist ein carbocyclischer aromatischer Rest mit 6 bis 14 C-Atomen ;
- Heterocyclyl: ist ein heteroaromatisches oder heteroaliphatisches Ringsystem, wobei unter "heteroaromatisches Ringsystem" ein Arylrest, worin mindestens eine CH-Gruppe durch N ersetzt ist und/oder mindestens zwei benachbarte CH-Gruppen durch S, NH oder O ersetzt sind, zu verstehen ist und unter "heteroaliphatisches Ringsystem" eine (C₃-C₈)-Cycloalkylrest, in dem mindestens eine Kohlenstoff-Einheit durch O, S oder eine Gruppe NR¹¹ ersetzt ist und R¹¹ Wasserstoff, (C₁-C₄)-Alkyl, (C₁-C₄)-Alkoxy oder Aryl bedeutet,
wobei die cyclischen Reste in der Bedeutung von R⁵, R⁶ gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino und (C₁-C₄)-Alkanoyl substituiert sind;
deren eine Stereoisomere an Doppelbindungen, Enantiomers oder Diasteromere sowie deren Gemische, N-Oxide und für den Gebrauch als Schädlingsbekämpfungsmittel geeignete Salze.

Überraschenderweise besitzen Verbindungen der Formel (I) eine bessere Akarizid- und Insektizidwirkung hinsichtlich des Wirkungsspektrums und der Wirkstärke als bekannte 1,3-Oxazolin-, 1,3-Thiazolin-, Pyrrolin-oderlmidazblin-Derivate.

Bevorzugt haben die Symbole und Indizes in der Formel (I) folgende Bedeutungen:
- X: ist bevorzugt Halogen, insbesondere Cl, Br oder F, Cyano, Nitro, (C₁-C₄)-Alkyl, (C₁-C₃)-Haloalkyl, (C₁-C₄)-Alkoxy oder (C₁-C₃)-Haloalkoxy.
- X: ist besonders bevorzugt Halogen, insbesondere Cl, Br oder F, (C₁-C₄)-Alkyl, (C₁-C₃)-Haloalkyl, (C₁-C₄)-Alkoxy oder (C₁-C₃)-Haloalkoxy.
- m: ist bevorzugt 0 oder 1.
- n: ist bevorzugt 1, 2 oder 3.
- Z: ist bevorzugt Sauerstoff oder CH₂.
- R¹: ist bevorzugt H, Halogen, (C₁-C₄)-Haloalkyl, (C₁-C₄)-Alkyl, (C₁-C₄)-Alkoxy, (C₁₋C₄)-Haloalkoxy.
- G: ist bevorzugt 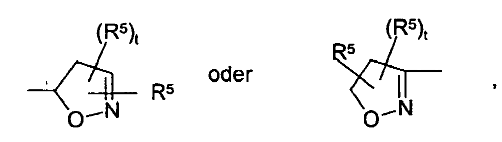 ganz besonders bevorzugt 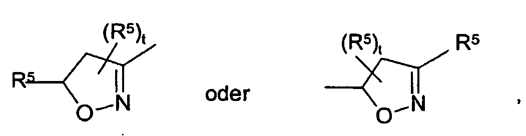 insbesondere 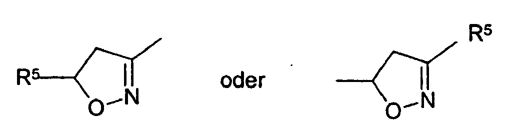
- t: ist vorzugsweise 0 oder 1.

Als Substituenten an dem Isoxazolinrest haben die Reste R⁵ bevorzugt folgende Bedeutungen:
- R⁵: ist gleich oder verschieden D-R⁷ oder zwei Reste R⁵ bilden zusammen mit den Atomen, an die sie gebunden sind, ein drei- bis achtgliedriges, gesättigtes oder ungesättigtes, gegebenenfalls mit einem oder mehreren Resten R⁷ substituiertes Ringsystem, das gegebenenfalls auch weitere Heteroatome, vorzugsweise O, N, S, SO und/oder SO₂, enthält;
- D: ist eine direkte Bindung oder (C₁-C₆)-Alkandiyl, gegebenenfalls substituiert durch ein oder mehrere Halogenatome;
- R⁷: ist gleich oder verschieden R⁸, R⁹, -C(W)R⁸, -C(=NOR⁸)R⁸, -C(=NNR⁸₂)R⁸, -C(=W)OR⁸, -C(=W)NR⁸₂, -OC(=W)R⁸, -OC(=W)OR⁸, -NR⁸C(=W)R⁸, -N[C(=W)R⁸]₂, -NR⁸C(=W)OR8⁸, -C(=W)NR⁸-NR⁸₂, -C(=W)NR⁸-N, R⁸[C(=W)R⁸], -NR⁸-C(=W)NR⁸₂, -NR⁸-NR⁸C(=W)R⁸, -NR⁸-N[C(=W)R⁸]₂, -N[(C=W)R⁸]-NR⁸₂, -NR⁸-N[(C=W)WR⁸], -NR⁸[(C=W)NR⁸₂], -NR⁸(C=NR⁸)R⁸, -NR⁸(C=NR⁸)NR⁸₂, -O-NR⁸₂, -O-NR⁸(C=W)R⁸, -SO₂NR⁸₂, -NR⁸SO₂R⁸, -SO₂OR⁸, -OSO₂R⁸, -OR⁸, -NR⁸₂, -SR⁸, -SiR⁸₃, -PR⁸₂, -P(=W)R⁸₂, -SOR⁸, -SO₂R⁸, -PW₂R⁸₂, -PW₃R⁸₂ oder zwei Reste R⁷ sind zusammen (=Y), (=N-R⁸), (= CR₂⁸) oder (= CHR⁸);
- W: ist O oder S;
- R⁸: ist gleich oder verschieden H, (C₁-C₆)-Alkyl, (C₂-C₆)-Alkenyl, (C₂-C₆)-Alkinyl, (C₃ C₈)-Cycloalkyl, (C₄-C₈)-Cycloalkenyl, (C₃-C₈)-Cycloalkyl-(C₁-C₄)-Alkyl, (C₄ C₈)-Cycloalkenyl-(C₁-C₄)-Alkyl, (C₃-C₈)-Cycloalkyl-( C₂-C₄)-Alkenyl, (C₄-C₈)-Cycloalkenyl-(C₂ C₄)-Alkenyl, (C₁-C₆)-Alkyl-(C₃-C₈)-Cycloalkyl, (C₂-C₆)-Alkenyl-(C₃-C₈)-Cycloalkyl, (C₂-C₆)-Alkinyl-(C₃-C₈)-Cycloalkyl, (C₁-C₆)-Alkyl-(C₄-C₈)-Cycloalkenyl, (C₂-C₆)-Alkenyl-(C₄-C₈)-Cycloalkenyl, Aryl, Heterocyclyl; wobei die genannten Reste gegebenenfalls mit einem oder mehreren Resten R⁹ substituiert sind und gegebenenfalls zwei Reste R⁸ zusammen ein Ringsystem bilden;
- R⁹: ist gleich oder verschieden Halogen, Cyano, Nitro, Hydroxy, Thio, Amino, (C₁₋C₆)-Alkanoyl, (C₂-C₆)-Haloalkanoyl, (C₁-C₆)-Alkoxy, (C₃-C₆)-Alkenyloxy, (C₃₋C₆)-Alkinyloxy, (C₁-C₆)-Haloalkyloxy, (C₃-C₆)-Haloalkenyloxy, (C₃-C₆)-Haloalkinyloxy, (C₃-C₈)-Cycloalkoxy, (C₄-C₈)-Cycloalkenyloxy, (C₃-C₈)-Halocycloalkoxy, (C₄-C₈)-Halocycloalkenyloxy, (C₃-C₈)-Cycloalkyl-(C₁-C₄)-Alkoxy, (C₄-C₈)-Cycloalkenyl-(C₁-C₄)-Alkoxy, (C₃-C₈)-Cycloalkyl-(C₂-C₄)-Alkenyloxy, (C₄-C₈)-Cycloalkenyl-(C₁-C₄)-Alkenyloxy, (C₁-C₆)-Alkyl-(C₃-C₈)-Cycloalkoxy, (C₂-C₆)-Alkenyl-(C₃-C₈)-Cycloalkoxy, (C₂-C₆)-Alkinyl-(C₃-C₈)-Cycloalkoxy, (C₁-C₆)-Alkyl-(C₄-C₈)-Cycloalkenyloxy, (C₂-C₆)-Alkenyl-(C₄-C₈)-Cycloalkenyloxy, (C₁-C₄)-Alkoxy-(C₁-C₆)-Alkoxy, (C₁-C₄)-Alkoxy-(C₃-C₆)-Alkenyloxy, Carbamoyl, (C₁-C₆)-Mono- oder Dialkylcarbamoyl, (C₁-C₆)-Mono-oder Dihaloalkylcarbamoyl, (C₃-C₈)-Mono- oder Dicycloalkylcarbamoyl, (C₁-C₆)-Alkoxycarbonyl, (C₃-C₈)-Cycloalkoxycarbonyl, (C₁-C₆)-Alkanoyloxy, (C₃-C₈)-Cycloalkanoyloxy, (C₁-C₆)-Haloalkoxycarbonyl, (C₁-C₆)-Haloalkanoyloxy, (C₁-C₆)-Alkanamido, (C₁-C₆)-Haloalkanamido, C(O)NH(C₁-C₆)-Alkyl, C(O)NH(C₁-C₆)-Haloalkyl, C(O)N[(C₁-C₆)-Alkyl]₂, C(O)N[(C₁-C₆)Haloalkyl]₂, (C₂-C₆)-Alkenamido, (C₃-C₈)-Cycloalkanamido, (C₃-C₈)-Cycloalkyl-(C₁-C₄)-Alkanamido, (C₁-C₆)-Alkylthio, (C₃-C₆)-Alkenylthio, (C₃-C₆)-Alkinylthio, (C₁-C₆)-Haloalkylthio, (C₃-C₆)-Haloalkenylthio, (C₃-C₆)-Haloalkinylthio, (C₃-C₈)-Cycloalkylthio, (C₄-C₈)-Cycloalkenylthio, (C₃ C₈)-Halocycloalkylthio, (C₄-C₈)-Halocycloalkenylthio, (C₃-C₈)-Cycloalkyl-(C₁-C₄)-Alkylthio, (C₄-C₈)-Cycloalkenyl-(C₁-C₄)-Alkylthio, (C₃-C₈)-Cycloalkyl-(C₃-C₄)-Alkenylthio, (C₄-C₈)-Cycloalkenyl-(C₃-C₄)-Alkenylthio, (C₁-C₆)-Alkyl-(C₃-C₈)-Cycloalkylthio, (C₂-C₆)-Alkenyl-(C₃-C₈)-Cycloalkylthio, (C₂-C₆)-Alkinyl-(C₃-C₈)-Cycloalkylthio, (C₁-C₈)-Alkyl-(C₄ C₈)-Cycloalkenylthio, (C₂-C₆)-Alkenyl-(C₄-C₈)-Cycloalkenylthio, (C₁-C₆)-Alkylsulfinyl, (C₃-C₆)-Alkenylsulfinyl, (C₃-C₆)-Alkinylsulfinyl, (C₁-C₆)-Haloalkylsulfinyl, (C₃-C₆)-Haloalkenylsulfinyl, (C₃-C₆)-Haloalkinylsulfinyl, (C₃-C₈)-Cycloalkylsulfinyl, (C₄-C₈)-Cycloalkenylsulfinyl, (C₃-C₈)-Halocycloalkylsulfinyl, (C₄-C₈)-Halocycloalkenylsulfinyl, (C₃-C₈)-Cycloalkyl-(C₁-C₄)-Alkylsulfinyl, (C₄-C₈)-Cycloalkenyl-(C₁-C₄)-Alkylsulfinyl, (C₃-C₈)-Cycloalkyl-(C₃-C₄)-Alkenylsulfinyl, (C₄-C₈)-Cycloalkenyl-(C₃-C₄)-Alkenylsulfinyl, (C₁-C₆)-Alkyl-(C₃-C₈)-Cycloalkylsulfinyl, (C₂-C₆)-Alkenyl-(C₃-C₈)-Cycloalkylsulfinyl, (C₂-C₆)-Alkinyl-(C₃-C₈)-Cycloalkylsulfinyl, (C₁-C₆)-Alkyl-(C₄-C₈)-Cycloalkenylsulfinyl, (C₂-C₆)-Alkenyl-(C₄-C₈)-Cycloalkenylsulfinyl, (C₁-C₆)-Alkylsulfonyl, (C₃-C₆)-Alkenylsulfonyl, (C₃-C₆)-Alkinylsulfonyl, (C₁-C₆)-Haloalkylsulfonyl, (C₃-C₆)-Haloalkenylsulfonyl, (C₃-C₆)-Haloalkinylsulfonyl, (C₃-C₈)-Cycloalkylsulfonyl, (C₄-C₈)-Cycloalkenylsulfonyl, (C₃-C₈)-Halocycloalkylsulfonyl, (C₄-C₈)-Halocycloalkenylsulfonyl, (C₃-C₈)-Cycloalkyl-(C₁-C₄)-Alkylsulfonyl, (C₄-C₈)-Cycloalkenyl-(C₁-C₄)-Alkylsulfonyl, (C₃-C₈)-Cycloalkyl-(C₃-C₄)-Alkenylsulfonyl, (C₄-C₈)-Cycloalkenyl-(C₃-C₄)-Alkenylsulfonyl, (C₁-C₈)-Alkyl-(C₃-C₈)-Cycloalkylsulfonyl, (C₂-C₆)-Alkenyl-(C₃ C₈)-Cycloalkylsulfonyl, (C₂-C₆)-Alkinyl-(C₃-C₈)-Cycloalkylsulfonyl, (C₁-C₆)-Alkyl-(C₄-C₈)-Cycloalkenylsulfonyl, (C₂-C₆)-Alkenyl-(C₄-C₈)-Cycloalkenylsulfonyl, (C₁-C₆)-Dialkylamino, (C₁-C₆)-Alkylamino, (C₃-C₆)- Alkenylamino, (C₃-C₆)-Alkinylamino, (C₁-C₆)-Haloalkylamino, (C₃-C₆)-Haloalkenylamino, (C₃-C₆)-Haloalkinylamino, (C₃-C₈)-Cycloalkylamino, (C₄-C₈)-Cycloalkenylamino, (C₃-C₈)-Halocycloalkamino, (C₄-C₈)-Halocycloalkenylamino, (C₃-C₈)Cycloalkyl-(C₁-C₄)-Alkylamino; (C₄-C₈)-Cycloalkenyl-(C₁-C₄)-Alkylamino, (C₃-C₈)-Cycloalkyl-(C₃-C₄)-Alkenylamino, (C₄ C₈)-Cycloalkenyl-(C₃-C₄)-Alkenylamino, (C₁-C₆)-Alkyl-(C₃-C₈)-Cycloalkylamino, (C₂-C₆)-Alkenyl-(C₃-C₈)-Cycloalkylamino, (C₂-C₆)-Alkinyl-(C₃-C₈)-Cycloalkylamino, (C₁-C₆)-Alkyl-(C₄ C₈)-Cycloalkenylamino, (C₂-C₆)-Alkenyl-(C₄-C₈)-Cycloalkenylamino, (C₁-C₆)-Trialkylsilyl, Aryl, Aryloxy, Arylthio, Arylamino, Aryl-(C₁-C₄)-Alkoxy, Aryl-(C₁-C₆)-Alkanoyl, Aryl-(C₃ C₄)-Alkenyloxy, Aryl-(C₁-C₄)-Alkylthio, Aryl-(C₂-C₄)-Alkenylthio, Aryl-(C₁-C₄)-Alkylamino, Aryl-(C₃-C₄)-Alkenylamino, Aryl-(C₁-C₆)-Dialkylsilyl, Diaryl-(C₁-C₆)-Alkylsilyl, Triarylsilyl und 5- oder 6-gliedriges Heterocyclyl, wobei die cyclischen Reste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino und (C₁-C₄)-Alkanoyl substituiert sind.

Besonders bevorzugt ist
- R⁵: CN, gegebenenfalls substituiertes Phenyl, gegebenenfalls substituiertes Phenoxy, (C₁-C₆)-Alkyl, (C₁-C₆)-Alkenyl, (C₁-C₆)-Haloalkyl, (C₁-C₆)-Haloalkenyl, -(C₁-C₆)-Alkandiyl-Aryl, wobei die Arylgruppe gegebenenfalls substituiert ist und wobei eine -CH₂-Einheit gegebenenfalls durch -C(O)-NR¹⁰-, NR¹⁰-(CO), NR¹⁰ oder O ersetzt ist.
- R¹⁰: ist H, (C₁-C₆)-Alkyl, (C₁-C₆)-Haloalkyl, gegebenenfalls substituiertes Phenyl, gegebenenfalls substituiertes Benzyl.

Besonders bevorzugt für 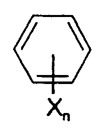 sind die Gruppen

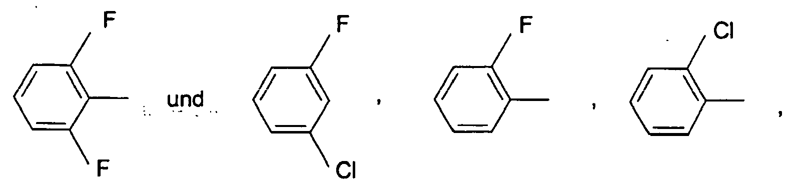

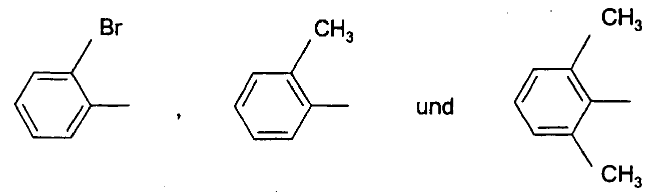

Besonders bevorzugte Gruppen von Verbindungen der Formel (I) sind solche der Formeln (I1) bis (I28):

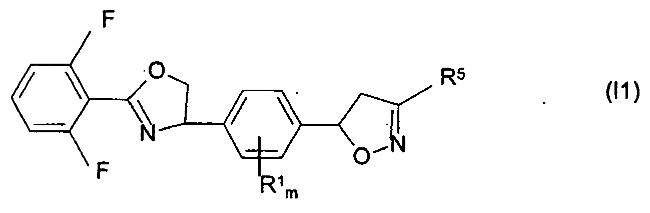

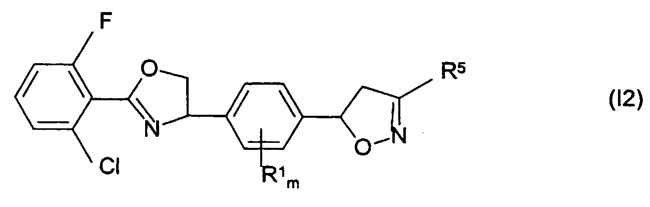

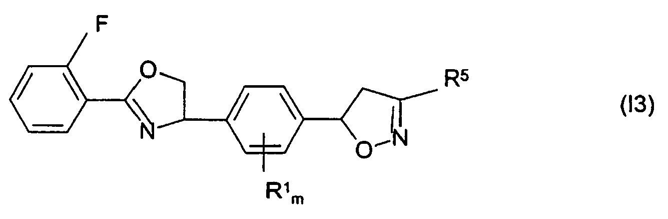

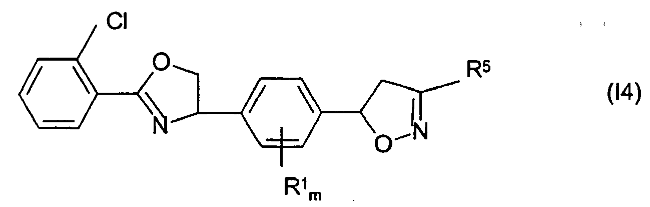

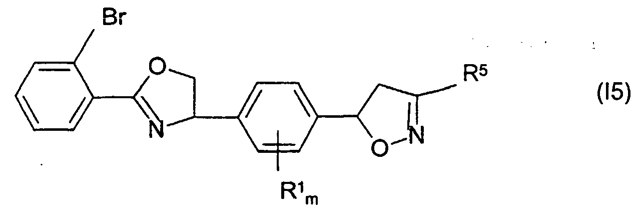

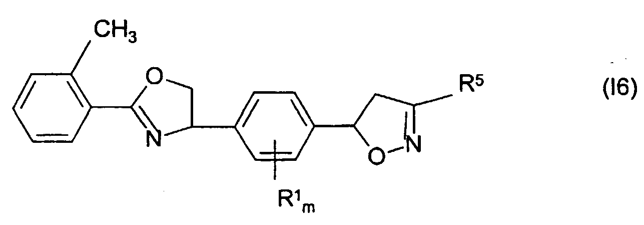

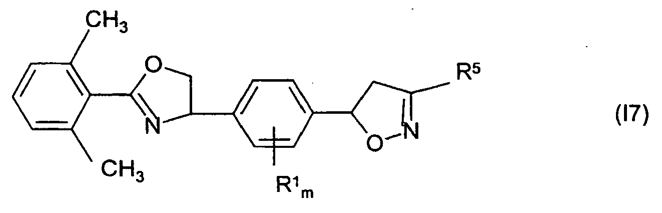

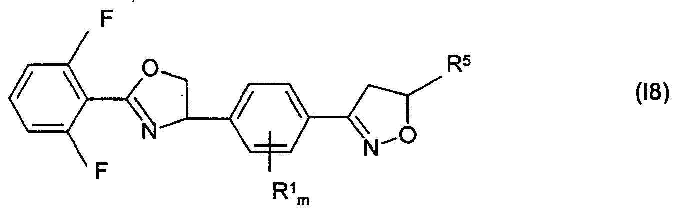

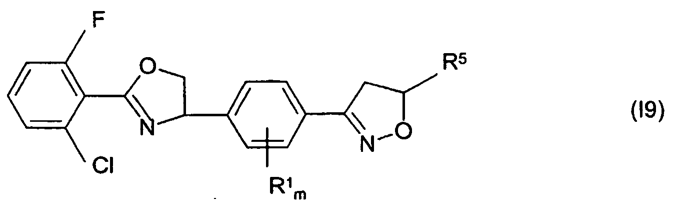

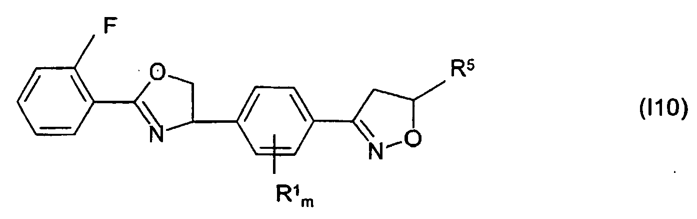

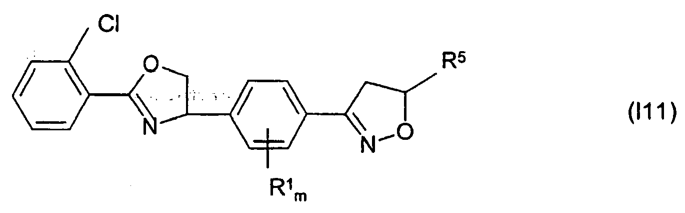

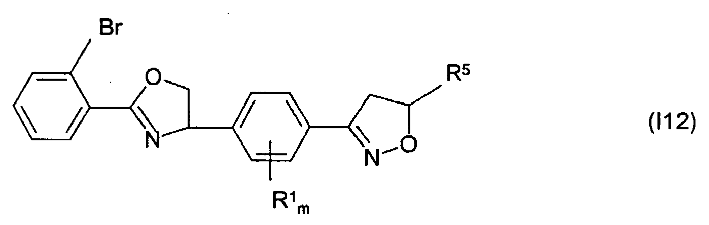

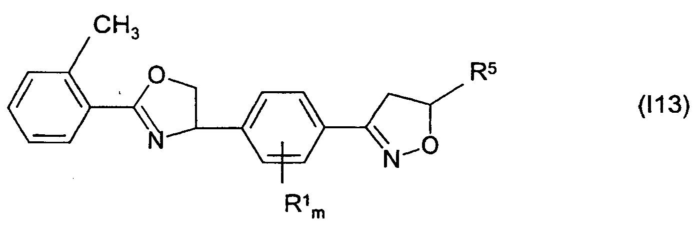

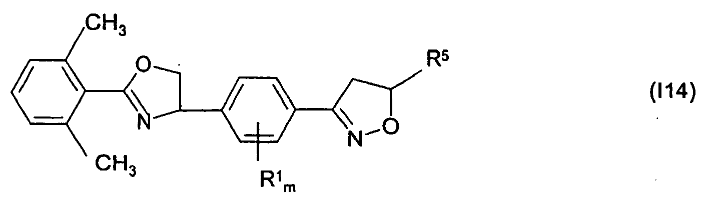

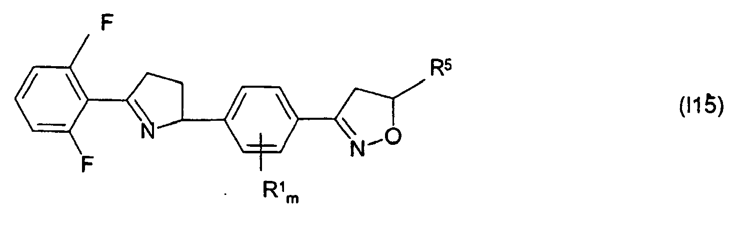

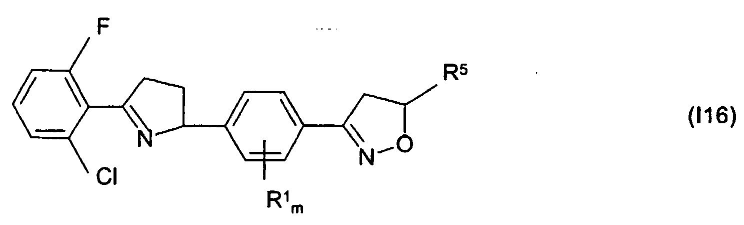

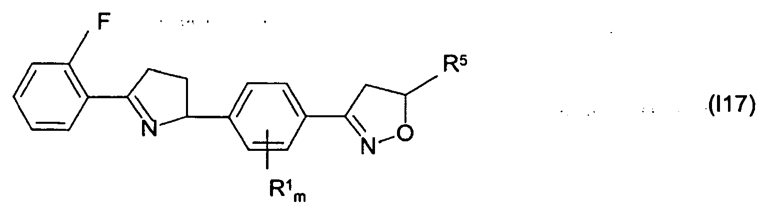

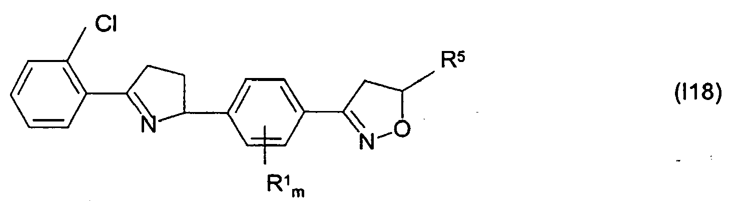

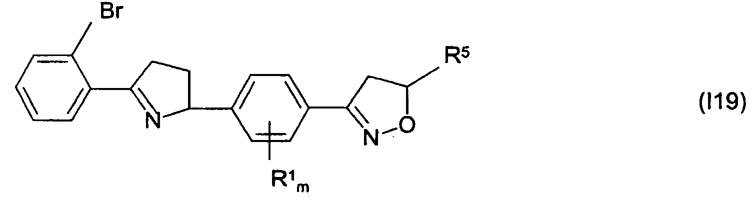

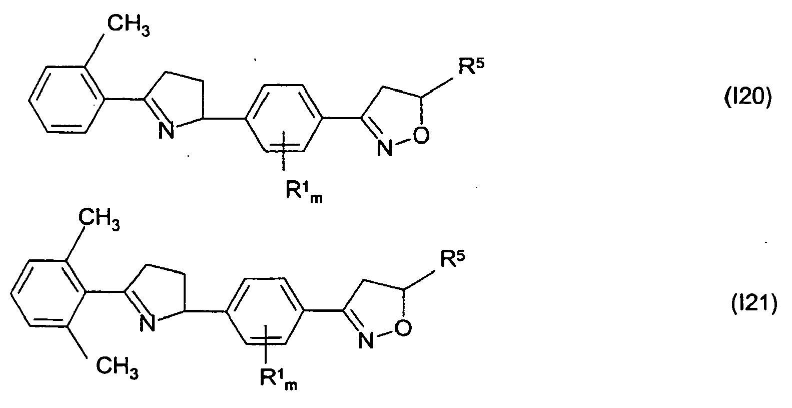

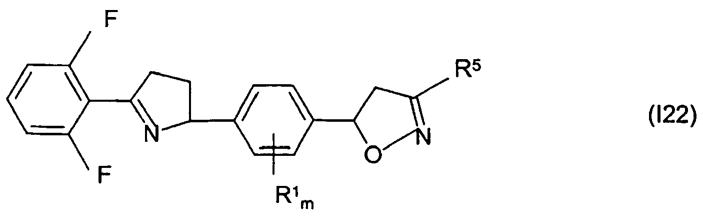

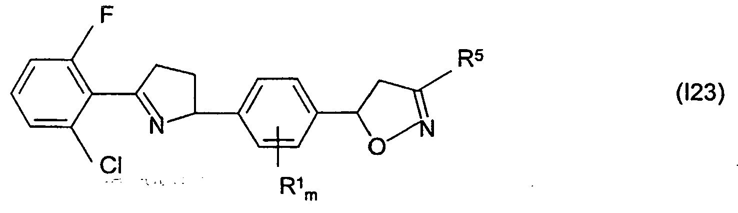

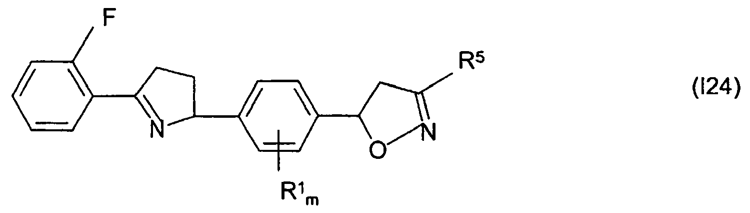

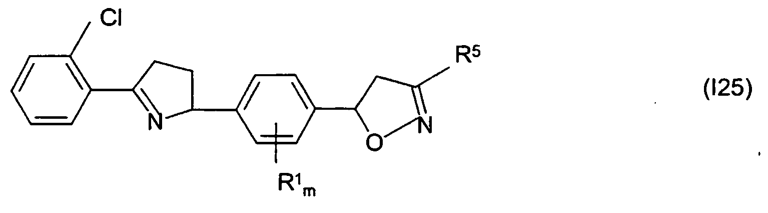

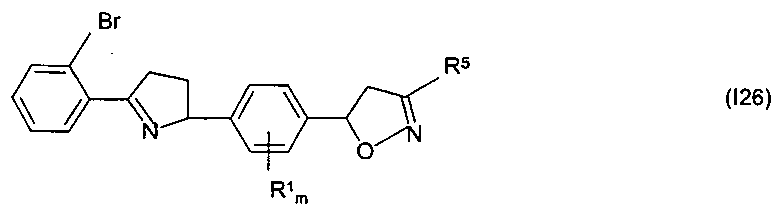

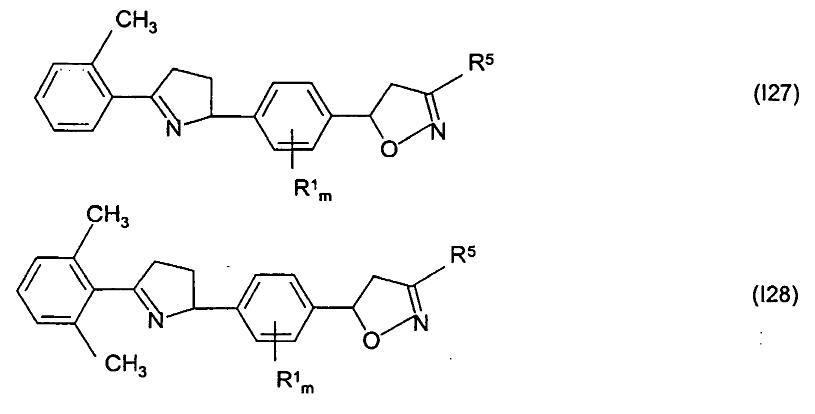

In der obigen Formel ist unter "Halogen" ein Fluor-, Chlor-, Brom- oder lodatom zu verstehen;
unter dem Ausdruck "(C₁-C₄)-Alkyl" ein unverzweigter oder verzweigter Kohlenwasserstoffrest mit 1 bis 4 Kohlenstoffatomen, wie z.B. der Methyl-, Ethyl-, Propyl-, Isopropyl-, 1-Butyl-, 2-Butyl-, 2-Methylpropyl- oder tert.-Butylrest zu verstehen;
unter dem Ausdruck "(C₁-C₆)-Alkyl" die vorgenannten Alkylreste sowie z.B. der Pentyl-, 2-Methylbutyl-, 1,1-Dimethylpropyl- oder der Hexylrest;
unter dem Ausdruck "(C₁-C₈)-Alkandiyl" ein unverzeigter oder verzweigter Alkandiylrest mit 1 bis 6 Kohlenstoffatomen, wie Methylen, Ethan-1,2-diyl, Propan-1,2-diyl, Propan-1,3-diyl, Butan-1,4-diyl, Butan-1,3-diyl oder 2-Methylpropan-1,3-diyl; unter dem Ausdruck "(C₁-C₄)-Halogenalkyl" eine unter dem Ausdruck "(C₁-C₄)-Alkyl" genannte Alkylgruppe, in der ein oder mehrere Wasserstoffatome durch die obengenannten Halogenatome, bevorzugt Chlor oder Fluor, ersetzt sind, wie beispielsweise die Trifluormethylgruppe, die 1-Fluorethylgruppe, die 2,2,2-Trifluorethylgruppe, die Chlormethyl-, Fluormethylgruppe, die Difluormethylgruppe oder die 1,1.2,2-Tetraftuorethytgruppe;
unter dem Ausdruck "(C₃-C₈)-Cycloalkyl" beispielsweise die Cyclopropyl-, Cyclobutyl- oder Cyclopentylgruppe; sowie der Cyclohexyl-, Cycloheptyl- oder Cyclooctyl-Rest; unter dem Ausdruck "(C₃-C₈)-Halogencycloalkyl" eine der oben aufgeführten (C₃-C₈)-Cycloalkylreste, in denen ein oder mehrere, im Falle von Fluor gegebenenfalls auch alle Wasserstoffatome, durch Halogen, bevorzugt Fluor oder Chlor, ersetzt sind, beispielsweise die 2,2-Difluor- oder 2,2-Dichlorcyclopropan-Gruppe oder der Fluorcyclopentan-Rest;
unter dem Ausdruck "(C₂-C₄)-Alkenyl" z.B. die Vinyl-, Allyl-, 2-Methyl-2-propenyl- oder 2-Butenyl-Gruppe;
unter dem Ausdruck "(C₂-C₄)-Halogenalkenyl" eine (C₂-C₄)-Alkenyl-Gruppe, in der die Wasserstoffatome teilweise oder im Falle von Fluor auch vollständig durch Halogen, bevorzugt Fluor oder Chlor ersetzt sind;
unter dem Ausdruck "(C₂-C₄)-Alkinyl" z.B. die Ethinyl-, Propargyl-, 2-Methyl-2-propinyl oder 2-Butinyl-Gruppe;
unter dem Ausdruck "(C₂-C₆)-Alkinyl" z.B. die vorstehend genannten Reste sowie z.B. die 1-Pentinyl, 2-Pentinyl-, 3-Pentinyl-, oder die 4-Pentinyl-Gruppe;
unter dem Ausdruck "Halogenalkinyl" eine Alkinylgruppe in der die Wasserstoffatome teilweise, im Falle von Fluor auch vollständig, durch Halogenatome, bevorzugt Fluor oder Chlor, ersetzt sind;
unter dem Ausdruck "(C₁-C₄)-Alkanoyl-(C₁-C₄)-alkyl" z.B. eine Acetylmethyl-, Propionylmethyl-, 2-Acetylethyl- oder eine Butyrylmethyl-Gruppe;
unter dem Ausdruck "(C₁-C₄)-Alkanoyl" z.B. die Formyl-, Acetyl-, Propionyl-, 2-Methylpropionyl- oder Butyryl-Gruppe;
unter dem Ausdruck "(C₁-C₆)-Alkanoyl" die vorstehend genannte Reste sowie z.B. die Valeroyl-, Pivaloyl oder Hexanoyl-Gruppe;
unter dem Ausdruck "(C₂-C₆)-Haloalkanoyl" eine (C₂-C₆)-Alkanoyl-Gruppe, in der die Wasserstoffatome teilweise, im Falle von Fluor auch vollständig, durch Halogenatome, bevorzugt Fluor oder Chlor ersetzt sind;
unter dem Ausdruck "(C₁-C₆)-Alkoxycarbonyl" z.B. die Methoxycarbonyl-, Ethoxycarbonyl-, Propoxycarbonyl-, Butoxycarbonyl, tert.-Butoxycarbonyl-, Pentyloxycarbonyl oder Hexyloxycarbonyl-Gruppe;
unter dem Ausdruck "(C₁-C₆)-Haloalkoxycarbonyl" eine (C₁-C₆)-Alkoxycarbonyl-Gruppe in der ein oder mehrere, im Falle von Fluor gegebenenfalls auch alle Wasserstoffatome, durch Halogen, bevorzugt Fluor oder Chlor, ersetzt sind;
unter dem Ausdruck "(C₁-C₆)-Alkylthio" eine Alkylthiogruppe, deren Kohlenwasserstoffrest die unter dem Ausdruck "(C₁-C₆)-Alkyl" angegebene Bedeutung hat;
unter dem Ausdruck "(C₁-C₆)-Halogenalkylthio" eine (C₁-C₆)-Alkylthio-Gruppe, in der ein oder mehrere, im Falle von Fluor gegebenenfalls auch alle Wasserstoffatome des Kohlenwasserstoff-Teils durch Halogen, insbesondere Chlor oder Fluor ersetzt sind;
unter dem Ausdruck "(C₁-C₆)-Alkylsulfinyl" z.B. die Methyl-, Ethyl-, Propyl-, Isopropyl-, Butyl-, Isobutyl-, sek.-Butyl-, tert.-Butyl-, Pentyl-, 2-Methylbutyl-oder Hexylsulfinyl-Gruppe;
unter dem Ausdruck "(C₁-C₆)-Alkylsulfonyl" z.B. die Methyl-, Ethyl-, Propyl-, Isopropyl-, Butyl-, Isobutyl-, sek.-Butyl-, tert.-Butyl-, Pentyl-, 2-Methylbutyl- oder Hexylsulfonyl-Gruppe;
unter den Ausdrücken "(C₁-C₆)-Halogenalkylsulfinyl" und "(C₁-C₆)-Halogenalkylsulfonyl" (C₁-C₆)-Alkylsulfinyl- und -sulfonyl-Reste mit den oben angegebenen Bedeutungen, bei denen ein oder mehrere, im Falle von Fluor gegebenenfalls auch alle Wasserstoffatome des Kohlenwasserstoff-Teils durch Halogen, insbesondere Chlor oder Fluor ersetzt sind;
unter dem Ausdruck "(C₁-C₆)-Alkoxy" eine Alkoxygruppe, deren Kohlenwasserstoffrest die unter dem Ausdruck "(C₁-C₆)-Alkyl" angegebene Bedeutung hat;
unter dem Ausdruck "(C₁-C₆)-Alkylamino" z.B. die Methylamino-, Ethylamino-, Propylamino-, Isopropylamino-, Butylamino-, Isobutylamino-, sek.-Butylamino-, tert.-Butylamino-, Pentylamino- oder die Hexylamino-Gruppe;
unter dem Ausdruck "(C₁-C₆)-Dialkylamino" z.B. die Dimethylamino-, Methyl-ethylamino-, Diethylamino-, Dipropylamino-, Dibutylamino, Dipentylamino- oder die Dihexylamino-Gruppe; aber auch cyclische Systeme wie z.B. die Pyrrolidino- oder Piperidino-Gruppe,
unter dem Ausdruck "(C₁-C₈)-Halogenalkoxy" eine Halogenalkoxygruppe, deren Halogen-Kohlenwasserstoffrest die unter dem Ausdruck "(C₁-C₆)-Halogenalkyl" angegebene Bedeutung hat;
unter dem Ausdruck "Aryl" ein carbocyclischer aromatischer Rest mit 6 bis 14, insbesondere 6 bis 12 C-Atomen, wie Phenyl oder Naphthyl, bevorzugt Phenyl;
unter dem Ausdruck "Heterocyclyl" ein heteroaromatisches oder heteroaliphatisches Ringsystem, wobei unter "heteroaromatisches Ringsystem" ein Arylrest, worin mindestens eine CH-Gruppe durch N ersetzt ist und/oder mindestens zwei benachbarte CH-Gruppen durch S, NH oder O ersetzt sind, zu verstehen ist, z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Triazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin oder 4H-Chinolizin;
und unter dem Ausdruck "heteroaliphatisches Ringsystem" einen (C₃-C₈)-Cycloalkylrest in dem mindestens eine Kohlenstoff-Einheit durch O S oder eine Gruppe NR¹¹ ersetzt ist und R¹¹ Wasserstoff, (C₁-C₄)-Alkyl, (C₁-C₄)-Alkoxy oder Aryl bedeutet;
unter dem Ausdruck "Arylthio" z.B. die Phenylthio-Gruppe;
unter dem Ausdruck "Aryloxy" z.B. die Phenoxy-Gruppe;
unter dem Ausdruck "Heterocyclyloxy" oder "Heterocyclylthio" einen der oben genannten heterocyclischen Reste die über ein Sauerstoff- oder Schwefelatom verknüpft sind;
unter dem Ausdruck "(C₃-C₈)-Cycloalkoxy" oder "(C₃-C₈)-Cycloalkylthio" eine der oben angeführten (C₃-C₈)-Cycloalkyl-Reste, die über ein Sauerstoff- oder Schwefelatom verknüpft sind;
unter dem Ausdruck "(C₃-C₈)-Cycloalkoxycarbonyl" z.B. die Cyclobutyloxycarbonyl-, Cyclopentyloxycarbonyl-, Cyclohexyloxycarbonyl- oder die Cycloheptyloxycarbonyl-Gruppe;
und unter dem Ausdruck "gegebenenfalls substituiertes Aryl, Heterocyclyl, Phenyl, etc." ist bevorzugt Substitution durch einen oder mehrere, vorzugsweise 1 bis 3, im Falle von Halogen auch bis zur maximalen Anzahl, Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Haloalkylthio, (C₁-C₄)-Alkylamino, (C₁-C₄)-Haloalkylamino, Formyl oder (C₁-C₄)-Alkanoyl zu verstehen.

Die oben angegebene Erläuterung gilt entsprechend für Homologe bzw. deren abgeleitete Reste.

Die vorliegende Erfindung betrifft die Verbindungen der Formel (I) in Form der freien Base oder eines Säureadditionssalzes. Säuren, die zur Salzbildung herangezogen werden können, sind beispielsweise anorganische Säuren, wie Salzsäure, Bromwasserstoffsäure, Salpetersäure, Schwefelsäure, Phosphorsäure oder organische Säuren, wie Ameisensäure, Essigsäure, Propionsäure, Malonsäure, Oxalsäure, Fumarsäure, Adipinsäure, Stearinsäure, Ölsäure, Methansulfonsäure, Benzolsulfonsäure oder Toluolsulfonsäure.

Die Verbindungen der Formel (I) weisen zum Teil ein oder mehrere chirale Kohlenstoffatome oder Stereoisomere an Doppelbindungen auf. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer chiralen, enantiomerenreinen Säure, Trennung der diastereomeren Salze und Freisetzung der reinen Enantiomeren mittels einer Base.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben sind.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die allgemeine Chemie der 1,3-Oxazoline ist beispielsweise beschrieben in Tetrahedron, 1994, 50, 2297-2360 sowie in Nachr. Chem. Tech. Lab. 1996, 44, 744-750.
Gegenstand der Erfindung ist auch ein Verfahren zur Herstellung von Verbindungen der Formel (I, G = 3-Isoxazolinyl) durch die Umsetzung von 1,3-Oxazolinen, 1,3-Thiazolinen, Pyrrolinen und Imidazolinen der Formel (II) (siehe z.B. WO-A-96/22283) (geeignet substituiert mit Xₙ und R¹ₘ) mit einem Halogenierungsmittel zu Verbindungen der Formel (III) und Umsetzung dieser Verbindungen mit einem Olefin (IV) (geeignet substituiert mit R⁵ₜ), wobei man zuerst ein Oxim der Formel (II), 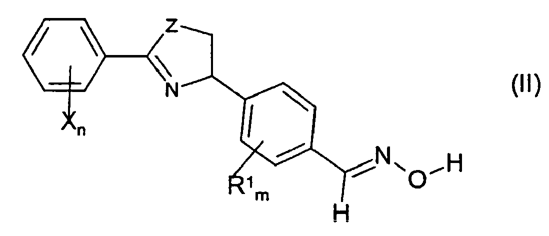 worin
X und Z die in der Formel (I) angegebenen Bedeutungen haben,
mit einem Halogenierungsmittel, vorzugsweise einem Chlorierungsmittel, zu einer Verbindung der Formel (III) umsetzt, 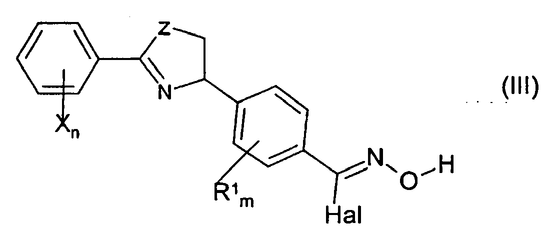 worin
Hal Halogen, vorzugsweise Cl, bedeutet,
und anschließend weiter mit einem Olefin der Formel (IV), 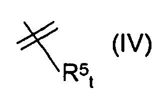 worin R⁵ und t die oben angegebenen Bedeutungen haben, umsetzt.

Gegenstand der Erfindung ist auch ein Verfahren zur Herstellung von Verbindungen der Formel (II) durch die Umsetzung von 1,3-Oxazolinen, 1,3-Thiazolinen, Pyrrolinen und Imidazolinen der Formel (V) (geeignet substituiert mit X und R¹) mit Hydroxylamin oder dessen Salzen, gegebenenfalls in Anwesenheit einer Base, 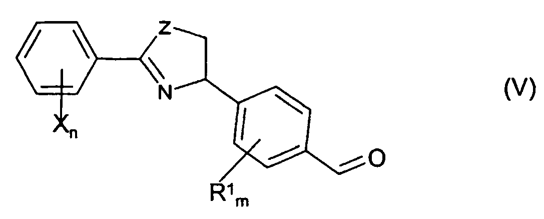 worin
Xₙ und Z die in Formel (I) angegebenen Bedeutungen haben.

Gegenstand der Erfindung ist auch ein Verfahren zur Herstellung von Verbindungen der Formel (V) aus 1,3-Oxazolinen, 1,3-Thiazolinen, Pyrrolinen und Imidazolinen der Formel (VI) (geeignet substituiert mit X und R¹), wobei man Verbindungen der Formel (VI), 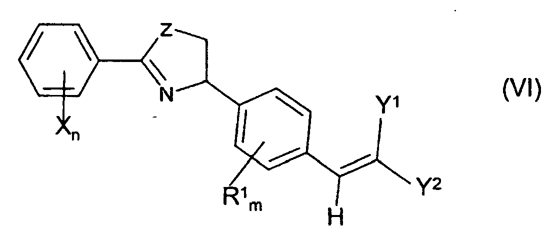 worin
Y¹ und Y² unabhängig voneinander Wasserstoff, (C₁-C₄)-Alkyl, (C₁-C₄)-Alkoxycarbonyl oder Phenyl bedeuten und
Xₙ und Z die in Formel (I) angegebenen Bedeutungen haben,
mit einem Oxidationsmittel zu Verbindungen der Formel (V) umsetzt.

Beispielhaft seien die Methoden A bis D zur Synthese verschiedener Untergruppen von Verbindungen der Formel (I), (G = 3-Isoxazinyl) aufgeführt:

Die Erzeugung des Isoxazolrings erfolgt vorteilhaft in Anwesenheit einer Base, beispielsweise aus der Gruppe Alkalihydroxyde, Alkalicarbonate, Alkoholat und Amine.

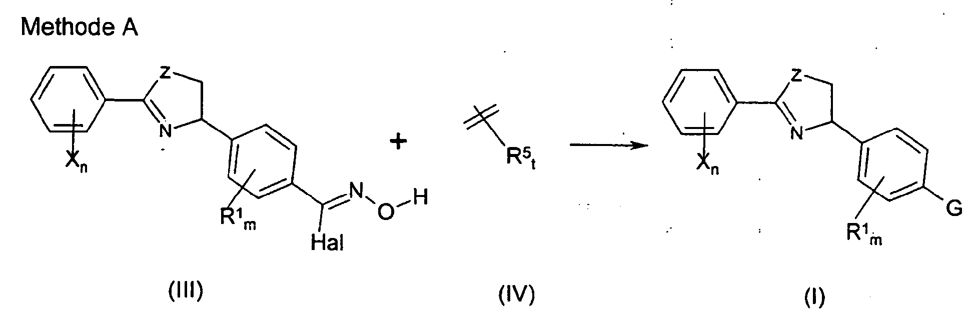

Oxime der Formel (II) werden durch Halogenierungsmittel in die Halogenoxime (III) überführt. Als Halogenierungsmittel eignen sich beispielsweise elementares Halogen, Hypohalogenite und N-Halogenimide:

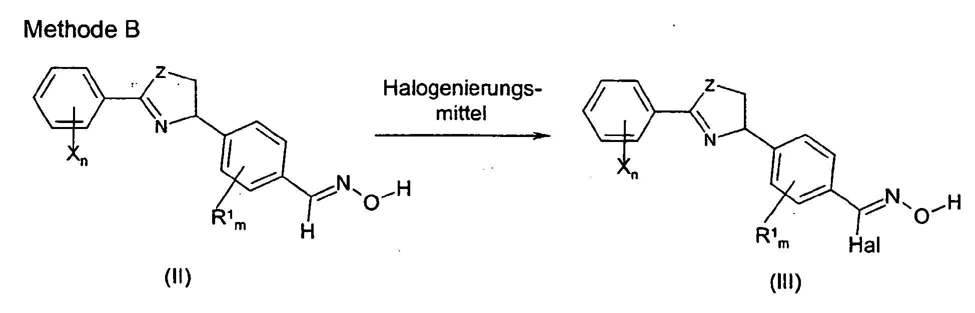

Oxime der Formel (II) werden durch Reaktion der Aldehyde (V) mit Hydroxylamin oder Hydroxylaminsalzen, gegenenfalls in Anwesenheit einer Base, hergestellt:

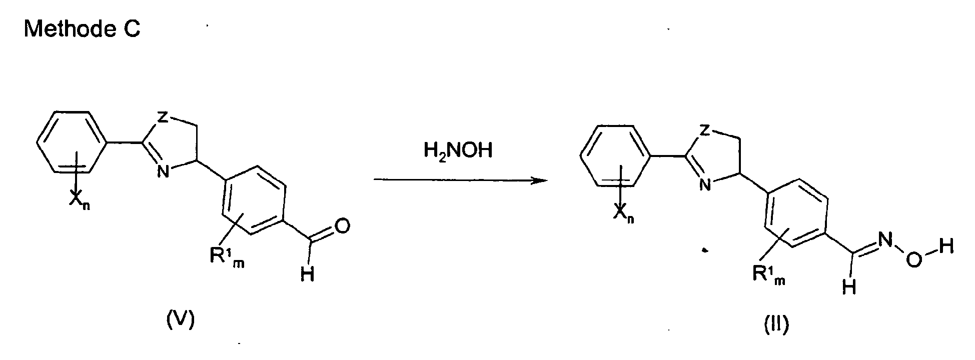

Aldehyde der Formel (V) werden durch Spaltung der Olefine der Formel (VI) mittels eines Oxidationsmittels erzeugt. Als Oxidationsmittel dienen beispielsweise Ruthenium- oder Osmiumverbindungen in Kombination mit einem Periodat, oder Ozon:

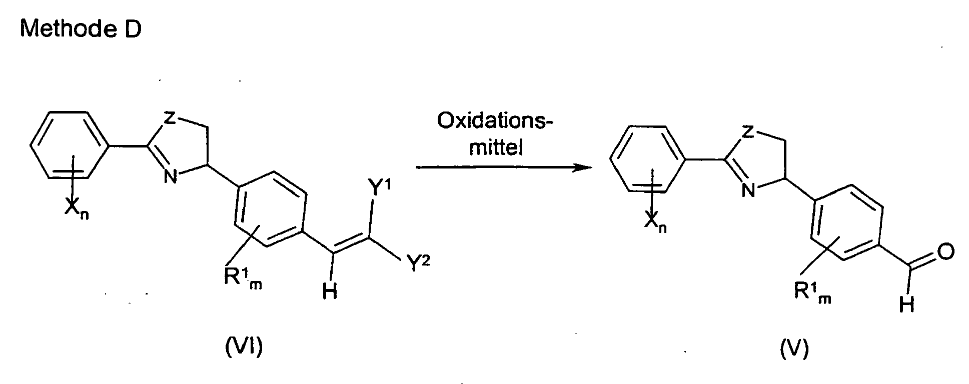

Verbindungen der Formel (VI) sind teils beschrieben (WO-A-95/04726), beziehungsweise können analog hergestellt werden.

Gegenstand der Erfindung ist auch ein Verfahren zur Herstellung von Verbindungen der Formel (I) (G = 5-Isoxazinyl) durch Umsetzung von 1,3-Oxazolinen, 1,3-Thiazolinen, Pyrrolinen und Imidazolinen der Formel (VII) (siehe z.B. WO-A 95/04726), geeignet substituiert mit Xₙ und R¹_{m'} mit einem Halogenoxim, wobei man ein Olefin der Formel (VII), 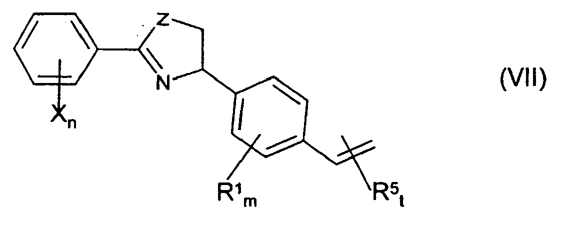 worin
Z und R⁵ₜ die oben angegebenen Bedeutungen haben, mit einem Halogenoxim der Formel (VIII) umsetzt, 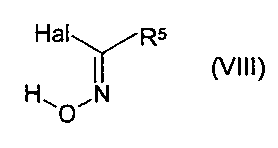 wobei R⁵ die oben angegebenen Bedeutungen hat.

Beispielhaft sei die Methode E zur Synthese von Verbindungen der Formel (I) (G = 3-Isoxazinyl) aufgeführt:

Die Erzeugung des Isoxazolrings erfolgt in Anwesenheit einer Base, beispielsweise aus der Gruppe Alkalihydroxyde, Alkalicarbonate, Alkoholat und Amine.

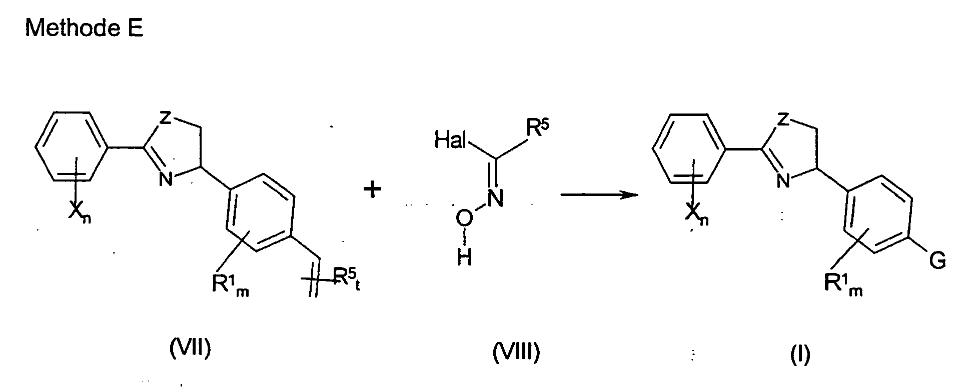

Unterschiedliche Ester und Amide als Reste R⁵ können beispielsweise aus Säurederivaten hergestellt werden. Diese wiederum sind beispielsweise durch Verseifung eines Esters erhältlich. Z.B.

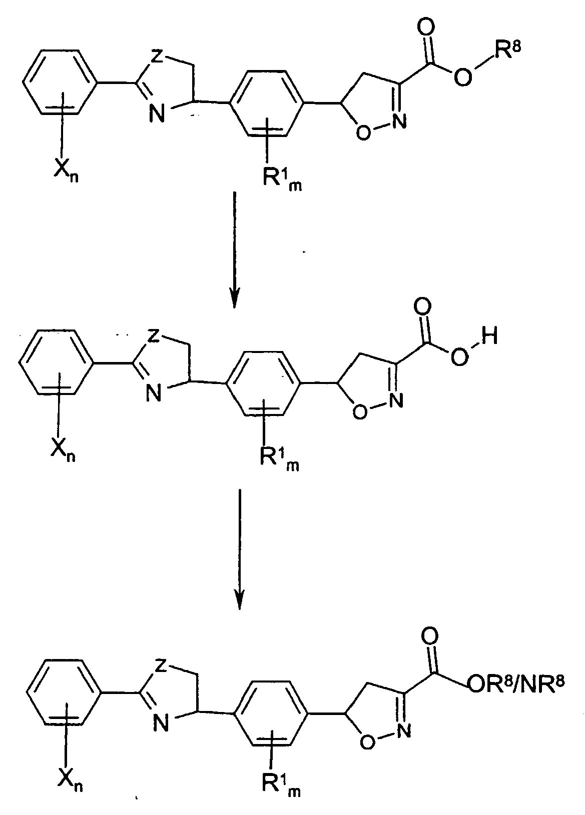

Als Verseifungsmittel können beispielsweise wäßrige Laugen dienen.
Bei der Herstellung der Amide oder Ester kann die Säure aktiviert werden, beispielsweise mit einem Carbodiimid, mit Carbonyldiimidazol oder mit einem anorganischen Säurechlorid, z. B. Thionylchlorid.

Unterschiedliche Ester und Amide als Rest R⁵ können beispielsweise auch aus Hydroxy- bzw. Aminderivaten hergestellt werden. Diese wiederum sind beispielsweise durch Verseifung eines Esters bzw. Amids erhältlich, z. B.:

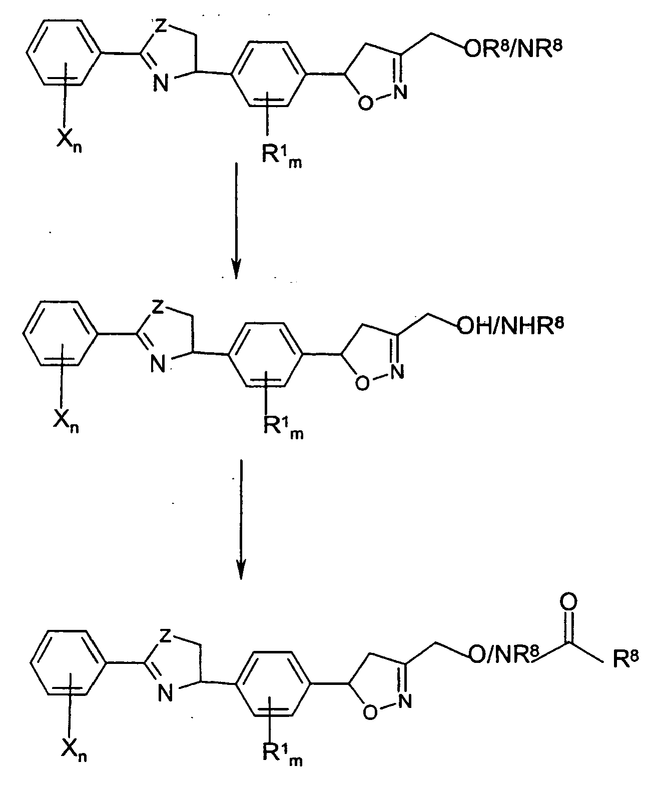

Als Verseifungsmittel können beispielsweise wäßrige Laugen dienen.
Zur der Herstellung der Amide oder Ester kann der Alkohol oder das Amin beispielsweise mit einer aktivierten Säure, z. B. einem Säurechlorid umgesetzt werden.

Kollektionen aus Verbindungen der Formel (I), die nach oben genannten Schema synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, CM9 8SE, England oder H+P Labortechnik GmbH, Bruckmannring 28, 85764 Oberschleißheim, Deutschland angeboten werden. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben dem hier beschriebenen kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z.B. Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die Verbindungen der Formel (I) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.. Aus der Ordnung der Isopoda z.B. Oniscus aselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung des Isoptera z.B. Reticulitermes spp..
Aus der Ordnung der Anoplura z.B. Phylloera vastatrix, Pemphigus spp., Pediculus humanus corporis, Haematopinus spp., Linognathus spp..
Aus der Ordnung der Mallophaga z.B. Trichodectes pp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.,
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopsis, Ceratophyllus spp.. Aus der Ordnung der Arachnida z.B. Scorpio maurus, Latrodectus mactans.
Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.

Aus der Klasse der Gastropoda z.B. Deroceras spp., Arion spp., Lymnaea spp., Galba spp., Succinea spp., Biomphalaria spp., Bulinus spp., Oncomelania spp.. Aus der Klasse der Bivalva z.B. Dreissena spp..

Zu den pflanzenparasitären Nematoden, die erfindungsgemäß bekämpft werden können, gehören beispielsweise die wurzelparasitären Bodennematoden, z.B. solche der Gattungen Meloidogyne (Wurzelgallennematoden, wie Meloidogyne incognita, Meloidogyne hapla und Meloidogyne javanica), Heterodera und Globodera (zystenbildende Nematoden, wie Globodera rostochiensis, Globodera pallida, Heterodera trifolii) sowie der Gattungen Radopholus, wie Radopholus similis, Pratylenchus, wie Pratyglenchus neglectus, Pratylenchus penetrans und Pratylenchus curvitatus,
Tylenchulus, wie Tylenchulus semipenetrans, Tylenchorhynchus, wie Tylenchorhynchus dubius und Tylenchorhynchus claytoni, Rotylenchus, wie Rotylenchus robustus, Heliocotylenchus, wie Haliocotylenchus multicinctus, Belonoaimus, wie Belonoaimus longicaudatus, Longidorus, wie Longidorus elongatus, Trichodorus, wie Trichodorus primitivus und Xiphinema, wie Xiphinema index.

Ferner lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, beispielsweise Pflanzenschutzmittel, vorzugsweise insektizide, akarizide, ixodizide, nematizide, molluskizide oder fungizide, besonders bevorzugt insektizide und akarizide Mittel, die eine oder mehrere Verbindungen der Formel (I) neben geeigneten Formulierungshilfsmitteln enthalten.

Die erfindungsgemäßen Mittel enthalten die Wirkstoffe der Formel (I) im allgemeinen zu 1 bis 95 Gew.-%.

Zur Herstellung der erfindungsgemäßen Mittel gibt man den Wirkstoff und die weiteren Zusätze zusammen und bringt sie in eine geeignete Anwendungsform.

Sie können auf verschiedene Art formuliert werden, je nachdem wie es durch die biologischen und/oder chemisch-physikalischen Parameter vorgegeben ist. Als Formulierungsmöglichkeiten kommen beispielsweise in Frage:
Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, wie Inertmaterialien, Tenside, Lösungsmittel und weitere Zusatzstoffe, sind ebenfalls bekannt und beispielsweise beschrieben in:
Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze, wie Ca-Dodecylbenzol-sulfonat, oder nichtionische Emulgatoren, wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man beispielsweise durch Vermahlen des Wirkstoffes mit fein verteilten festen Stoffen, z.B. Talkum oder natürlichen Tonen, wie Kaolin, Bentonit, Pyrophyllit oder Diatomeenerde. Granulate können entweder durch Verdüsen des Wirkstoffes auf adsorptionsfähiges, granuliertes Inertmaterial oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen, wie Sand oder Kaolinite, oder von granuliertem Inertmaterial hergestellt werden. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen, wie Temperatur oder Feuchtigkeit, variiert die erforderliche Aufwandmenge. Sie kann innerhalb weiter Grenzen schwanken, z.B. zwischen 0,0005 und 10,0 kg/ha oder mehr Aktivsubstanz, vorzugsweise liegt sie jedoch zwischen 0,001 und 5 kg/ha.

Die erfindungsgemäßen Wirkstoffe können in ihren handelsüblichen Formulierungen sowie in den aus diesen Formulierungen bereiteten Anwendungsformen (siehe die oben angegebenen Mittel) in Mischungen mit anderen Wirkstoffen, wie Insektiziden, Lockstoffen, Sterilantien, Akariziden, Nematiziden, Fungiziden, Molluskiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:
1. aus der Gruppe der Phosphorverbindungen
   Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, Isazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;
2. aus der Gruppe der Carbamate
   Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;
3. aus der Gruppe der Carbonsäureester
   Acrinathrin, Allethrin, Alphametrin, 5-Benzyl-3-furylmethyl-(E)-(1 R)-cis-2,2-dimethyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, ABG-9008, Acetamiprid, Anagrapha falcitera, AKD-1022, AKD-3059, ANS-118, Bacillus thuringiensis, Beauveria bassiana, Bensultap, Bifenazate (D-2341), Binapacryl, BJL-932, Bromopropylate, BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy) phenyl)carbamoyl)-2-chlorbenzcarboximidsäureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granulose- und Kempolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345), Halofenprox (MTI-732), Hexaflumuron (DE_473), Hexythiazox, HOI-9004, Hydramethylnon (AC 217300), IKI 220, Imidacloprid, Indoxacarb (DPX-MP062), Kanemite (AKD-2023), M-020, MTI-446, Ivermectin, Lufenuron, M-020, Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601, Silafluofen, Silomadine (CG-177), Spinosad, SU-9118, Tebufenozide, Tebufenpyrad (MK-239), Teflubenzuron, Tetradifon, Tetrasul, Thiacloprid, Thiocyclam, TI-435, Tolfenpyrad (OMI-88); Triazamate (RH-7988), Triflumuron, Verbutin, Vertalec (Mykotal), YI-5301,

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen. Die Anwendung geschieht in einer den Anwendungsformen angepaßten üblichen Weise.

Gegenstand der Erfindung ist auch ein Verfahren zur Bekämpfung von Schadinsekten, Acarina, Mollusken und/oder Nematoden, bei welchem man auf diese oder die von ihnen befallenen Pflanzen, Flächen oder Substrate eine wirksame Menge einer erfindungsgemäßen Verbindung oder eines erfindungsgemäßen Mittels aufbringt.

Ebenso Gegenstand der Erfindung ist die Verwendung einer erfindungsgemäßen Verbindung oder eines erfindungsgemäßen Mittels zur Bekämpfung von Schadinsekten, Acarina, Mollusken und/oder Nematoden.

Die erfindungsgemäßen Wirkstoffe eignen sich auch auf dem veterinärmedizinischen Gebiet, vorzugsweise zur Bekämpfung von Endo- und Ektoparasiten, und auf dem Gebiet der Tierhaltung.

Die Anwendung der erfindungsgemäßen Wirkstoffe kann hierbei in bekannter Weise geschehen, wie durch orale Anwendung in Form von beispielsweise Tabletten, Kapseln, Tränken oder Granulaten, durch dermale Anwendung in Form beispielsweise des Tauchens (Dippen), Sprühens (Sprayen), Aufgießen (pour-on and spot-on) und des Einpuderns, sowie durch parenterale Anwendung in Form beispielsweise der Injektion.

Die erfindungsgemäßen Verbindungen der Formel (I) können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneter Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Gegenstand der Erfingung ist daher auch die Verwendung einer Verbindung der Formel (I) oder eines der obengenannten Mittel zur Herstellung eines Tierarzneimittels.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Verbindungen der Formel (I) können in ihren handelsüblichen Formulierungen entweder allein oder in Kombination mit weiteren literaturbekannten Fungiziden angewendet werden.

Als literaturbekannte Fungizide, die erfindungsgemäß mit den Verbindungen der Formel (I) kombiniert werden können, sind z.B. folgende Produkte zu nennen: Aldimorph, Andoprim, Anilazine, BAS 480F, BAS 450F, Benalaxyl, Benodanil, Benomyl, Binapacryl, Bitertanol, Bromuconazol, Buthiobate, Captafol, Captan, Carbendazim, Carboxin, CGA 173506, Cyprofuram, Dichlofluanid, Dichlomezin, Diclobutrazol, Diethofencarb, Difenconazol (CGA 169374), Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Ferimzone (TF164), Fluazinam, Fluobenzimine,Fluquinconazole, Fluorimide, Flusilazole, Flutolanil, Flutriafol, Folpet, Fosetylaluminium,Fuberidazole, Fulsulfamide (MT-F 651), Furalaxyl, Furconazol, Furmecyclox, Guazatine, Hexaconazole, ICI A5504, Imazalil, Imibenconazole, Iprobenfos, Iprodione, Isoprothiolane, KNF 317, Kupferverbindungen wie Cu-oxychlorid, Oxine-Cu, Cu-oxide, Mancozeb, Maneb, Mepanipyrim (KIF 3535), Metconazol, Mepronil, Metalaxyl, Methasulfocarb, Methfuroxam, MON 24000, Myclobutanil, Nabam, Nitrothalidopropyl, Nuarimol, Ofurace, Oxadixyl, Oxycarboxin, Penconazol, Pencycuron, PP 969, Probenazole, Propineb, Prochloraz, Procymidon, Propamocarb, Propiconazol, Prothiocarb, Pyracarbolid, Pyrazophos, Pyrifenox, Pyroquilon, Rabenzazole, RH7592, Schwefel, Tebuconazole, TF 167, Thiabendazole, Thicyofen, Thiofanatemethyl, Thiram, Tolclofos-methyl, Tolylfluanid, Triadimefon, Triadimenol, Tricyclazole, Tridemorph, Triflumizol, Triforine, Validamycin, Vinchlozolin, XRD 563, Zineb, Natriumdodecylsulfonate, Natrium-dodecyl-sulfat, Natrium-C13/C15-alkohol-ethersulfonat, Natrium-cetostearyl-phosphatester, Dioctyl-natrium-sulfosuccinat, Natrium-isopropyl-naphthalenesulfonat, Natrium-methylenebisnaphthalene-sulfonat, Cetyl-trimethyl-ammoniumchlorid, Salze von langkettigen primären, sekundären oder tertiären Aminen, Alkyl-propyleneamine, Lauryl-pyrimidiniumbromid, ethoxylierte quarternierte Fettamine, Alkyl-dimethyl-benzyl-ammoniumchlorid und 1-Hydroxyethyl-2-alkyl-imidazolin.

Die oben genannten Kombinationspartner stellen bekannte Wirkstoffe dar, die zum großen Teil in C.D.S. Tomlin, S.B. Walker, The Pesticide Manual, 12. Auflage, British Crop Protection Council, Farnham 2000 beschrieben sind.

Gegenstand der Erfindung ist auch Saatgut, enthaltend eine oder beschichtet mit einer wirksamen Menge einer erfindungsgemäßen Verbindung oder eines erfindungsgemäßen Mittels.

Die Verbindungen der Formel (I) können auch zur Bekämpfung von Schadorganismen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten wie bestimmten Insekten oder Mikroorganismen, wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung in wirtschaftlich bedeutenden transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten.

Bei der Anwendung in transgenen Kulturen, insbesondere, solchen in denen die Pflanzen ein Insektizid exprimieren, treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Spektrum an Schädlingen, die bekämpft werden können, oder veränderte Aufwandmengen, die für die Applikation eingesetzt werden können.

Gegenstand der Erfindung ist deshalb auch die Verwendung von Verbindungen der Formel (I) zur Bekämpfung von Schadorganismen in transgenen Kulturpflanzen.

Die erfindungsgemäße Verwendung von Verbindungen der Formel (I) bzw. diese enthaltenden Mitteln, beispielsweise als Insektizid, Akarizid, Molluskizid oder Nematizid, schließt auch den Fall ein, bei dem die Verbindung der Formel (I) oder deren Salz erst nach dem Ausbringen, beispielsweise im Insekt, einer Pflanze oder im Boden, aus einer Vorläufersubstanz gebildet wird.

Nachfolgende Beispiele dienen zur Erläuterung der Erfindung, ohne daß diese darauf beschränkt wäre.

### A. Herstellungsbeispiele

### 3-Arylisoxazoline

### Vorprodukt VP1: 2-(2,6-Difluorphenyl)-4-(4-(2-phenylethenyl)phenyl)oxazolin

2-(2,6-Difluorphenyl)-4-(4-bromphenyl)oxazolin (33.8 g, 0.1 mol) und Styrol (22.9 ml, 0.2 mol) wurden mit Natriumcarbonat (11.66 g, 0.11 mol), Tris(2.4-di-tert-butylphenyl)phosphit (6.47 g, 10 mmol) und Palladiumacetat (0.45 g, 2 mmol) in 300 ml DMF 20 h auf Rückfluß erhitzt. Nach extraktiver Aufarbeitung mit Essigester wurde der Rückstand mit Heptan/Dichlormethan (1:1) ausgerührt. Man erhielt 27 g Kristalle, Smp.141 °C.

### Vorprodukt VP2: 2-(2,6-Difluorphenyl)-4-(4-formylphenyl)oxazolin

2-(2,6-Difluorphenyl)-4-(4-(2-phenylethenyl)phenyl)oxazolin (7.22 g, 20 mmol) und Natriummetaperiodat (8.55 g, 20 mmol) wurden bei 0°C in Acetonitril-Aceton-Wasser (1:1:1, 180 ml) suspendiert und mit einer katalytischen Menge an Rutheniumtrichlorid-Hydrat versetzt. Nach extraktiver Aufarbeitung mit Essigester und Säulenchromatographie erhielt man 5.6 g des Aldehyds,als zähes Öl.

### Vorprodukt VP3:2-(2,6-Difluorphenyl)-4-(4-(hydroxyiminomethyl)phenyl)oxazolin

2-(2,6-Difluorphenyl)-4-(4-formylphenyl)oxazolin (5.6 g), Hydroxylamin-Hydrochlorid (1.53 g, 1.1 Äquivalente) und Natriumacetat (4.9 g, 3 Äquivalente) wurden bei Raumtemperatur in 50 ml Ethanol 24 h gerührt. Nach extraktiver Aufarbeitung mit Essigester und Säulenchromatographie erhielt man 4.2 g Kristalle, Smp.159°C.

### 2-(2,6-Difluorphenyl)-4-(4-(5-tert-butylisoxazolin-3-yl)phenyl)oxazolin (Bsp.-Nr. 9)

2-(2,6-Difluorphenyl)-4-(4-(hydroxyiminomethyl)phenyl)oxazolin (40 mg, 0.13 mmol) und N-Chlorsuccinimid (19 mg, 1.1 Äquivalente) wurden in 2 ml DMF bei 50°C für 4 h erhitzt. Nach Abkühlen auf Raumtemperatur wurden 3,3-Dimethylbuten (33 mg, 0.4 mmol) und Triethylamin (41 mg, 0.4 mmol) zugegeben. Nach Rühren für 16 h wurde extraktiv mit Essigester aufgearbeitet und der Rückstand säulenchromatographisch gereinigt. Man erhielt 19 mg Produkt.

### 2-(2,6-Difluorphenyl)-4-(4-(5-trifluormethylisoxazolin-3-yl)phenyl)oxazolin (Bsp.-Nr. 43)

2-(2,6-Difluorphenyl)-4-(4-(hydroxyiminomethyl)phenyl)oxazolin (40 mg, 0.13 mmol) und N-Chlorsuccinimid (19 mg, 1.1 Äquivalente) wurden in 2 ml DMF bei 50°C für 4 h erhitzt. Nach Abkühlen auf Raumtemperatur wurden 2 ml einer mit 3,3,3-Trifluorpropen gesätigten DMF-Lösung und Triethylamin (41 mg, 0.4 mmol) zugegeben. Nach Rühren für 16 h wurde extraktiv mit Essigester aufgearbeitet und der Rückstand säulenchromatographisch gereinigt. Man erhielt 37 mg Produkt.

### 2-(2,6-Difluorphenyl)-4-(4-(5-(trifluoracetamidomethyl)isoxazolin-3-yl)phenyl)oxazolin (Bsp.-Nr. 115)

2-(2,6-Difluorphenyl)-4-(4-(hydroxyiminomethyl)phenyl)oxazolin (1.2 g, 4 mmol) und N-Chlorsuccinimid (560 mg, 1.05 Äquivalente) wurden in 6 ml DMF bei 50°C für 4h erhitzt. Nach Abkühlen auf Raumtemperatur wurden N-Allyl-trifluoracetamid (2.75 g, 3 Äquivalente) und Triethylamin (1.66 ml, 3 Äquivalente) zugegeben. Nach Rühren für 16 h wurde extraktiv mit Essigester aufgearbeitet und der Rückstand säulenchromatographisch gereinigt. Man erhielt 920 mg Produkt.

### 2-(2,6-Difluorphenyl)-4-(4-(5-(propionylaminomethyl)isoxazolin-3-yl)phenyl)oxazolin (Bsp.-Nr. 116)

2-(2,6-Difluorphenyl)-4-(4-(5-(trifluoracetamidomethyl)isoxazolin-3-yl)phenyl)oxazolin (43 mg) wurden in 2 ml Methanol mit 0.5 ml 2N Natronlauge versetzt und für 16 h gerührt. Nach extraktiver Aufarbeitung mit Dichlormethan wurde das rohe Amin in 2 ml Dichlormethan bei 0°C mit Triethylamin (0.05 ml) und Propionylchlorid (50 mg) versetzt. Nach Rühren für 2 h wurde extraktiv mit Essigester aufgearbeitet und der Rückstand säulenchromatographisch gereinigt. Man erhielt 40 mg Produkt.

### 5-Arylisoxazoline

### Vorprodukt VP4: 2-(2,6-Difluorphenyl)-4-(4-ethenylphenyl)oxazolin

2-(2,6-Difluorphenyl)-4-(4-bromphenyl)oxazolin (6.0 g, 18 mmol) wurde im Autoklaven mit Natriumcarbonat (2.9 g, 21 mmol), Tris(2,4-di-tert-butylphenyl)phosphit (1.2 g, 1.8 mmol) und Palladiumacetat (64 mg, 2 % Äquivalente) in 100 ml DMF unter 20 bar Ethylen für 44 h auf 150°C erhitzt. Nach extraktiver Aufarbeitung mit Essigester und Säulenchromatographie wurden 3.75 g Kristalle erhalten, Smp.76°C.

### 2-(2,6-Difluorphenyl)-4-(4-(3-methylisoxazolin-5-yl)phenyl)oxazolin (Bsp.-Nr. 566)

Acetaldoxim (30 mg, 0.5 mmol) und N-Chlorsuccinimid (67 mg, 1 Äquivalent) in 3 ml DMF wurden 3 h bei Raumtemperatur gerührt. Anschließend, wurden 2-(2,6-Difluorphenyl)-4-(4-ethenylphenyl)oxazolin (43 mg, 0.15 mmol) und Triethylamin (46 mg, 0.45 mmol) zugegeben und 16 h gerührt. Nach extraktiver Aufarbeitung mit Essigester und Säulenchromatographie wurden 32 mg Produkt erhalten.

### 2-(2,6-Difluorphenyl)-4-(4-(3-tert-butylisoxazolin-5-yl)phenyl)oxazolin (Bsp.-Nr. 573)

Pivalinaldehydoxim (51 mg, 0.5 mmol) und N-Chlorsuccinimid (67 mg, 1 Äquivalent) in 3 ml DMF wurden 3 h bei Raumtemperatur gerührt. Anschließend wurden 2-(2,6-Difluorphenyl)-4-(4-ethenylphenyl)oxazolin (43 mg, 0.15 mmol) und Triethylamin (46 mg, 0.45 mmol) zugegeben und 16 h gerührt. Nach extraktiver Aufarbeitung mit Essigester und Säulenchromatographie wurden 30 mg Produkt erhalten.

### 2-(2,6-Difluorphenyl)-4-(4-(3-ethoxycarbonylisoxazolin-5-yl)phenyl)oxazolin (Bsp.-Nr. 614)

2-(2,6-Difluorphenyl)-4-(4-ethenylphenyl)oxazolin (570 mg, 2 mmol) und 2-Chlor-2-hydroxyimino-essigsäureethylester (320 mg, 1.05 Äquivalente) in 10 ml Dichlorethan wurden bei 0°C mit Triethylamin (0.33 ml, 1.2 Äquivalente) versetzt und 16 h bei Raumtemperatur gerührt. Nach extraktiver Aufarbeitung mit Essigester und Säulenchromatographie wurden 420 mg Produkt erhalten.

### 2-(2,6-Difluorphenyl)-4-(4-(3-(2,2,2-trifluorethylaminocarbonyl)isoxazolin-5-yl)phenyl)oxazolin (Bsp.-Nr. 628)

2-(2,6-Difluorphenyl)-4-(4-(3-ethoxycarbonylisoxazolin-5-yl)phenyl)oxazolin (769 mg, 1.9 mmol) wurden in 20 ml Ethanol und 6.5 ml 2N Natronlauge für 3 h bei Raumtemperatur gerührt. Nach Ansäuem mit 2N Salzsäure wurde extraktiv mit Dichlormethan aufgearbeitet. Man erhielt 715 mg rohe Säure, die direkt weiter eingesetzt werden konnte.
47 mg, 0 13 mmol davon in 2 ml DMF wurden mit Hydroxybenzotriazol (18 mg, 1 Äquivalent) und N-Ethyl-N'-(3-dimethylaminopropyl)carbodiimid (25 mg, 1 Äquivalent) versetzt. Anschließend gab man Ethyldiisopropylamin (17 mg, 1 Äquivalent) in 1 ml THF und 2,2,2-Trifluorethylamin (0.015 ml) in 1 ml THF zu. Nach Rühren bei 50°C für 16 h, extraktiver Aufarbeitung mit Essigester und Säulenchromatographie wurden 41 mg Produkt erhalten.

### B. Chemische Beispiele (Tabellen 1 - 4)

**Tabelle 1 Oxazoline der Formel (I), Z= O, G = 3-Isoxazolinyl**

| | | | | | |
|---|---|---|---|---|---|
| 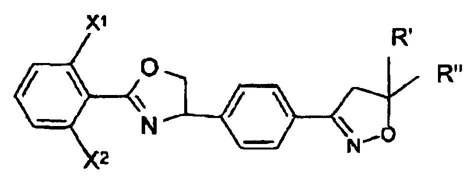 | | | | | |

| **Bsp.-Nr.** | **X**^{**1**} | **X**^{**2**} | **R**^{**6**} | **R**^{**66**} | **Phys. Daten** |
|---|---|---|---|---|---|
| 2 | F | F | H | CH₃ | NMR |
| 3 | " | " | " | C₂H₅ | |
| 4 | " | " | " | n-C₃H₇ | |
| 5 | " | " | " | i-C₃H₇ | NMR |
| 6 | " | " | " | n-C₄H₉ | |
| 7 | " | " | " | i-C₄H₉ | |
| 8 | " | " | " | s-C₄H₉ | |
| 9 | " | " | " | t-C₄H₉ | NMR |
| 10 | " | " | " | n-C₆H₁₃ | NMR |
| 11 | " | " | " | CH₂-t-Bu | NMR |
| 12 | " | " | " | CH₂Cl | NMR |
| 13 | " | " | " | CH₂Br | NMR |
| 14 | F | F | CH₃ | CH₃ | |
| 15 | " | " | " | C₂H₅ | |
| 16 | " | " | " | n-C₃H₇ | |
| 17 | " | " | " | i-C₃H₇ | |
| 18 | " | " | " | s-C₄H₉ | NMR |
| 19 | " | " | " | i-C₄H₉ | NMR |
| 20 | " | " | " | t-C₄H₉ | NMR |
| 21 | " | " | " | n-C₆H₁₃ | |
| 22 | " | " | " | CH₂- t-Bu | NMR |
| 23 | " | " | " | CH₂Cl | NMR |
| 24 | F | F | H | OCH₃ | |
| 25 | " | " | " | OC₂H₅ | |
| 26 | " | " | " | O-n-C₃H₇ | |
| 27 | " | " | " | O-n-C₄H₉ | |
| 28 | " | " | " | O-i-C₄H₉ | NMR |
| 29 | " " | | " | CN | NMR |
| 30 | " | " | " | CH₂CN | NMR |
| 31 | " | " | " | CH₂OCH₃ | |
| 32 | " | " | " | CH₂OC₂H₅ | NMR |
| 33 | " | " | " | CH₂O-n-C₃H₇ | NMR |
| 34 | " | " | " | CH₂O-i-C₃H₇ | |
| 35 | " | " | " | CH₂O-n-C₄H₉ | NMR |
| 36 | " | " | " | CH₂O-i-C₄H₉ | |
| 37 | " | " | " | CH₂O-s-C₄H₉ | |
| 38 | " | " | " | CH₂O-t-C₄H₉ | |
| 39 | " | " | " | CH₂OCF₂CF₂H | NMR |
| 40 | " | " | " | CH₂OCH₂CF₃ | NMR |
| 41 | " | " | " | CH₂O-Phenyl | NMR |
| 42 | " | " | " | CH₂O-2-Pyridyl | NMR |
| 43 | " | " | " | CF₃ | NMR |
| 44 | " | " | " | C₂F₅ | |
| 45 | " | " | " | n-C₃F₇ | NMR |
| 46 | " | " | " | n-C₄F₉ | NMR |
| 47 | " | " | " | n-C₅F₁₁ | |
| 48 | " | " | " | n-C₆F₁₃ | NMR |
| 49 | " | " | " | Phenyl | NMR |
| 50 | " | " | " | 2-F-Phenyl | NMR |
| 51 | " | " | " | 3-F-Phenyl | NMR |
| 52 | " | " | " | 4-F-Phenyl | NMR |
| 53 | " | " | " | 2-Cl-Phenyl | NMR |
| 54 | " | " | " | 3-Cl-Phenyl | |
| 55 | " | " | " | 4-Cl-Phenyl | NMR |
| 56 | " | " | " | 2,4-Cl₂-Phenyl | |
| 57 | " | " | " | 3,4-Cl₂-Phenyl | |
| 58 | " | " | " | 2,6-Cl₂-Phenyl | NMR |
| 59 | " | " | " | 4-Br-Phenyl | NMR |
| 60 | " | " | " | 2-CF₃-Phenyl | NMR |
| 61 | " | " | " | 3-CF₃-Phenyl | |
| 62 | " | " | " | 3,5-(CF₃)₂-Phenyl | NMR |
| 63 | " | " | " | 4-CF₃-Phenyl | NMR |
| 64 | " | " | " | 2-CH₃-Phenyl | NMR |
| 65 | " | " | " | 4-CH₃-Phenyl | NMR |
| 66 | " | " | " | 2,4-(CH₃)₂-Phenyl | NMR |
| 67 | " | " | " | 2,6-(CH₃)₂-Phenyl | |
| 68 | " | " | " | 2,4,6-(CH₃)₃-Phenyl | NMR |
| 69 | " | " | " | 2-CH₃O-Phenyl | |
| 70 | " | " | " | 4-CH₃O-Phenyl | NMR |
| 71 | " | " | " | 4-C₂H₅O-Phenyl | |
| 72 | " | " | " | 4-CF₃O-Phenyl | NMR |
| 73 | " | " | " | 4-CN-Phenyl | NMR |
| 74 | " | " | " | 4-t-Bu-Phenyl | NMR |
| 75 | " | " | " | 4-NO₂-Phenyl | |
| 76 | " | " | " | CH₂-Phenyl | NMR |
| 77 | " | " | " | CH₂-(4-F-Phenyl) | |
| 78 | " | " | " | C₂H₄Br | NMR |
| 79 | " | " | " | CH₂SCH₃ | NMR |
| 80 | " | " | " | CH₂SOCH₃ | |
| 81 | " | " | " | CH₂SO₂CH₃ | |
| 82 | " | " | " | CH₂SC₂H₅ | |
| 83 | " | " | " | CH₂S-n-C₃H₇ | |
| 84 | " | " | " | COOCH₃ | NMR |
| 85 | " | " | " | COOC₂H₅ | |
| 86 | " | " | " | COOCH₂CF₃ | NMR |
| 87 | " | " | " | COOC₂H₄CF₃ | NMR |
| 88 | " | " | " | C₂F₄Br | NMR |
| 89 | " | " | " | CONHCH₃ | |
| 90 | " | " | " | CONHC₂H₅ | NMR |
| 91 | " | " | " | CON(CH₃)₂ | NMR |
| 92 | " | " | " | CON(C₂H₅)₂ | NMR |
| 93 | " | " | " | CONH(n-C₃H₇) | NMR |
| 94 | " | " | " | CONHCH₂C₂F₅ | NMR |
| 95 | " | " | " | CONHCH₂C₂H₃ | NMR |
| 96 | " | " | " | CONH-t-C₄H₉ | NMR |
| 97 | " | " | " | CONH-n-C₅H₁₁ | NMR |
| 98 | " | " | " | CONHC₃H₆OCH₃ | NMR |
| 99 | " | " | " | CONHCH₂C₃F₇ | NMR |
| 100 | " | " | " | CONHCH₂-(2-Tetrahydrofuranyl) | NMR |
| 101 | " | " | " | CONH-Phenyl | |
| 102 | " | " | " | CONH(4-F-Phenyl) | |
| 103 | " | " | " | CONH(4-CF₃-Phenyl) | NMR |
| 104 | " | " | " | CONCH₃(Phenyl) | |
| 105 | " | " | " | CONCH₃(4-F-Phenyl) | |
| 106 | " | " | " | CONH(4-Cl-Phenyl) | |
| 107 | " | " | " | CONHC₂H₄(1-Piperidinyl) | NMR |
| 108 | " | " | " | CONHCH₂CF₃ | NMR |
| 109 | " | " | " | CONHCH₂Phenyl | |
| 110 | " | " | " | CONHCH₂(2,6-F₂-Phenyl) | NMR |
| 111 | " | " | " | CONHCH₂(4-F-Phenyl) | |
| 112 | " | " | " | CONHCH₂(4-CF₃-Phenyl) | |
| 113 | " | " | " | CONHCH₂(3-CF₃-Phenyl) | NMR |
| 114 | " | " | " | CH₂NHCOCH₃ | NMR |
| 115 | " | " | " | CH₂NHCOCF₃ | NMR |
| 116 | " | " | " | CH₂NHCOC₂H₅ | NMR |
| 117 | " | " | " | CH₂NHCOC₂F₅ | NMR |
| 118 | " | " | " | CH₂NHCO-n-C₃H₇ | |
| 119 | " | " | " | CH₂NHCO-i-C₃H₇ | |
| 120 | " | " | " | CH₂NHCO-n-C₃F₇ | NMR |
| 121 | " | " | " | CH₂NHCOC₂H₄CF₃ | NMR |
| 122 | " | " | " | CH₂NHCO-t-C₄H₉ | |
| 123 | " | " | " | CH₂NHCOphenyl | |
| 124 | " | " | " | CH₂NHCO(4-Cl-phenyl) | NMR |
| 125 | " | " | " | CH₂NHCO(2-Cl-5-pyridyl) | NMR |
| 126 | F | H | H | CH₃ | |
| 127 | " | " | " | C₂H₅ | |
| 128 | " | " | " | n-C₃H₇ | |
| 129 | " | " | " | i-C₃H₇ | |
| 130 | " | " | " | n-C₄H₉ | |
| 131 | " | " | " | i-C₄H₉ | |
| 132 | " | " | " | s-C₄H₉ | |
| 133 | " | " | " | t-C₄H₉ | |
| 134 | " | " | " | n-C₆H₁₃ | |
| 135 | " | " | " | CH₂-t-Bu | |
| 136 | " | " | " | CF₃ | |
| 137 | " | " | " | C₂F₅ | |
| 138 | " | " | " | n-C₃F₇ | |
| 139 | " | " | " | n-C₄F₉ | NMR |
| 140 | " | " | " | n-C₅F₁₁ | |
| 141 | " | " | " | n-C₆F₁₃ | NMR |
| 142 | " | " | " | Phenyl | |
| 143 | " | " | " | 2-F-Phenyl | |
| 144 | " | " | " | 3-F-Phenyl | |
| 145 | " | " | " | 4-F-Phenyl | |
| 146 | " | " | " | 2-Cl-Phenyl | |
| 147 | " | " | " | 3-Cl-Phenyl | |
| 148 | " | " | " | 4-Cl-Phenyl | NMR |
| 149 | " | " | " | 2,4-Cl₂-Phenyl | |
| 150 | " | " | " | 3,4-Cl₂-Phenyl | |
| 151 | " | " | " | 2,5-Cl₂-Phenyl | |
| 152 | " | " | " | 2,6-Cl₂-Phenyl | |
| 153 | " | " | " | 2-CF₃-Phenyl | |
| 154 | " | " | " | 3-CF₃-Phenyl | |
| 155 | " | " | " | 3,5-(CF₃)₂-Phenyl | |
| 156 | " | " | " | 4-CF₃-Phenyl | |
| 157 | " | " | " | 2-CH₃-Phenyl | |
| 158 | " | " | " | 4-CH₃-Phenyl | |
| 159 | " | " | " | 2,4-(CH₃)₂-Phenyl | |
| 160 | " | " | " | 2,6-(CH₃)₂-Phenyl | |
| 161 | " | " | " | 2,4,6-(CH₃)₃-Phenyl | |
| 162 | " | " | " | 2-CH₃O-Phenyl | |
| 163 | " | " | " | 4-CH₃O-Phenyl | |
| 164 | " | " | " | 4-C₂H₅O-Phenyl | |
| 165 | " | " | " | 4-CF₃O-Phenyl | |
| 166 | " | " | " | 4-CN-Phenyl | |
| 167 | " | " | " | 3-NO₂-Phenyl | |
| 168 | " | " | " | 4-NO₂-Phenyl | |
| 169 | F | Cl | H | CH₃ | |
| 170 | " | " | " | C₂H₅ | |
| 171 | " | " | " | n-C₃H₇ | |
| 172 | " | " | " | i-C₃H₇ | |
| 173 | " | " | " | n-C₄H₉ | |
| 174 | " | " | " | i-C₄H₉ | |
| 175 | " | " | " | S-C₄H₉ | |
| 176 | " | " | " | t-C₄H₉ | |
| 177 | " | " | " | n-C₆H₁₃ | |
| 178 | " | " | " | CH₂-t-Bu | |
| 179 | " | " | " | CF₃ | |
| 180 | " | " | " | C₂F₅ | |
| 181 | " | " | " | n-C₃F₇ | |
| 182 | " | " | " | n-C₄F₉ | NMR |
| 183 | " | " | " | n-C₅F₁₁ | |
| 184 | " | " | " | n-C₆F₁₃ | NMR |
| 185 | " | " | " | Phenyl | |
| 186 | " | " | " | 2-F-Phenyl | |
| 187 | " | " | " | 3-F-Phenyl | |
| 188 | " | " | " | 4-F-Phenyl | |
| 189 | " | " | " | 2-Cl-Phenyl | |
| 190 | " | " | " | 3-Cl-Phenyl | |
| 191 | " | " | " | 4-Cl-Phenyl | NMR |
| 192 | " | " | " | 2,4-Cl₂-Phenyl | |
| 193 | " | " | " | 3,4-Cl₂-Phenyl | |
| 194 | " | " | " | 2,5-Cl₂-Phenyl | |
| 195 | " | " | " | 2,6-Cl₂-Phenyl | |
| 196 | " | " | " | 2-CF₃-Phenyl | |
| 197 | " | " | " | 3-CF₃-Phenyl | |
| 198 | " | " | " | 3,5-(CF₃)₂-Phenyl | |
| 199 | " | " | " | 4-CF₃-Phenyl | |
| 200 | " | " | " | 2-CH₃-Phenyl | |
| 201 | " | " | " | 4-CH₃-Phenyl | |
| 202 | " | " | " | 2,4-(CH₃)₂-Phenyl | |
| 203 | " | " | " | 2,6-(CH₃)₂-Phenyl | |
| 204 | " | " | " | 2,4,6-(CH₃)₃-Phenyl | |
| 205 | " | " | " | 2-CH₃O-Phenyl | NMR |
| 206 | " | " | " | 4-CH₃O-Phenyl | |
| 207 | " | " | " | 4-C₂H₅O-Phenyl | |
| 208 | " | " | " | 4-CF₃O-Phenyl | |
| 209 | " | " | " | 4-CN-Phenyl | |
| 210 | " | " | " | 3-NO₂-Phenyl | |
| 211 | " | " | " | 4-NO₂-Phenyl | |
| 212 | Cl | H | H | CH₃ | |
| 213 | " | " | " | C₂H₅ | |
| 214 | " | | " | n-C₃H₇ | |
| 215 | " | " | " | i-C₃H₇ | |
| 216 | " | " | " | n-C₄H₉ | |
| 217 | " | " | " | i-C₄H₉ | |
| 218 | " | " | " | s-C₄H₉ | |
| 219 | " | " | " | t-C₄H₉ | |
| 220 | " | " | " | n-C₆H₁₃ | |
| 221 | " | " | " | CH₂-t-Bu | |
| 222 | " | " | " | CF₃ | |
| 223 | " | " | " | C₂F₅ | |
| 224 | " | " | " | n-C₃F₇ | |
| 225 | " | " | " | n-C₄F₉ | NMR |
| 226 | " | " | " | n-C₅F₁₁ | |
| 227 | " | " | " | n-C₆F₁₃ | NMR |
| 228 | " | " | " | Phenyl | |
| 229 | " | " | " | 2-F-Phenyl | |
| 230 | " | " | " | 3-F-Phenyl | |
| 231 | " | " | " | 4-F-Phenyl | |
| 232 | " | " | " | 2-Cl-Phenyl | |
| 233 | " | " | " | 3-Cl-Phenyl | |
| 234 | " | " | " | 4-Cl-Phenyl | NMR |
| 235 | " | " | " | 2,4-Cl₂-Phenyl | |
| 236 | " | " | " | 3,4-Cl₂-Phenyl | |
| 237 | " | " | " | 2,5-Cl₂-Phenyl | |
| 238 | " | " | " | 2,6-Cl₂-Phenyl | |
| 239 | " | " | " | 2-CF₃-Phenyl | |
| 240 | " | " | " | 3-CF₃-Phenyl | |
| 241 | " | " | " | 3,5-(CF₃)₂-Phenyl | |
| 242 | " | " | " | 4-CF₃-Phenyl | |
| 243 | " | " | " | 2-CH₃-Phenyl | |
| 244 | " | " | " | 4-CH₃-Phenyl | |
| 245 | " | " | " | 2,4-(CH₃)₂-Phenyl | |
| 246 | " | " | " | 2,6-(CH₃)₂-Phenyl | |
| 247 | " | " | " | 2,4,6-(CH₃)₃-Phenyl | |
| 248 | " | " | " | 2-CH₃O-Phenyl | |
| 249 | " | " | " | 4-CH₃O-Phenyl | |
| 250 | " | " | " | 4-C₂H₅O-Phenyl | |
| 251 | " | " | " | 4-CF₃O-Phenyl | |
| 252 | " | " | " | 4-CN-Phenyl | |
| 253 | " | " | " | 3-NO₂-Phenyl | |
| 254 | " | " | " | 4-NO₂-Phenyl | |
| 255 | CH₃ | H | H | CH₃ | |
| 256 | " | " | " | C₂H₅ | |
| 257 | " | " | " | n-C₃H₇ | |
| 258 | " | " | " | i-C₃H₇ | |
| 259 | " | " | " | n-C₄H₉ | |
| 260 | " | " | " | i-C₄H₉ | |
| 261 | " | " | " | s-C₄H₉ | |
| 262 | " | " | " | t-C₄H₉ | |
| 263 | " | " | " | n-C₆H₁₃ | |
| 264 | " | " | " | CH₂-t-Bu | |
| 265 | " | " | " | CF₃ | |
| 266 | " | " | " | C₂F₅ | NMR |
| 267 | " | " | " | n-C₃F₇ | |
| 268 | " | " | " | n-C₄F₉ | |
| 269 | " | " | " | n-C₅F₁₁ | |
| 270 | " | " | " | n-C₆F₁₃ | |
| 271 | " | " | " | Phenyl | |
| 272 | " | " | " | 2-F-Phenyl | |
| 273 | " | " | " | 3-F-Phenyl | |
| 274 | " | " | " | 4-F-Phenyl | |
| 275 | " | " | " | 2-Cl-Phenyl | |
| 276 | " | " | " | 3-Cl-Phenyl | |
| 277 | " | " | " | 4-Cl-Phenyl | |
| 278 | " | " | " | 2,4-Cl₂-Phenyl | |
| 279 | " | " | " | 3,4-Cl₂-Phenyl | |
| 280 | " " | | " | 2,5-Cl₂-Phenyl | |
| 281 | " | " | " | 2,6-Cl₂-Phenyl | |
| 282 | " | " | " | 2-CF₃-Phenyl | |
| 283 | " | " | " | 3-CF₃-Phenyl | |
| 284 | " | " | " | 3,5-(CF₃)₂-Phenyl | |
| 285 | " | " | " | 4-CF₃-Phenyl | |
| 286 | " | " | " | 2-CH₃-Phenyl | |
| 287 | " | " | " | 4-CH₃-Phenyl | |
| 288 | " | " | " | 2,4-(CH₃)₂-Phenyl | |
| 289 | " | " | " | 2,6-(CH₃)₂-Phenyl | |
| 290 | " | " | " | 2,4,6-(CH₃)₃-Phenyl | |
| 291 | " | " | " | 2-CH₃O-Phenyl | |
| 292 | " | " | " | 4-CH₃O-Phenyl | |
| 293 | " | " | " | 4-C₂H₅O-Phenyl | |
| 294 | " | " | " | 4-CF₃O-Phenyl | |
| 295 | " | " | " | 4-CN-Phenyl | |
| 296 | " | " | " | 3-NO₂-Phenyl | |
| 297 | " | " | " | 4-NO₂-Phenyl | |
| 298 | Br | H | H | CH₃ | |
| 299 | " | " | " | C₂H₅ | |
| 300 | " | " | " | n-C₃H₇ | |
| 301 | " | " | " | i-C₃H₇ | |
| 302 | " | " | " | n-C₄H₉ | |
| 303 | " | " | " | i-C₄H₉ | |
| 304 | " | " | " | s-C₄H₉ | |
| 305 | " | " | " | t-C₄H₉ | NMR |
| 306 | " | " | " | n-C₆H₁₃ | |
| 307 | " | " | " | CH₂-t-Bu | |
| 308 | " | " | " | CF₃ | |
| 309 | " | " | " | C₂F₅ | |
| 310 | " | " | " | n-C₃F₇ | |
| 311 | " | " | " | n-C₄F₉ | NMR |
| 312 | " | " | " | n-C₅F₁₁ | |
| 313 | " | " | " | n-C₆F₁₃ | NMR |
| 314 | " | " | " | Phenyl | |
| 315 | " | " | " | 2-F-Phenyl | |
| 316 | " | " | " | 3-F-Phenyl | |
| 317 | " | " | " | 4-F-Phenyl | |
| 318 | " | " | " | 2-Cl-Phenyl | |
| 319 | " | " | " | 3-Cl-Phenyl | |
| 320 | " | " | " | 4-Cl-Phenyl | |
| 321 | " | " | " | 2,4-Cl₂-Phenyl | |
| 322 | " | " | " | 3,4-Cl₂-Phenyl | |
| 323 | " | " | " | 2,5-Cl₂-Phenyl | |
| 324 | " | " | " | 2,6-Cl₂-Phenyl | |
| 325 | " | " | " | 2-CF₃-Phenyl | |
| 326 | " | " | " | 3-CF₃-Phenyl | |
| 327 | " | " | " | 3,5-(CF₃)₂-Phenyl | |
| 328 | " | " | " | 4-CF₃-Phenyl | NMR |
| 329 | " | " | " | 2-CH₃-Phenyl | |
| 330 | " | " | " | 4-CH₃-Phenyl | |
| 331 | " | " | " | 2,4-(CH₃)₂-Phenyl | |
| 332 | " | " | " | 2,6-(CH₃)₂-Phenyl | |
| 333 | " | " | " | 2,4,6-(CH₃)₃-Phenyl | |
| 334 | " | " | " | 2-CH₃O-Phenyl | NMR |
| 335 | " | " | " | 4-CH₃O-Phenyl | |
| 336 | " | " | " | 4-C₂H₅O-Phenyl | |
| 337 | " | " | " | 4-CF₃O-Phenyl | |
| 338 | " | " | " | 4-CN-Phenyl | |
| 339 | " | " | " | 3-NO₂-Phenyl | |
| 340 | " | " | " | 4-NO₂-Phenyl | |
| 341 | CH₃ | CH₃ | H | CH₃ | |
| 342 | " | " | " | C₂H₅ | |
| 343 | " | " | " | n-C₃H₇ | |
| 344 | " | " | " | i-C₃H₇ | |
| 345 | " | " | " | n-C₄H₉ | |
| 346 | " | " | " | i-C₄H₉ | |
| 347 | " | " | " | S-C₄H₉ | |
| 348 | " | " | " | t-C₄H₉ | |
| 349 | " | " | " | n-C₆H₁₃ | |
| 350 | " | " | " | CH₂-t-Bu | |
| 351 | " | " | " | CF₃ | |
| 352 | " | " | " | C₂F₅ | |
| 353 | " | " | " | n-C₃F₇ | |
| 354 | " | " | " | n-C₄F₉ | NMR |
| 355 | " | " | " | n-C₅F₁₁ | |
| 356 | " | " | | n-C₆F₁₃ | NMR |
| 357 | " | " | " | Phenyl | |
| 358 | " | " | " | 2-F-Phenyl | |
| 359 | " | " | " | 3-F-Phenyl | |
| 360 | " | " | " | 4-F-Phenyl | |
| 361 | " | " | " | 2-Cl-Phenyl | |
| 362 | " | " | " | 3-Cl-Phenyl | |
| 363 | " | " | " | 4-Cl-Phenyl | NMR |
| 364 | " | " | " | 2,4-Cl₂-Phenyl | |
| 365 | " | " | " | 3,4-Cl₂-Phenyl | |
| 366 | " | " | " | 2,5-Cl₂-Phenyl | |
| 367 | " | " | " | 2,6-Cl₂-Phenyl | |
| 368 | " | " | " | 2-CF₃-Phenyl | |
| 369 | " | " | " | 3-CF₃-Phenyl | |
| 370 | " | " | " | 3,5-(CF₃)₂-Phenyl | |
| 371 | " | " | " | 4-CF₃-Phenyl | |
| 372 | " | " | " | 2-CH₃-Phenyl | |
| 373 | " | " | " | 4-CH₃-Phenyl | |
| 374 | " | " | " | 2,4-(CH₃)₂-Phenyl | |
| 375 | " | " | " | 2,6-(CH₃)₂-Phenyl | |
| 376 | " | " | " | 2,4,6-(CH₃)₃-Phenyl | |
| 378 | " | " | " | 2-CH₃O-Phenyl | |
| 379 | " | " | " | 4-CH₃O-Phenyl | |
| 380 | " | " | " | 4-C₂H₅O-Phenyl | |
| 381 | " | " | " | 4-CF₃O-Phenyl | |
| 382 | " | " | " | 4-CN-Phenyl | |
| 383 | " | " | " | 3-NO₂-Phenyl | |
| 384 | " | " | " | 4-NO₂-Phenyl | |

**Tabelle 2 Oxazoline, Pyrroline und Imidazoline der Formel (I), G = 3-Isoxazolinyl**

| | | | | | |
|---|---|---|---|---|---|
| 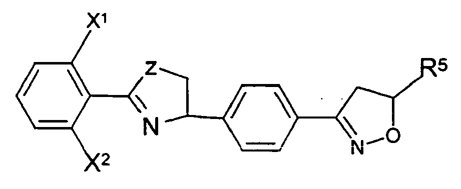 | | | | | |

| **Bsp.-Nr.** | **X**^{**1**} | **X**^{**2**} | **Z** | **R**^{**5**} | **phys. Daten** |
|---|---|---|---|---|---|
| 385 | F | F | CH₂ | CH₃ | |
| 386 | " | " | " | C₂H₅ | |
| 387 | " | " | " | n-C₃H₇ | |
| 388 | " | " | " | i-C₃H₇ | |
| 389 | " | " | " | n-C₄H₉ | |
| 390 | " | " | " | i-C₄H₉ | |
| 400 | " | " | " | S-C₄H₉ | |
| 401 | " | " | " | t-C₄H₉ | |
| 402 | " | " | " | n-C₆H₁₃ | |
| 403 | " | " | " | CH₂-t-Bu | |
| 404 | " | " | " | CF₃ | |
| 405 | " | " | " | C₂F₅ | |
| 406 | " | " | " | n-C₃F₇ | |
| 407 | " | " | " | n-C₄F₉ | |
| 408 | " | " | " | n-C₅F₁₁ | |
| 409 | " | " | " | n-C₆F₁₃ | |
| 410 | " | " | " | Phenyl | |
| 411 | " | " | " | 2-F-Phenyl | |
| 412 | " | " | " | 3-F-Phenyl | |
| 413 | " | " | " | 4-F-Phenyl | |
| 414 | " | " | " | 2-Cl-Phenyl | |
| 415 | " | " | " | 3-Cl-Phenyl | |
| 416 | " | " | " | 4-Cl-Phenyl | |
| 417 | " | " | " | 2,4-Cl₂-Phenyl | |
| 418 | " | " | " | 3,4-Cl₂-Phenyl | |
| 419 | " | " | " | 2,5-Cl₂-Phenyl | |
| 420 | " | " | " | 2,6-Cl₂-Phenyl | |
| 421 | " | " | " | 2-CF₃-Phenyl | |
| 422 | " | " | " | 3-CF₃-Phenyl | |
| 423 | " | " | " | 3,5-(CF₃)₂-Phenyl | |
| 424 | " | " | " | 4-CF₃-Phenyl | |
| 425 | " | " | " | 2-CH₃-Phenyl | |
| 426 | " | " | " | 4-CH₃-Phenyl | |
| 427 | " | " | " | 2,4-(CH₃)₂-Phenyl | |
| 428 | " | " | " | 2,6-(CH₃)₂-Phenyl | |
| 429 | " | " | " | 2,4,6-(CH₃)₃-Phenyl | |
| 430 | " | " | " | 2-CH₃O-Phenyl | |
| 431 | " | " | " | 4-CH₃O-Phenyl | |
| 432 | " | " | " | 4-C₂H₅O-Phenyl | |
| 433 | " | " | " | 4-CF₃O-Phenyl | |
| 434 | " | " | " | 4-CN-Phenyl | |
| 435 | " | " | " | 3-NO₂-Phenyl | |
| 436 | " | " | " | 4-NO₂-Phenyl | |
| 437 | F | H | CH₂ | CH₃ | |
| 438 | " | " | " | C₂H₅ | |
| 439 | " | " | " | n-C₃H₇ | |
| 440 | " | " | " | i-C₃H₇ | |
| 441 | " | " | " | n-C₄H₉ | |
| 442 | " | " | " | i-C₄H₉ | |
| 443 | " | " | " | s-C₄H₉ | |
| 444 | " | " | " | t-C₄H₉ | |
| 445 | " | " | " | n-C₆H₁₃ | |
| 446 | " | " | " | CH₂-t-Bu | |
| 447 | " | " | " | CF₃ | |
| 448 | " | " | " | C₂F₅ | |
| 449 | " | " | " | n-C₃F₇ | |
| 450 | " | " | " | n-C₄F₉ | |
| 451 | " | " | " | n-C₅F₁₁ | |
| 452 | " | " | " | n-C₆F₁₃ | |
| 453 | " | " | " | Phenyl | |
| 454 | " | " | " | 2-F-Phenyl | |
| 455 | " | " | " | 3-F-Phenyl | |
| 456 | " | " | " | 4-F-Phenyl | |
| 457 | " | " | " | 2-Cl-Phenyl | |
| 458 | " | " | " | 3-Cl-Phenyl | |
| 459 | " | " | " | 4-Cl-Phenyl | |
| 460 | " | " | " | 2,4-Cl₂-Phenyl | |
| 461 | " | " | " | 3,4-Cl₂-Phenyl | |
| 462 | " | " | " | 2,5-Cl₂-Phenyl | |
| 463 | " | " | " | 2,6-Cl₂-Phenyl | |
| 464 | " | " | " | 2-CF₃-Phenyl | |
| 465 | " | " | " | 3-CF₃-Phenyl | |
| 466 | " | " | " | 3,5-(CF₃)₂-Phenyl | |
| 467 | " | " | " | 4-CF₃-Phenyl | |
| 468 | " | " | " | 2-CH₃-Phenyl | |
| 469 | " | " | " | 4-CH₃-Phenyl | |
| 470 | " | " | " | 2,4-(CH₃)₂-Phenyl | |
| 471 | " | " | " | 2,6-(CH₃)₂-Phenyl | |
| 472 | " | " | " | 2,4,6-(CH₃)₃-Phenyl | |
| 473 | " | " | " | 2-CH₃O-Phenyl | |
| 474 | " | " | " | 4-CH₃O-Phenyl | |
| 475 | " | " | " | 4-C₂H₅O-Phenyl | |
| 476 | " | " | " | 4-CF₃O-Phenyl | |
| 477 | " | " | " | 4-CN-Phenyl | |
| 478 | " | " | " | 3-NO₂-Phenyl | |
| 479 | " | " | " | 4-NO₂-Phenyl | |
| 480 | F | Cl | CH₂ | CH₃ | |
| 481 | " | " | " | C₂H₅ | |
| 482 | " | " | " | n-C₃H₇ | |
| 483 | " | " | " | i-C₃H₇ | |
| 484 | " | " | " | n-C₄H₉ | |
| 485 | " | " | " | i-C₄H₉ | |
| 486 | " | " | " | s-C₄H₉ | |
| 487 | " | " | " | t-C₄H₉ | |
| 488 | " | " | " | n-C₆H₁₃ | |
| 489 | " | " | " | CH₂-t-Bu | |
| 490 | " | " | " | CF₃ | |
| 491 | " | " | " | C₂F₅ | |
| 492 | " | " | " | n-C₃F₇ | |
| 493 | " | " | " | n-C₄F₉ | |
| 494 | " | " | " | n-C₅F₁₁ | |
| 495 | " | " | " | n-C₆F₁₃ | |
| 496 | " | " | " | Phenyl | |
| 497 | " | " | " | 2-F-Phenyl | |
| 498 | " | " | " | 3-F-Phenyl | |
| 499 | " | " | " | 4-F-Phenyl | |
| 500 | " | " | " | 2-Cl-Phenyl | |
| 501 | " | " | " | 3-Cl-Phenyl | |
| 502 | " | " | " | 4-Cl-Phenyl | |
| 503 | " | " | " | 2,4-Cl₂-Phenyl | |
| 504 | " | " | " | 3,4-Cl₂-Phenyl | |
| 505 | " | " | " | 2,5-Cl₂-Phenyl | |
| 506 | " | " | " | 2,6-Cl₂-Phenyl | |
| 507 | " | " | " | 2-CF₃-Phenyl | |
| 508 | " | " | " | 3-CF₃-Phenyl | |
| 509 | " | " | " | 3,5-(CF₃)₂-Phenyl | |
| 510 | " | " | " | 4-CF₃-Phenyl | |
| 511 | " | " | " | 2-CH₃-Phenyl | |
| 512 | " | " | " | 4-CH₃-Phenyl | |
| 513 | " | " | " | 2,4-(CH₃)₂-Phenyl | |
| 514 | " | " | " | 2,6-(CH₃)₂-Phenyl | |
| 515 | " | " | " | 2,4,6-(CH₃)₃-Phenyl | |
| 516 | " | " | " | 2-CH₃O-Phenyl | |
| 517 | " | " | " | 4-CH₃O-Phenyl | |
| 518 | " | " | " | 4-C₂H₅O-Phenyl | |
| 519 | " | " | " | 4-CF₃O-Phenyl | |
| 520 | " | " | " | 4-CN-Phenyl | |
| 521 | " | " | " | 3-NO₂-Phenyl | |
| 522 | " | " | " | 4-NO₂-Phenyl | |
| 523 | F | F | NCOOEt | CH₃ | |
| 524 | " | " | " | C₂H₅ | |
| 525 | " | " | " | n-C₃H₇ | |
| 526 | " | " | " | i-C₃H₇ | |
| 527 | " | " | " | n-C₄H₉ | |
| 528 | " | " | " | i-C₄H₉ | |
| 529 | " | " | " | s-C₄H₉ | |
| 530 | " | " | " | t-C₄H₉ | |
| 531 | " | " | " | n-C₆H₁₃ | |
| 532 | " | " | " | CH₂-t-Bu | |
| 533 | " | " | " | CF₃ | |
| 534 | " | " | " | C₂F₅ | |
| 535 | " | " | " | n-C₃F₇ | |
| 536 | " | " | " | n-C₄F₉ | |
| 537 | " | " | " | n-C₅F₁₁ | |
| 538 | " | " | " | n-C₆F₁₃ | |
| 539 | " | " | " | Phenyl | |
| 540 | " | " | " | 2-F-Phenyl | |
| 541 " | " | " | " | 3-F-Phenyl | |
| 542 | " | " | " | 4-F-Phenyl | |
| 543 | " | " | " | 2-Cl-Phenyl | |
| 544 | " | " | " | 3-Cl-Phenyl | |
| 545 | " | " | " | 4-Cl-Phenyl | |
| 546 | " | " | " | 2,4-Cl₂-Phenyl | |
| 547 | " | " | " | 3,4-Cl₂-Phenyl | |
| 548 | " | " | " | 2,5-Cl₂-Phenyl | |
| 549 | " | " | " | 2,6-Cl₂-Phenyl | |
| 550 | " | " | " | 2-CF₃-Phenyl | |
| 551 | " | " | " | 3-CF₃-Phenyl | |
| 552 | " | " | " | 3,5-(CF₃)₂-Phenyl | |
| 553 | " | " | " | 4-CF₃-Phenyl | |
| 554 | " | " | " | 2-CH₃-Phenyl | |
| 555 | " | " | " | 4-CH₃-Phenyl | |
| 556 | " | " | " | 2,4-(CH₃)₂-Phenyl | |
| 557 | " | " | " | 2,6-(CH₃)₂-Phenyl | |
| 558 | " | " | " | 2,4,6-(CH₃)₃-Phenyl | |
| 559 | " | " | " | 2-CH₃O-Phenyl | |
| 560 | " | " | " | 4-CH₃O-Phenyl | |
| 561 | " | " | " | 4-C₂H₅O-Phenyl | |
| 562 | " | " | " | 4-CF₃O-Phenyl | |
| 563 | " | " | " | 4-CN-Phenyl | |
| 564 | " | " | " | 3-NO₂-Phenyl | |
| 565 | " | " | " | 4-NO₂-Phenyl | |

**Tabelle 3 Oxazoline der Formel (I), Z= O, G = 5-Isoxazolinyl**

| | | | | |
|---|---|---|---|---|
| 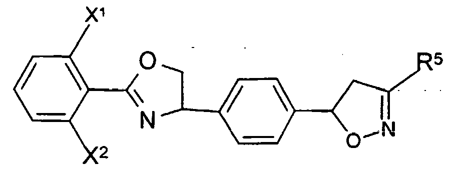 | | | | |

| **Bsp.-Nr.** | **X**^{**1**} | **X**^{**2**} | **R**^{**5**} | **phys. Daten** |
|---|---|---|---|---|
| 566 | F | F | CH₃ | NMR |
| 567 | " | " | C₂H₅ | NMR |
| 568 | " | " | n-C₃H₇ | NMR |
| 569 | " | " | i-C₃H₇ | NMR |
| 570 | " | " | n-C₄H₉ | NMR |
| 571 | " | " | i-C₄H₉ | |
| 572 | " | " | S-C₄H₉ | |
| 573 | " | " | t-C₄H₉ | NMR |
| 574 | " | " | n-C₅H₁₁ | NMR |
| 575 | " | " | n-C₆H₁₃ | |
| 576 | " | " | CH(C₂H₅)₂ | NMR |
| 577 | " | " | CH₂-t-Bu | |
| 578 | " | " | CH₂CF₃ | NMR |
| 579 | " | " | C₂H₄CF₃ | NMR |
| 580 | " | " | CF₃ | |
| 581 | " | " | C₂F₅ | |
| 582 | " | " | n-C₃F₇ | |
| 583 | " | " | n-C₄F₉ | |
| 584 | " | " | n-C₅F₁₁ | |
| 585 | " | " | n-C₆F₁₃ | |
| 586 | " | " | Phenyl | |
| 587 | " | " | 2-F-Phenyl | |
| 588 | " | " | 3-F-Phenyl | |
| 589 | " | " | 4-F-Phenyl | NMR |
| 590 | " | " | 2-Cl-Phenyl | |
| 591 | " | " | 3-Cl-Phenyl | |
| 592 | " | " | 4-Cl-Phenyl | NMR |
| 593 | " | " | 2,4-Cl₂-Phenyl | |
| 594 | " | " | 3,4-Cl₂-Phenyl | |
| 595 | " | " | 2,5-Cl₂-Phenyl | |
| 596 | " | " | 2,6-Cl₂-Phenyl | |
| 597 | " | " | 2-CF₃-Phenyl | |
| 598 | " | " | 3-CF₃-Phenyl | |
| 599 | " | " | 3,5-(CF₃)₂-Phenyl | |
| 600 | " | " | 4-CF₃-Phenyl | NMR |
| 601 | " | " | 2-CH₃-Phenyl | |
| 602 | " | " | 4-CH₃-Phenyl | |
| 603 | " | " | 2,4-(CH₃)₂-Phenyl | |
| 604 | " | " | 2,6-(CH₃)₂-Phenyl | |
| 605 | " | " | 2,4,6-(CH₃)₃-Phenyl | NMR |
| 606 | " | " | 2-CH₃O-Phenyl | |
| 607 | " | " | 4-CH₃O-Phenyl | |
| 608 | " | " | 4-C₂H₅O-Phenyl | |
| 609 | " | " | 4-CF₃O-Phenyl | |
| 610 | " | " | 4-CN-Phenyl | |
| 611 | " | " | 3-NO₂-Phenyl | |
| 612 | " | " | 4-NO₂-Phenyl | |
| 613 | " | " | COOH | NMR |
| 614 | " | " | COOC₂H₅ | NMR |
| 615 | " | " | COOCH₂CF₃ | NMR |
| 616 | " | " | COOC₂H₄CF₃ | NMR |
| 617 | " | " | CONH₂ | |
| 618 | " | " | CONHCH₃ | |
| 619 | " | " | CONHC₂H₅ | NMR |
| 620 | " | " | CON(CH₃)₂ | |
| 621 | " | " | CON(C₂H₅)₂ | NMR |
| 622 | " | " | CONH(n-C₃H₇) | NMR |
| 623 | " | " | CONHCH₂C₂F₅ | NMR |
| 624 | " | " | CONH-t-C₄H₉ | NMR |
| 625 | " | " | CONHCH₂C₂H₃ | NMR |
| 626 | " | " | CONHCH₂C₃F₇ | NMR |
| 627 | " | " | CONH-s-C₅H₁₁ | NMR |
| 628 | " | " | CONHCH₂CF₃ | NMR |
| 629 | " | " | CONHC₃H₆OCH₃ | NMR |
| 630 | " | " | CONHCH₂-(2-tetrahydrofuranyl) | NMR |
| 631 | " | " | CONHCH₂-(2,6-F₂-Phenyl) | NMR |
| 632 | " | " | CONHCH₂-(4-F-Phenyl) | |
| 633 | " | " | CONHCH₂-(3-CF₃-Phenyl) | NMR |
| 634 | " | " | CONHCH₂-(4-CF₃-Phenyl) | |
| 635 | " | " | CONH(2,5-F₂-Phenyl) | |
| 636 | " | " | CONH(4-F-phenyl) | |
| 637 | " | " | CONH(3-CF₃-Phenyl) | |
| 638 | " | " | CONH(4-CF₃-phenyl) | |
| 639 | F | H | CH₃ | |
| 640 | " | " | C₂H₅ | |
| 641 | " | " | n-C₃H₇ | |
| 642 | " | " | i-C₃H₇ | |
| 643 | " | " | n-C₄H₉ | |
| 644 | " | " | i-C₄H₉ | |
| 645 | " | " | s-C₄H₉ | |
| 646 | " | " | t-C₄H₉ | |
| 647 | " | " | n-C₅H₁₁ | |
| 648 | " | " | n-C₆H₁₃ | |
| 649 | " | " | CH(C₂H₅)₂ | |
| 650 | " | " | CH₂-t-Bu | |
| 651 | " | " | CH₂CF₃ | |
| 652 | " | " | C₂H₄CF₃ | |
| 653 | " | " | CF₃ | |
| 654 | " | " | C₂F₅ | |
| 655 | " | " | n-C₃F₇ | |
| 656 | " | " | n-C₄F₉ | |
| 657 | " | " | n-C₅F₁₁ | |
| 658 | " | " | n-C₆F₁₃ | |
| 659 | " | " | Phenyl | |
| 660 | " | " | 2-F-Phenyl | |
| 661 | " | " | 3-F-Phenyl | |
| 662 | " | " | 4-F-Phenyl | |
| 663 | " | " | 2-Cl-Phenyl | |
| 664 | " | " | 3-Cl-Phenyl | |
| 665 | " | " | 4-Cl-Phenyl | |
| 666 | " | " | 2,4-Cl₂-Phenyl | |
| 667 | " | " | 3,4-Cl₂-Phenyl | |
| 668 | " | " | 2,5-Cl₂-Phenyl | |
| 669 | " | " | 2,6-Cl₂-Phenyl | |
| 670 | " | " | 2-CF₃-Phenyl | |
| 671 | " | " | 3-CF₃-Phenyl | |
| 672 | " | " | 3,5-(CF₃)₂-Phenyl | |
| 673 | " | " | 4-CF₃-Phenyl | |
| 674 | " | " | 2-CH₃-Phenyl | |
| 675 | " | " | 4-CH₃-Phenyl | |
| 676 | " | " | 2,4-(CH₃)₂-Phenyl | |
| 677 | " | " | 2,6-(CH₃)₂-Phenyl | |
| 678 | " | " | 2,4,6-(CH₃)₃-Phenyl | |
| 679 | " | " | 2-CH₃O-Phenyl | |
| 680 | " | " | 4-CH₃O-Phenyl | |
| 681 | " | " | 4-C₂H₅O-Phenyl | |
| 682 | " | " | 4-CF₃O-Phenyl | |
| 683 | " | " | 4-CN-Phenyl | |
| 684 | " | " | 3-NO₂-Phenyl | |
| 685 | " | " | 4-NO₂-Phenyl | |
| 686 | F | H | CH₃ | |
| 687 | " | " | C₂H₅ | |
| 688 | " | " | n-C₃H₇ | |
| 689 | " | " | i-C₃H₇ | |
| 690 | " | " | n-C₄H₉ | |
| 691 | " | " | i-C₄H₉ | |
| 692 | " | " | s-C₄H₉ | |
| 693 | " | " | t-C₄H₉ | |
| 694 | " | " | n-C₅H₁₁ | |
| 695 | " | " | n-C₆H₁₃ | |
| 696 | " | " | CH(C₂H₅)₂ | |
| 697 | " | " | CH₂-t-Bu | |
| 698 | " | " | CH₂CF₃ | |
| 699 | " | " | C₂H₄CF₃ | |
| 700 | " | " | CF₃ | |
| 701 | " | " | C₂F₅ | |
| 702 | " | " | n-C₃F₇ | |
| 703 | " | " | n-C₄F₉ | |
| 704 | " | " | n-C₅F₁₁ | |
| 705 | " | " | n-C₆F₁₃ | |
| 706 | " | " | Phenyl | |
| 707 | " | " | 2-F-Phenyl | |
| 708 | " | " | 3-F-Phenyl | |
| 709 | " | " | 4-F-Phenyl | |
| 710 | " | " | 2-Cl-Phenyl | |
| 711 | " | " | 3-Cl-Phenyl | |
| 712 | " | " | 4-Cl-Phenyl | |
| 713 | " | " | 2,4-Cl₂-Phenyl | |
| 714 | " | " | 3,4-Cl₂-Phenyl | |
| 715 | " | " | 2,5-Cl₂-Phenyl | |
| 716 | " | " | 2,6-Cl₂-Phenyl | |
| 717 | " | " | 2-CF₃-Phenyl | |
| 718 | " | " | 3-CF₃-Phenyl | |
| 719 | " | " | 3,5-(CF₃)₂-Phenyl | |
| 720 | " | " | 4-CF₃-Phenyl | |
| 721 | " | " | 2-CH₃-Phenyl | |
| 722 | " | " | 4-CH₃-Phenyl | |
| 723 | " | " | 2,4-(CH₃)₂-Phenyl | |
| 724 | " | " | 2,6-(CH₃)₂-Phenyl | |
| 725 | " | " | 2,4,6-(CH₃)₃-Phenyl | |
| 726 | " | " | 2-CH₃O-Phenyl | |
| 727 | " | " | 4-CH₃O-Phenyl | |
| 728 | " | " | 4-C₂H₅O-Phenyl | |
| 729 | " | " | 4-CF₃O-Phenyl | |
| 730 | " | " | 4-CN-Phenyl | |
| 731 | " | " | 3-NO₂-Phenyl | |
| 732 | " | " | 4-NO₂-Phenyl | |
| 733 | Cl | H | CH₃ | |
| 734 | " | " | C₂H₅ | |
| 735 | " | " | n-C₃H₇ | |
| 736 | " | " | i-C₃H₇ | |
| 737 | " | " | n-C₄H₉ | |
| 738 | " | " | i-C₄H₉ | |
| 739 | " | " | s-C₄H₉ | |
| 740 | " | " | t-C₄H₉ | |
| 741 | " | " | n-C₅H₁₃ | |
| 742 | " | " | n-C₆H₁₃ | |
| 743 | " | " | CH(C₂H₅)₂ | |
| 744 | " | " | CH₂-t-Bu | |
| 745 | " | " | CH₂CF₃ | |
| 746 | " | " | C₂H₄CF₃ | |
| 747 | " | " | CF₃ | |
| 748 | " | " | C₂F₅ | |
| 749 | " | " | n-C₃F₇ | |
| 750 | " | " | n-C₄F₉ | |
| 751 | " | " | n-C₅F₁₁ | |
| 752 | " | " | n-C₆F₁₃ | |
| 753 | " | " | Phenyl | |
| 754 | " | " | 2-F-Phenyl | |
| 755 | " | " | 3-F-Phenyl | |
| 756 | " | " | 4-F-Phenyl | |
| 757 | " | " | 2-Cl-Phenyl | |
| 758 | " | " | 3-Cl-Phenyl | |
| 759 | " | " | 4-Cl-Phenyl | |
| 760 | " | " | 2,4-Cl₂-Phenyl | |
| 761 | " | " | 3,4-Cl₂-Phenyl | |
| 762 | " | " | 2,5-Cl₂-Phenyl | |
| 763 | " | " | 2,6-Cl₂-Phenyl | |
| 764 | " | " | 2-CF₃-Phenyl | |
| 765 | " | " | 3-CF₃-Phenyl | |
| 767 | " | " | 3,5-(CF₃)₂-Phenyl | |
| 768 | " | " | 4-CF₃-Phenyl | |
| 769 | " | " | 2-CH₃-Phenyl | |
| 770 | " | " | 4-CH₃-Phenyl | |
| 771 | " | " | 2,4-(CH₃)₂-Phenyl | |
| 772 | " | " | 2,6-(CH₃)₂-Phenyl | |
| 773 | " | " | 2,4,6-(CH₃)₃-Phenyl | |
| 774 | " | " | 2-CH₃O-Phenyl | |
| 776 | " | " | 4-CH₃O-Phenyl | |
| 777 | " | " | 4-C₂H₅O-Phenyl | |
| 778 | " | " | 4-CF₃O-Phenyl | |
| 779 | " | " | 4-CN-Phenyl | |
| 780 | " | " | 3-NO₂-Phenyl | |
| 781 | " | " | 4-NO₂-Phenyl | |
| 782 | CH₃ | H | CH₃ | |
| 783 | " | " | C₂H₅ | |
| 784 | " | " | n-C₃H₇ | |
| 785 | " | " | i-C₃H₇ | |
| 786 | " | " | n-C₄H₉ | |
| 787 | " | " | i-C₄H₉ | |
| 788 | " | " | S-C₄H₉ | |
| 789 | " | " | t-C₄H₉ | |
| 790 | " | " | n-C₅H₁₁ | |
| 791 | " | " | n-C₆H₁₃ | |
| 792 | " | " | CH(C₂H₅)₂ | |
| 793 | " | " | CH₂-t-Bu | |
| 794 | " | " | CH₂CF₃ | |
| 795 | " | " | C₂H₄CF₃ | |
| 796 | " | " | CF₃ | |
| 797 | " | " | C₂F₅ | |
| 798 | " | " | n-C₃F₇ | |
| 799 | " | " | n-C₄F₉ | |
| 800 | " | " | n-C₅F₁₁ | |
| 801 | " | " | n-C₆F₁₃ | |
| 802 | " | " | Phenyl | |
| 803 | " | " | 2-F-Phenyl | |
| 804 | " | " | 3-F-Phenyl | |
| 805 | " | " | 4-F-Phenyl | |
| 806 | " | " | 2-Cl-Phenyl | |
| 807 | " | " | 3-Cl-Phenyl | |
| 808 | " | " | 4-Cl-Phenyl | |
| 809 | " | " | 2,4-Cl₂-Phenyl | |
| 810 | " | " | 3,4-Cl₂-Phenyl | |
| 811 | " | " | 2,5-Cl₂-Phenyl | |
| 812 | " | " | 2,6-Cl₂-Phenyl | |
| 813 | " | " | 2-CF₃-Phenyl | |
| 814 | " | " | 3-CF₃-Phenyl | |
| 815 | " | " | 3,5-(CF₃)₂-Phenyl | |
| 816 | " | " | 4-CF₃-Phenyl | |
| 817 | " | " | 2-CH₃-Phenyl | |
| 818 | " | " | 4-CH₃-Phenyl | |
| 819 | " | " | 2,4-(CH₃)₂-Phenyl | |
| 820 | " | " | 2,6-(CH₃)₂-Phenyl | |
| 821 | " | " | 2,4,6-(CH₃)₃-Phenyl | |
| 822 | " | " | 2-CH₃O-Phenyl | |
| 823 | " | " | 4-CH₃O-Phenyl | |
| 824 | " | " | 4-C₂H₅O-Phenyl | |
| 825 | " | " | 4-CF₃O-Phenyl | |
| 826 | " | " | 4-CN-Phenyl | |
| 827 | " | " | 3-NO₂-Phenyl | |
| 828 | " | " | 4-NO₂-Phenyl | |
| 829 | Br | H | CH₃ | |
| 830 | " | " | C₂H₅ | |
| 831 | " | " | " n-C₃H₇ | |
| 832 | " | " | i-C₃H₇ | |
| 833 | " | " | n-C₄H₉ | |
| 834 | " | " | i-C₄H₉ | |
| 835 | " | " | s-C₄H₉ | |
| 836 | " | " | t-C₄H₉ | |
| 837 | " | " | n-C₅H₁₁ | |
| 838 | " | " | n-C₆H₁₃ | |
| 839 | " | " | CH(C₂H₅)₂ | |
| 840 | " | " | CH₂-t-Bu | |
| 841 | " | " | CH₂CF₃ | |
| 842 | " | " | C₂H₄CF₃ | |
| 843 | " | " | CF₃ | |
| 844 | " | " | C₂F₅ | |
| 845 | " | " | n-C₃F₇ | |
| 846 | " | " | n-C₄F₉ | |
| 847 | " | " | n-C₅F₁₁ | |
| 848 | " | " | n-C₆F₁₃ | |
| 849 | " | " | Phenyl | |
| 850 | " | " | 2-F-Phenyl | |
| 851 | " | " | 3-F-Phenyl | |
| 852 | " | " | 4-F-Phenyl | |
| 853 | " | " | 2-Cl-Phenyl | |
| 854 | " | " | 3-Cl-Phenyl | |
| 855 | " | " | 4-Cl-Phenyl | |
| 856 | " | " | 2,4-Cl₂-Phenyl | |
| 857 | " | " | 3,4-Cl₂-Phenyl | |
| 858 | " | " | 2,5-Cl₂-Phenyl | |
| 859 | " | " | 2,6-Cl₂-Phenyl | |
| 860 | " | " | 2-CF₃-Phenyl | |
| 861 | " | " | 3-CF₃-Phenyl | |
| 862 | " | " | 3,5-(CF₃)₂-Phenyl | |
| 863 | " | " | 4-CF₃-Phenyl | |
| 864 | " | " | 2-CH₃-Phenyl | |
| 865 | " | " | 4-CH₃-Phenyl | |
| 866 | " | " | 2,4-(CH₃)₂-Phenyl | |
| 867 | " | " | 2,6-(CH₃)₂-Phenyl | |
| 868 | " | " | 2,4,6-(CH₃)₃-Phenyl | |
| 869 | " | " | 2-CH₃O-Phenyl | |
| 870 | " | " | 4-CH₃O-Phenyl | |
| 871 | " | " | 4-C₂H₅O-Phenyl | |
| 872 | " | " | 4-CF₃O-Phenyl | |
| 873 | " | " | 4-CN-Phenyl | |
| 874 | " | " | 3-NO₂-Phenyl | |
| 875 | " | " | 4-NO₂-Phenyl | |

**Tabelle 4 Pyrroline und Imidazoline der Formel (I), G = 5-lsoxazolinyl**

| | | | | | |
|---|---|---|---|---|---|
| 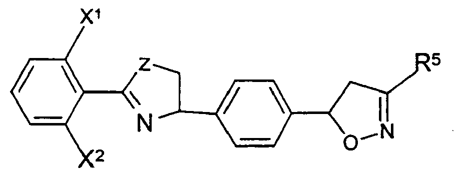 | | | | | |

| **Bsp.-Nr.** | **X**^{**1**} | **X**^{**2**} | **Z** | **R**^{**5**} | **phys. Daten** |
|---|---|---|---|---|---|
| 876 | F | F | CH₂ | CH₃ | |
| 877 | " | " | " | C₂H₅ | |
| 878 | " | " | " | n-C₃H₇ | |
| 879 | " | " | " | i-C₃H₇ | |
| 880 | " | " | " | n-C₄H₉ | |
| 881 | " | " | " | i-C₄H₉ | |
| 882 | " | " | " | s-C₄H₉ | |
| 883 | " | " | " | t-C₄H₉ | |
| 884 | " | " | " | n-C₆H₁₃ | |
| 885 | " | " | " | CH₂-t-Bu | |
| 886 | " | " | " | CF₃ | |
| 887 | " | " | " | C₂F₅ | |
| 888 | " | " | " | n-C₃F₇ | |
| 889 | " | " | " | n-C₄F₉ | |
| 890 | " | " | " | n-C₅F₁₁ | |
| 891 | " | " | " | n-C₆F₁₃ | |
| 892 | " | " | " | Phenyl | |
| 893 | " | " | " | 2-F-Phenyl | |
| 894 | " | " | " | 3-F-Phenyl | |
| 895 | " | " | " | 4-F-Phenyl | |
| 896 | " | " | " | 2-Cl-Phenyl | |
| 897 | " | " | " | 3-Cl-Phenyl | |
| 898 | " | " | " | 4-Cl-Phenyl | |
| 899 | " | " | " | 2,4-Cl₂-Phenyl | |
| 900 | " | " | " | 3,4-Cl₂-Phenyl | |
| 901 | " | " | " | 2,5-Cl₂-Phenyl | |
| 902 | " | " | " | 2,6-Cl₂-Phenyl | |
| 903 | " | " | " | 2-CF₃-Phenyl | |
| 904 | " | " | " | 3-CF₃-Phenyl | |
| 905 | " | " | " | 3,5-(CF₃)₂-Phenyl | |
| 906 | " | " | " | 4-CF₃-Phenyl | |
| 907 | " | " | " | 2-CH₃-Phenyl | |
| 908 | " | " | " | 4-CH₃-Phenyl | |
| 909 | " | " | " | 2,4-(CH₃)₂-Phenyl | |
| 910 | " | " | " | 2,6-(CH₃)₂-Phenyl | |
| 911 | " | " | " | 2,4,6-(CH₃)₃-Phenyl | |
| 912 | " | " | " | 2-CH₃O-Phenyl | |
| 913 | " | " | " | 4-CH₃O-Phenyl | |
| 914 | " | " | " | 4-C₂H₅O-Phenyl | |
| 915 | " | " | " | 4-CF₃O-Phenyl | |
| 916 | " | " | " | 4-CN-Phenyl | |
| 917 | " | " | " | 3-NO₂-Phenyl | |
| 918 | " | " | " | 4-NO₂-Phenyl | |
| 919 | F | H | CH₂ | CH₃ | |
| 920 | " | " | " | C₂H₅ | |
| 921 | " | " | " | n-C₃H₇ | |
| 922 | " | " | " | i-C₃H₇ | |
| 923 | " | " | " | n-C₄H₉ | |
| 924 | " | " | " | i-C₄H₉ | |
| 925 | " | " | " | s-C₄H₉ | |
| 926 | " | " | " | t-C₄H₉ | |
| 927 | " | " | " | n-C₆H₁₃ | |
| 928 | " | " | " | CH₂-t-Bu | |
| 929 | " | " | " | CF₃ | |
| 930 | " | " | " | C₂F₅ | |
| 931 | " | " | " | n-C₃F₇ | |
| 932 | " | " | " | n-C₄F₉ | |
| 933 | " | " | " | n-C₅F₁₁ | |
| 934 | " | " | " | n-C₆F₁₃ | |
| 935 | " | " | " | Phenyl | |
| 936 | " | " | " | 2-F-Phenyl | |
| 937 | " | " | " | 3-F-Phenyl | |
| 938 | " | " | " | 4-F-Phenyl | |
| 939 | " | " | " | 2-Cl-Phenyl | |
| 940 | " | " | " | 3-Cl-Phenyl | |
| 941 | " | " | " | 4-Cl-Phenyl | |
| 942 | " | " | " | 2,4-Cl₂-Phenyl | |
| 943 | " | " | " | 3,4-Cl₂-Phenyl | |
| 944 | " | " | " | 2,5-Cl₂-Phenyl | |
| 945 | " | " | " | 2,6-Cl₂-Phenyl | |
| 946 | " | " | " | 2-CF₃-Phenyl | |
| 947 | " | " | " | 3-CF₃-Phenyl | |
| 948 | " | " | " | 3,5-(CF₃)₂-Phenyl | |
| 949 | " | " | " | 4-CF₃-Phenyl | |
| 950 | " | " | " | 2-CH₃-Phenyl | |
| 951 | " | " | " | 4-CH₃-Phenyl | |
| 952 | " | " | " | 2,4-(CH₃)₂-Phenyl | |
| 953 | " | " | " | 2,6-(CH₃)₂-Phenyl | |
| 954 | " | " | " | 2,4,6-(CH₃)₃-Phenyl | |
| 955 | " | " | " | 2-CH₃O-Phenyl | |
| 956 | " | " | " | 4-CH₃O-Phenyl | |
| 957 | " | " | " | 4-C₂H₅O-Phenyl | |
| 958 | " | " | " | 4-CF₃O-Phenyl | |
| 959 | " | " | " | 4-CN-Phenyl | |
| 960 | " | " | " | 3-NO₂-Phenyl | |
| 961 | " | " | " | 4-NO₂-Phenyl | |
| 962 | F | Cl | CH₂ | CH₃ | |
| 963 | " | " | " | C₂H₅ | |
| 964 | " | " | " | n-C₃H₇ | |
| 965 | " | " | " | i-C₃H₇ | |
| 966 | " | " | " | n-C₄H₉ | |
| 967 | " | " | " | i-C₄H₉ | |
| 968 | " | " | " | s-C₄H₉ | |
| 969 | " | " | " | t-C₄H₉ | |
| 970 | " | " | " | n-C₆H₁₃ | |
| 071 | " | " | " | CH₂-t-Bu | |
| 972 | " | " | " | CF₃ | |
| 973 | " | " | " | C₂F₅ | |
| 974 | " | " | " | n-C₃F₇ | |
| 975 | " | " | " | n-C₄F₉ | |
| 976 | " | " | " | n-C₅F₁₁ | |
| 977 | " | " | " | n-C₆F₁₃ | |
| 978 | " | " | " | Phenyl | |
| 979 | " | " | " | 2-F-Phenyl | |
| 980 | " | " | " | 3-F-Phenyl | |
| 981 | " | " | " | 4-F-Phenyl | |
| 982 | " | " | " | 2-Cl-Phenyl | |
| 983 | " | " | " | 3-Cl-Phenyl | |
| 984 | " | " | " | 4-Cl-Phenyl | |
| 985 | " | " | " | 2,4-Cl₂-Phenyl | |
| 986 | " | " | " | 3,4-Cl₂-Phenyl | |
| 987 | " | " | " | 2,5-Cl₂-Phenyl | |
| 988 | " | " | " | 2,6-Cl₂-Phenyl | |
| 989 | " | " | " | 2-CF₃-Phenyl | |
| 990 | " | " | " | 3-CF₃-Phenyl | |
| 991 | " | " | " | 3,5-(CF₃)₂-Phenyl | |
| 992 | " | " | " | 4-CF₃-Phenyl | |
| 993 | " | " | " | 2-CH₃-Phenyl | |
| 994 | " | " | " | 4-CH₃-Phenyl | |
| 995 | " | " | " | 2,4-(CH₃)₂-Phenyl | |
| 996 | " | " | " | 2,6-(CH₃)₂-Phenyl | |
| 997 | " | " | " | 2,4,6-(CH₃)₃-Phenyl | |
| 998 | " | " | " | 2-CH₃O-Phenyl | |
| 999 | " | " | " | 4-CH₃O-Phenyl | |
| 1001 | " | " | " | 4-C₂H₅O-Phenyl | |
| 1002 | " | " | " | 4-CF₃O-Phenyl | |
| 1003 | " | " | " | 4-CN-Phenyl | |
| 1004 | " | " | " | 3-NO₂-Phenyl | |
| 1005 | " | " | " | 4-NO₂-Phenyl | |
| 1006 | F | F | NCOOEt | CH₃ | |
| 1007 | " | " | " | C₂H₅ | |
| 1008 | " | " | " | n-C₃H₇ | |
| 1009 | " | " | " | i-C₃H₇ | |
| 1010 | " | " | " | n-C₄H₉ | |
| 1011 | " | " | " | i-C₄H₉ | |
| 1012 | " | " | " | s-C₄H₉ | |
| 1013 | " | " | " | t-C₄H₉ | |
| 1014 | " | " | " | n-C₆H₁₃ | |
| 1015 | " | " | " | CH₂-t-Bu | |
| 1016 | " | " | " | CF₃ | |
| 1017 | " | " | " | C₂F₅ | |
| 1018 | " | " | " | n-C₃F₇ | |
| 1019 | " | " | " | n-C₄F₉ | |
| 1020 | " | " | " | n-C₅F₁₁ | |
| 1021 | " | " | " | n-C₆F₁₃ | |
| 1022 | " | " | " | Phenyl | |
| 1023 | " | " | " | 2-F-Phenyl | |
| 1024 | " | " | " | 3-F-Phenyl | |
| 1025 | " | " | " | 4-F-Phenyl | |
| 1026 | " | " | " | 2-Cl-Phenyl | |
| 1027 | " | " | " | 3-Cl-Phenyl | |
| 1028 | " | " | " | 4-Cl-Phenyl | |
| 1029 | " | " | " | 2,4-Cl₂-Phenyl | |
| 1030 | " | " | " | 3,4-Cl₂-Phenyl | |
| 1031 | " | " | " | 2,5-Cl₂-Phenyl | |
| 1032 | " | " | " | 2,6-Cl₂-Phenyl | |
| 1033 | " | " | " | 2-CF₃-Phenyl | |
| 1034 | " | " | " | 3-CF₃-Phenyl | |
| 1035 | " | " | " | 3,5-(CF₃)₂-Phenyl | |
| 1036 | " | " | " | 4-CF₃-Phenyl | |
| 1037 | " | " | " | 2-CH₃-Phenyl | |
| 1038 | " | " | " | 4-CH₃-Phenyl | |
| 1039 | " | " | " | 2,4-(CH₃)₂-Phenyl | |
| 1040 | " | " | " | 2,6-(CH₃)₂-Phenyl | |
| 1041 | " | " | " | 2,4,6-(CH₃)₃-Phenyl | |
| 1042 | " | " | " | 2-CH₃O-Phenyl | |
| 1043 | " | " | " | 4-CH₃O-Phenyl | |
| 1044 | " | " | " | 4-C₂H₅O-Phenyl | |
| 1045 | " | " | " | 4-CF₃O-Phenyl | |
| 1046 | " | " | " | 4-CN-Phenyl | |
| 1047 | " | " | " | 3-NO₂-Phenyl | |
| 1048 | " | " | " | 4-NO₂-Phenyl | |

### C. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### D. Biologische Beispiele

### Beispiel 1 : Wirkung auf die Spinnmilben Tetranychus urticae

Abgeschnittene Stengel mit einem Blatt von Bohnenpflanzen (Phaseolus vulgaris) werden in mit Leitungswasser gefüllte Braunglasfläschen übertragen und anschließend mit ca. 100 Spinnmilben (Tetranychus urticae) belegt. Pflanzenblatt und Spinnmilben werden dann für 5 Sekunden in eine wäßrige Lösung des zu prüfenden und formulierten Präparates getaucht. Nach dem Abtropfen werden Pflanze und Tiere in einer Klimakammer gelagert (16 Stunden Licht/Tag, 25 °C, 40-60 % RF). Nach 6 Tagen Lagerung wird die Mortalität des Präparates auf alle Stadien der Spinnmilben festgestellt. Bei einer Konzentration von 500 ppm (bezogen auf den Gehalt an Wirkstoff) bewirken die Präparate gemäß Beispiel Nr. 2, 5, 9, 10, 12, 22, 23, 32, 33, 35, 39, 41, 42, 43, 45, 46, 48, 49, 52, 63, 76, 79, 87, 90, 91, 92, 95, 96, 97, 99, 108, 110, 113, 117, 120, 569, 570, 573, 574, 576, 578, 579, 589, 600, 605, 619, 623, 624, 625, 626, 628, 629, 630, 631 eine 80-100 %ige Mortalität.

### Beispiel 2 : Wirkung auf die Blattlaus Aphis fabae

Abgeschnittene Stengel mit einem Blatt von Bohnenpflanzen (Phaseolus vulgaris) werden in mit Leitungswasser gefüllte Braunglasfläschen übertragen und anschließend mit ca. 100 Blattläusen (Aphis fabea) belegt. Pflanzenblatt und Blattläuse werden dann für 5 Sekunden in eine wäßrige Lösung des zu prüfenden und formulierten Präparates getaucht. Nach dem Abtropfen werden Pflanze und Tiere in einer Klimakammer gelagert (16 Stunden Licht/Tag, 25 °C, 40-60 % RF). Nach 6 Tagen Lagerung wird die Mortalität des Präparates auf alle Stadien der Blattlaus festgestellt. Bei einer Konzentration von 500 ppm (bezogen auf den Gehalt an Wirkstoff) bewirken die Präparate gemäß Beispiel Nr. 96, 103 eine 80-100 %ige Mortalität.

### Beispiel 3 : Wirkung auf das Ei-Larven-Stadium von Heliothis virescens

Eine Petrischale, deren Boden mit Filterpapier belegt ist und ca. 5 ml Nährmedium enthält, wird vorbereitet. Filterpapierstücke mit ca. 30, 24 Stunden alten Eiern der amerikanischen Tabakknospeneule (Heliothis virescens) werden für 5 Sekunden in einer wäßrigen Lösung des zu prüfenden und formulierten Präparates getaucht und anschließend in der Petrischale ausgelegt. Weitere 200 µl der wäßrigen Lösung werden über das Nährmedium verteilt. Nach dem Verschließen der Petrischale wird diese bei ca. 25 °C in einer Klimakammer gelagert. Nach 6 Tagen Lagerung wird die Mortalität des Präparates auf die Eier und die evtl. hieraus geschlüpften Larven festgestellt. Bei einer Konzentration von 500 ppm (bezogen auf den Gehalt an Wirkstoff) bewirken die Präparate gemäß Beispiel Nr. 5, 9, 20, 22, 28, 38, 45, 46, 48, 49, 52, 63, 94, 103, 116, 568, 567, 573, 579, 589, 619, 623 eine 80-100 %ige Mortalität.

### Beispiel 4: Fraßwirkung auf die Schmetterlingslarve Heliothis virescens

Nährmedium (gefriergetrockneter Würfel) wird in eine wäßrige Lösung des zu prüfenden und formulierten Präparates getaucht und anschließend in eine Petrischale gelegt. Danach werden zehn L2-Larven der amerikanischen Tabakknospeneule (Heliothis virescens) eingesetzt. Anschließend wird die Petrischale mit einem Deckel verschlossen. Nach 4 Tagen Lagerung bei ca. 23°C wird die Wirkung des Präparates auf die Larven festgestellt Bei einer Konzentration von 500 ppm (bezogen auf den Gehalt an Wirkstoff) bewirken die Präparate gemäß Beispiel Nr. 2, 9, 13, 20, 22, 28, 29, 30, 32, 35, 39, 40, 41, 42, 43, 45, 46, 48, 49, 52, 63, 76, 78, 91, 93, 94, 95, 98, 103, 114, 116, 117, 120, 121, 124, 125, 567, 573, 589, 600, 605, 613, 614, 615, 619, 621, 623, 624, 629 eine 80-100 %ige Mortalität der Larven.

### Beispiel 5 : Fraßwirkung auf die Schmetterlingslarve Spodoptera litoralis

Nährmedium (gefriergetrockneter Würfel) wird in eine wäßrige Lösung des zu prüfenden und formulierten Präparates getaucht und anschließend in eine Petrischale gelegt. Danach werden zehn L2-Larven des ägyptischen Baumwoltwurms (Spodoptera littoralis) eingesetzt. Anschließend wird die Petrischale mit einem Deckel verschlossen. Nach 4 Tagen Lagerung bei ca. 23 °C wird die Wirkung des Präparates auf die Larven festgestellt. Bei einer Konzentration von 500 ppm (bezogen auf den Gehalt an Wirkstoff) bewirken die Präparate gemäß Beispiel Nr. 5, 9, 12, 28, 29, 30, 35, 39, 40, 41, 42, 43, 45, 46, 48, 52, 63, 87. 90, 91, 92, 94, 97, 98, 99, 100, 103, 107, 113. 117, 120, 125, 566, 567. 573, 574, 589, 600, 613, 614, 619, 626 eine 80-100 %ige Mortalität der Larven.

## Patentansprüche

1. Arylisoxazolin-Derivate der Formel (I), 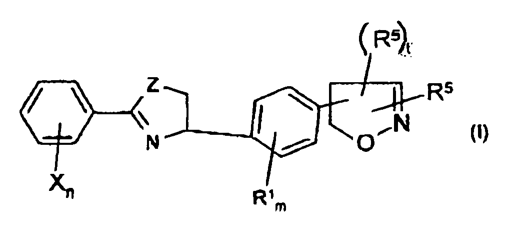 worin die Symbole und Indizes folgende Bedeutungen haben:
X ist gleich oder verschieden
a) Halogen, Cyano. Nitro;
b) (C₁-C₄)-Alkyl, (C₁-C₄)-Alkoxy, (C₁-C₄)-Alkylthio, (C₁-C₄)-Alkylsulfinyl, wobei die Reste der Gruppe b gegebenenfalls durch einen oder mehrere Reste aus der Gruppe Halogen substituiert sind;
R¹ ist gleich oder verschieden Halogen, (C₁-C₄)Haloalkyl, (C₁-C₄)Alkyl, (C₁-C₄)Alkoxy, (C₁-C₄)Haloalkoxy oder Cyano;
m ist 0, 1, 2, 3 oder 4;
n ist 1, 2, 3, 4 oder 5;
Z ist Sauerstoff, Schwefel, CH₂ oder NR²;
R² ist CN, (C₁-C₄)-Alkoxy-(C₁-C₄)-alkyl, CHO, (C₁-C₆)-Alkylcarbonyl, (C₁-C₆)-Alkoxycarbonyl oder (CW)NR³R⁴;
R³, R⁴ sind gleich oder verschieden H, (C₁-C₆)-Alkyl;
W ist O oder S;
t ist 0,1,2 oder 3;
R⁵ ist gleich oder verschieden
a) Halogen, CN, NO₂
b) eine geradkettige oder verzweigte Alkylgruppe mit 1 bis 12 C-Atomen, wobei eine oder mehrere (CH₂)-Gruppen gegebenenfalls durch -O-, -S(O)_{-0,1,2}, -NH-, -NR⁶-, -CO-, -CS-, -CH=CH-, -C≡C-, Aryl-diyl, Heterocyclyl-diyl, (C₃-C₈)-Cycloalkandiyl oder (C₃-C₈)-Cycloalkendiyl ersetzt sind, mit der Maßgabe, daß Chalkogene nicht benachbart sein dürfen, wobei einzelne Wasserstoffatome gegebenenfalls durch Halogen ersetzt sind;
c) im Falle von zwei α-ständigen Resten R⁵ auch (=Y) wobei Y (=O). (=S), (=NOR⁶) oder (=CR₂⁶) bedeutet;
mit der Maßgabe, daß der oder die Reste R⁵ zusammen nicht mehr als ein fünf- oder mehrgliedriges Ringsystem enthalten;
R⁶ ist (C₁-C₄)-Alkyl, Phenyl oder Benzyl;
Aryl ist ein carbocyclischer aromatischer Rest mit 6 bis 14 C-Atomen ;
Heterocyclyl ist ein heteroaromatisches oder heteroaliphatisches Ringsystem, wobei unter "heteroaromatisches Ringsystem" ein Arylrest, worin mindestens eine CH-Gruppe durch N ersetzt ist und/oder mindestens zwei benachbarte CH-Gruppen durch S, NH oder O ersetzt sind, zu verstehen ist und unter "heteroaliphatisches Ringsystem" eine (C₃-C₈)-Cycloalkylrest, in dem mindestens eine Kohlenstoff-Einheit durch O, S oder eine Gruppe NR¹¹ ersetzt ist und R¹¹ Wasserstoff, (C₁-C₄)-Alkyl, (C₁-C₄)-Alkoxy oder Aryl bedeutet,
wobei die cyclischen Reste in der Bedeutung von R⁵, R⁶ gegebenenfalls durch einen oder mehrere Reste aus der Gruppe Halogen, Cyano, Nitro, Amino, Hydroxy. Thio. (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino und (C₁-C₄)-Alkanoyl substituiert sind;
deren reine Stereoisomere an Doppelbindungen, Enantiomere und Diastereomere sowie deren Gemische,
N-Oxide und für den Gebrauch als Schädlingsbekämpfungsmittel geeignete Salze.

2. Verbindung der Formel (I) nach Anspruch 1, wobei die Symbole und Indizes folgende Bedeutungen haben:
X ist Halogen, Cyano, Nitro, (C₁-C₄)-Alkyl, (C₁-C₃)-Haloalkyl, (C₁-C₄)-Alkoxy oder (C₁-C₃)-Haloalkoxy;
m ist 0 oder 1;
n ist 1, 2 oder 3;
Z ist Sauerstoff oder CH₂;
R¹ ist H, Halogen, (C₁-C₄)-Haloalkyl, (C₁-C₄)-Alkyl, (C₁-C₄)-Alkoxy oder (C₁-C₄)-Haloalkoxy;
t ist 0,1,2 oder 3;
R⁵ ist gleich oder verschieden
a) Halogen, CN, NO₂,
b) eine geradkettige oder verzweigte Alkylgruppe mit 1 bis 12 C-Atomen, wobei eine oder mehrere (CH₂)-Gruppen gegebenenfalls durch -O-, -S(O)-_{0,1,2,} -NH-, -NR⁶-, -CO-, -CS-, -CH=CH-, -C≡C-, Aryl-diyl, Heterocyclyl-diyl, (C₃-C₈)-Cycloalkyl oder (C₃-C₈)-Cycloalkenyl ersetzt sind, mit der Maßgabe, daß Chalkogene nicht benachbart sein dürfen, wobei einzelne Wasserstoffatome gegebenenfalls durch Halogen ersetzt sind;
c) im Falle von zwei α-ständigen Resten R⁵ auch (=Y) wobei Y (=O), (=S), (=NOR⁶) oder (=CR₂⁶) bedeutet;
mit der Maßgabe, daß der oder die Reste R⁵ zusammen nicht mehr als ein fünf- oder mehrgliedriges Ringsystem enthalten;
R⁶ ist (C₁-C₄)-Alkyl, Phenyl oder Benzyl;
Aryl ist ein carbocyclischer aromatischer Rest mit 6 bis 14 C-Atomen;
Heterocyclyl ist ein heteroaromatisches oder heteroaliphatisches Ringsystem, wobei unter "heteroaromatisches Ringsystem" ein Arylrest, worin mindestens eine CH-Gruppe durch N ersetzt ist und/oder mindestens zwei benachbarte CH-Gruppen durch S, NH oder O ersetzt sind, zu verstehen ist und unter "heteroaliphatisches Ringsystem" eine (C₃-C₈)-Cycloalkylrest, in dem mindestens eine Kohlenstoff-Einheit durch O, S oder eine Gruppe NR¹¹ ersetzt ist und R¹¹ Wasserstoff, (C₁-C₄)-Alkyl, (C₁-C₄)-Alkoxy oder Aryl bedeutet,
wobei die cyclischen Reste in der Bedeutung von R⁵, R⁶ gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino und (C₁-C₄)-Alkanoyl substituiert sind und
wobei der Isoxazolinring in der 3- oder 5-Position mit dem Phenylrest verknüpft ist.

3. Verbindung gemäß Anspruch 2, wobei die Gruppen R⁵ folgende Bedeutungen haben:
R⁵ ist CN, gegebenenfalls substituiertes Phenyl, gegebenenfalls substituiertes Phenoxy, (C₁-C₆)-Alkyl, (C₁-C₆)-Alkenyl, (C₁-C₈)-Haloalkyl, (C₁-C₆)-Haloalkenyl, -(C₁-C₆)-Alkandiyl-Aryl, wobei die Arylgruppe gegebenenfalls substituiert ist und wobei eine -CH₂-Einheit gegebenenfalls durch -C(O)-NR¹⁰-, NR¹⁰-(CO), NR¹⁰ oder O ersetzt ist;
R¹⁰ ist H, (C₁-C₆)-Alkyl, (C₁-C₈)-Haloalkyl, gegebenenfalls substituiertes Phenyl, gegebenenfalls substituiertes Benzyl.

4. Verbindung nach einem oder mehreren der Ansprüche 1 bis 3, wobei 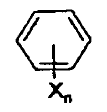 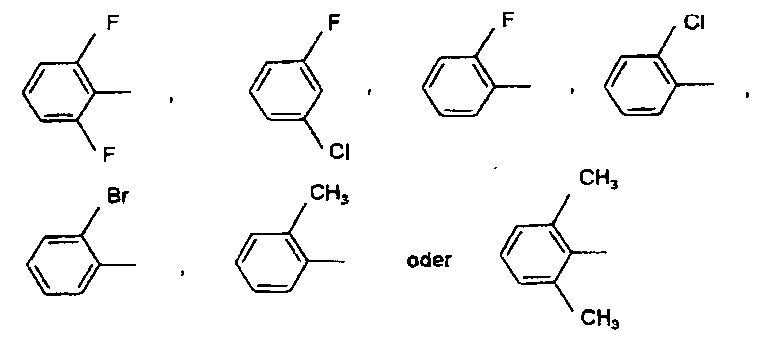 bedeutet.

5. Verbindungen nach einem oder mehreren der Ansprüche 1 bis 4, aus der Gruppe (I1) bis (I28): 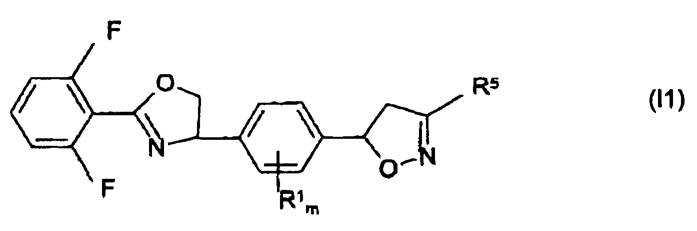 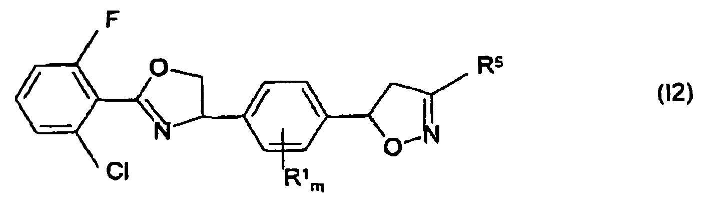 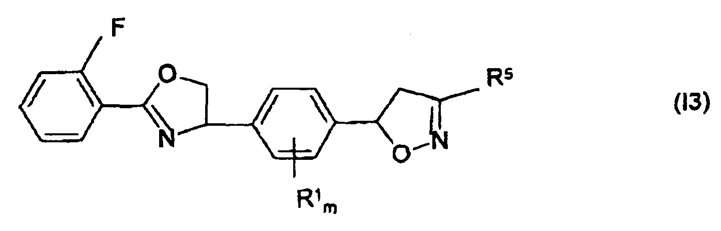 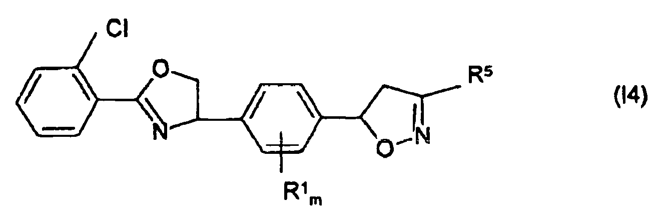 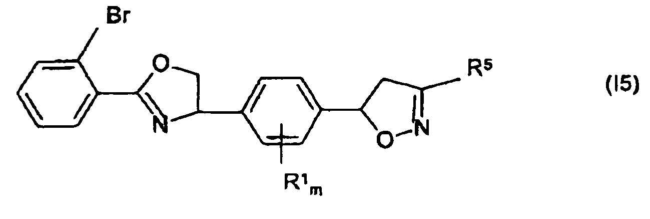 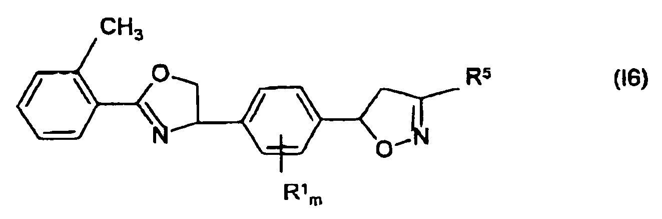 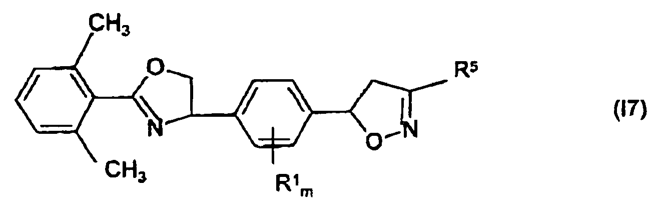 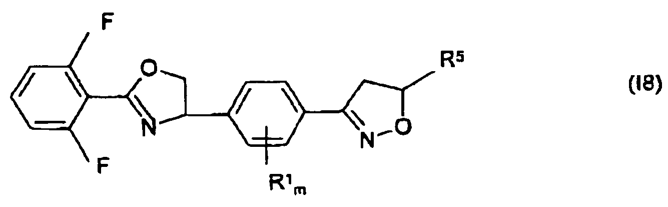 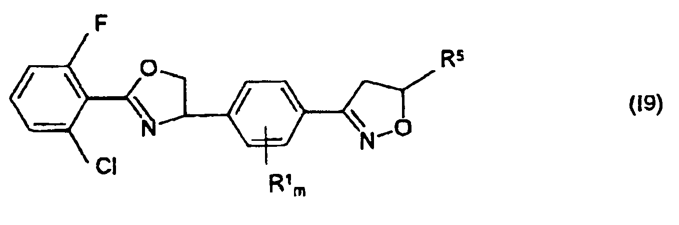 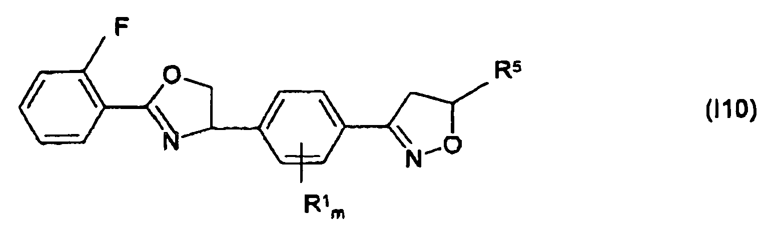 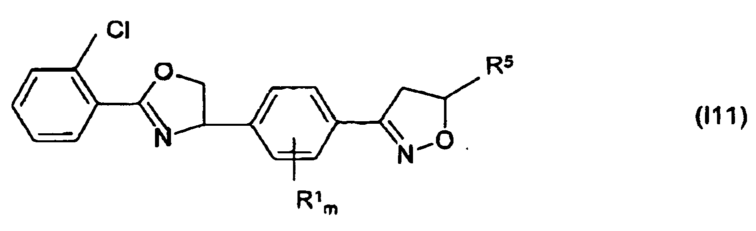 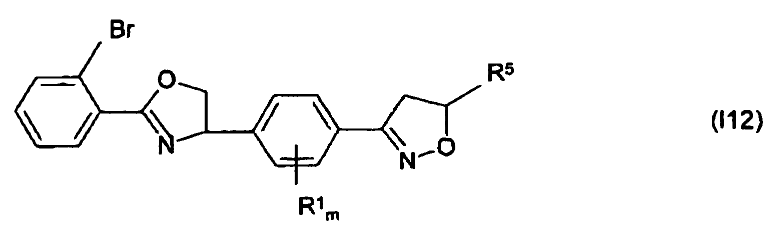 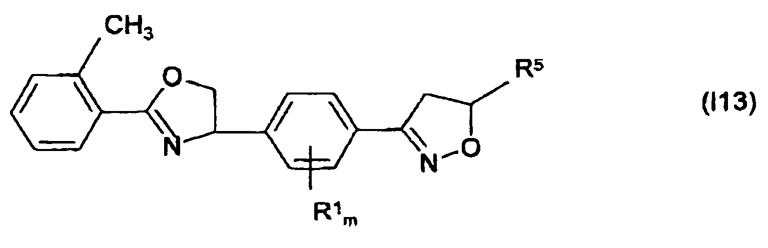 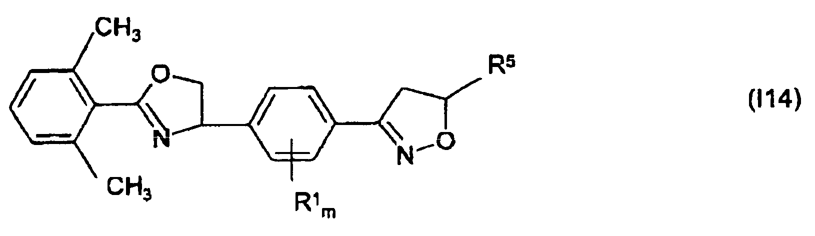 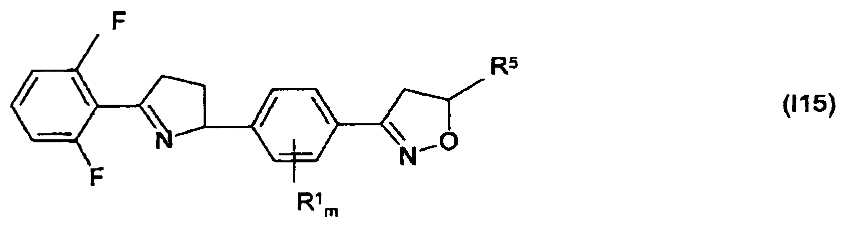 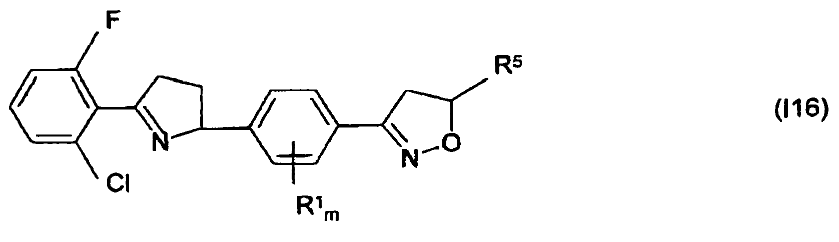 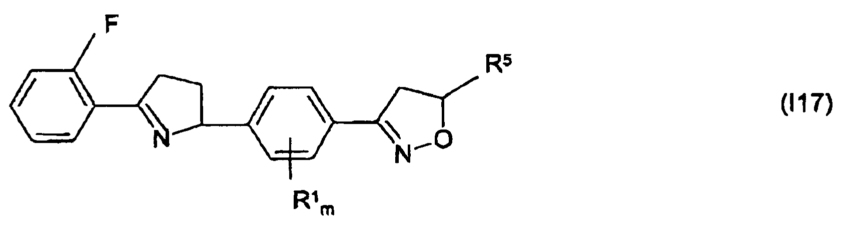 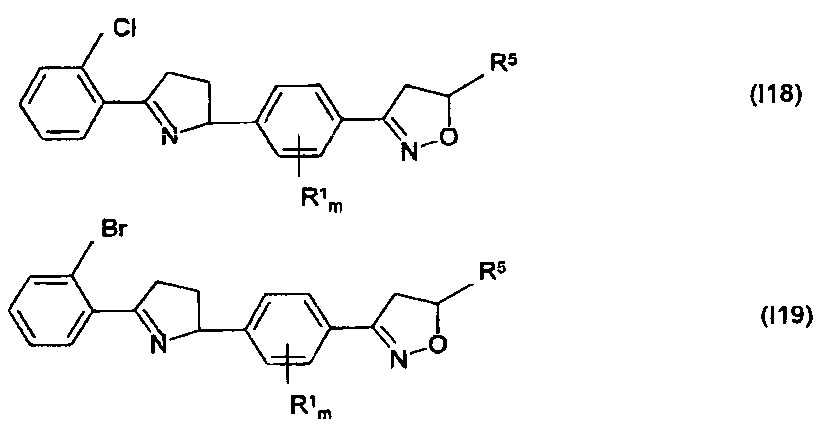 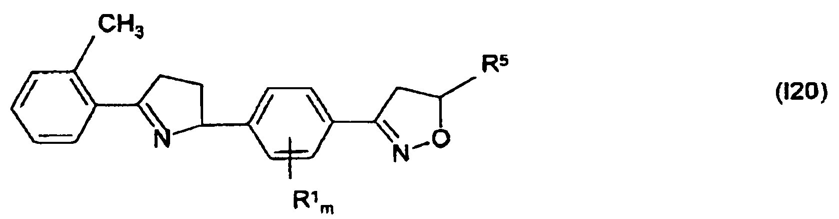 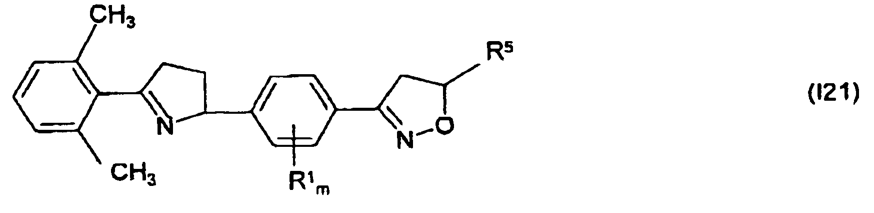 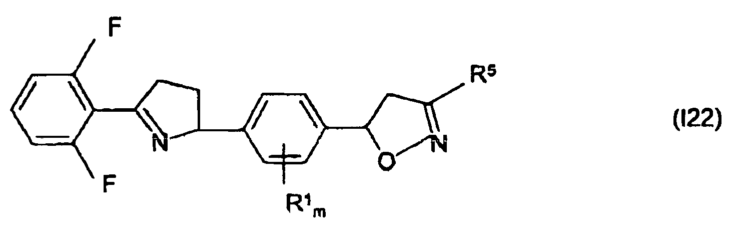 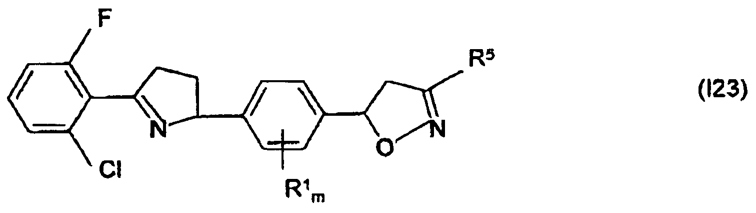 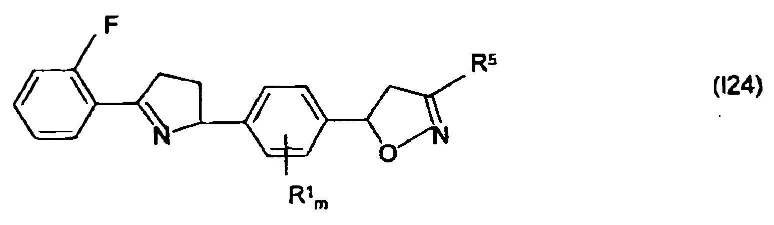 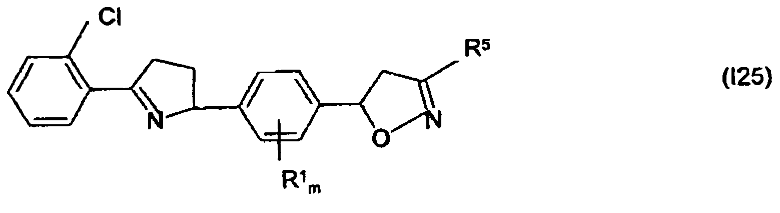 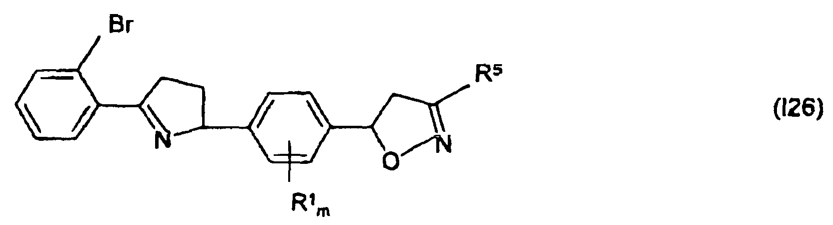 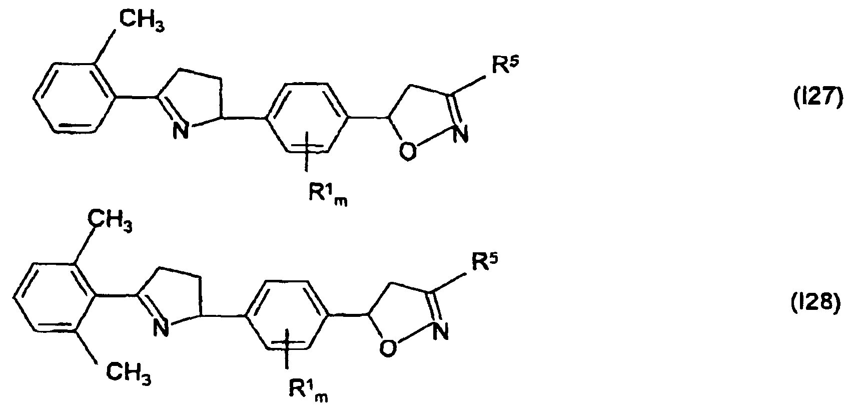 wobei R¹ₘ und R⁵ die in Anspruch 2 angegebenen Bedeutungen haben.

6. Verfahren zur Herstellung von Verbindungen der Formel (I) nach einem oder mehreren der Ansprüche 1 bis 5, wobei man
a) zur Herstellung von Verbindungen mit einem 3-Isoxazolinyl-Rest ein Oxim der Formel (II), 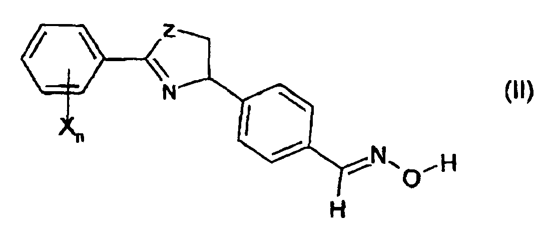 worin
X und Z die in der Formel (I) angegebenen Bedeutungen haben,
mit einem Halogenierungsmittel zu einer Verbindung der Formel (III) umsetzt, 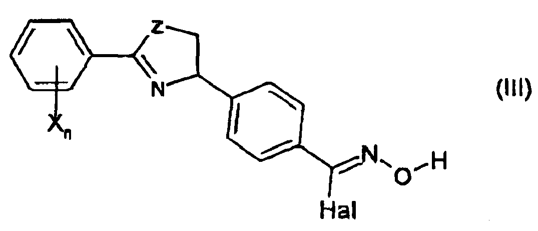 worin
Hal Halogen bedeutet,
und anschließend weiter mit einem Olefin der Formel (IV), 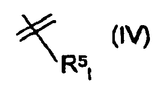 worin R⁵ und t die in Anspruch 1 angegebenen Bedeutungen haben, umsetzt; oder
b) zur Herstellung in Verbindungen mit einem 5-Isoxazinyl-Rest ein Olefin der Formel (VII) 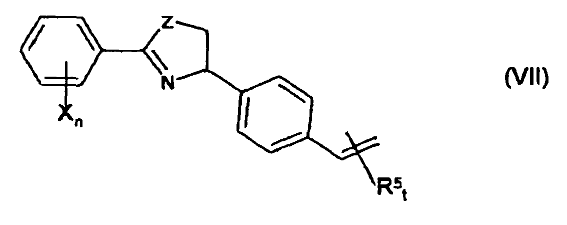 worin
Z und R⁵ₜ die in Anspruch 2 Bedeutungen haben, mit einem Halogenoxim der Formel (VIII), 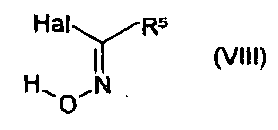 wobei R⁵ die in Anspruch 2 angegebenen Bedeutungen hat, umsetzt.

7. Schädlingsbekämpfungsmittel, enthaltend mindestens eine Verbindung gemäß einem der Ansprüche 1 bis 5 und mindestens einen Formulierungshilfttoff.

8. Insektizides, akarizides und/oder nematizides Mittel gemäß Anspruch 7, enthaltend eine wirksame Menge mindestens einer Verbindung gemäß einem oder mehreren der Ansprüche 1 bis 5 zusammen mit für diese Anwendung üblichen Zusatz- oder Hilfsstoffen.

9. Schädlingsbekämpfungsmittel, enthaltend eine insektizid, akarizid und/oder nematizid wirksame Menge mindestens einer Verbindung gemäß einem oder mehreren der Ansprüche 1 bis 5 und mindestens einem weiteren Wirkstoff zusammen mit für diese Anwendung üblichen Hilfs- und Zusatzstoffen.

10. Mittel zur Anwendung im Holzschutz oder als Konservierungsmittel in Dichtmassen, in Anstrichfarben, in Kühlschmiermitteln für die Metallbearbeitung oder in Bohr- und Schneidölen, enthaltend eine wirksame Menge mindestens einer Verbindung gemäß einem der Ansprüche 1 bis 5 zusammen mit den für diese Anwendungen üblichen Hilfs- und Zusatzstoffen.

11. Verwendung einer Verbindung gemäß einem oder mehreren der Ansprüche 1 bis 5 oder eines Mittels gemäß Anspruch 7, 8 oder 9 zur Herstellung eines Tierarzneimittels.

12. Verfahren zur Herstellung eines Mittels gemäß einem oder mehreren der Ansprüche 7 bis 11, wobei man den Wirkstoff und die weiteren Zusätze zusammen gibt und in eine geeignete Anwendungsform bringt.

13. Verwendung einer Verbindung gemäß einem oder mehreren der Ansprüche 1 bis 5 oder eines Mittels gemäß einem oder mehreren der Ansprüche 7, 8 und 9 als Holzschutzmittel, als Konservierungsmittel in Dichtmitteln, in Anstrichfarben, in Kühlschmiermitteln für die Metallbearbeitung und/oder in Bohr- und Schneideölen.

14. Verwendung von Verbindungen gemäß einem oder mehreren der Ansprüche 1 bis 5 oder eines Mittels gemäß einem oder mehreren der Ansprüche 7, 8, 9 oder 10 zur Bekämpfung von Schadinsekten, Acarina, Mollusken und Nematoden.

15. Verfahren zur Bekämpfung von Schadinsekten, Acarina, Mollusken und/oder Nematoden, wobei man die genannten Organismen in Kontakt mit einer wirksamen Menge einer oder mehreren Verbindungen nach einem oder mehreren der Ansprüche 1 bis 5 oder eines Mittels gemäß einem oder mehreren der Ansprüche 7,8,9, oder 10 bringt.

16. Verfahren zur Bekämpfung von Schadinsekten, Acarina, Mollusken und/oder Nematoden gemäß Anspruch 15, bei welchem man auf diese oder die von ihnen befallenen Pflanzen, Flächen oder Substrate eine wirksame Menge einer Verbindung gemäß einem der Ansprüche 1 bis 5 oder eines Mittels gemäß einem der Ansprüche 7, 8 oder 9 appliziert.

17. Saatgut, enthaltend oder beschichtet mit einer wirksamen Menge einer Verbindung gemäß einem der Ansprüche 1 bis 5 oder eines Mittels gemäß einem der Ansprüche 7, 8 oder 9.

## Claims

1. Arylisoxazoline derivative of the formula (I), 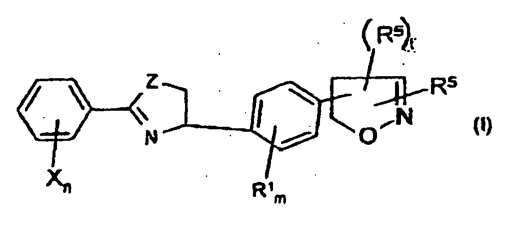 in which the symbols and indices are as defined below:
X is identical or different
a) halogen, cyano, nitro;
b) (C₁-C₄)-alkyl, (C₁-C₄)-alkoxy, (C₁-C₄)-alkylthio, (C₁-C₄)-alkylsulfinyl, where the radicals of group b are unsubstituted or substituted by one or more radicals selected from the group consisting of halogen;
R¹ is identical or different halogen, (C₁-C₄)-haloalkyl, (C₁-C₄)-alkyl, (C₁-C₄)-alkoxy, (C₁-C₄)-haloalkoxy or cyano;
m is 0, 1, 2, 3 or 4;
n is 1, 2, 3, 4 or 5;
Z is oxygen, sulfur, CH₂ or NR²;
R² is CN, (C₁-C₄)-alkoxy-(C₁-C₄)-alkyl, CHO, (C₁-C₆)-alkylcarbonyl, (C₁-C₆)-alkoxycarbonyl or (CW)NR³R⁴;
R³, R⁴ are identical or different H, (C₁-C₆)-alkyl;
W is O or S;
t is 0, 1, 2 or 3;
R⁵ is identical or different
a) halogen, CN, NO₂;
b) a straight-chain or branched alkyl group having 1 to 12 carbon atoms, where one or more (CH₂) groups are optionally replaced by -O-, -S(O)-₀, _{1, 2},-NH-, -NR⁶- -CO-, -CS-, -CH=CH-, -C≡C-, aryldiyl, heterocyclyldiyl, (C₃-C₈)-cycloalkanediyl or (C₃-C₈)-cycloalkenyldiyl, with the proviso that chalcogens may not be adjacent to one another, where individual hydrogen atoms are optionally replaced by halogen;
c) in the case of two radicals R⁵ located in the α-position, the radicals are also (=Y), where Y is (=O), (=S), (=NOR⁶) or (=CR₂⁶⁾;
with the proviso that the radical(s) R⁵ together do not comprise more than one ring system having five or more members;
R⁶ is (C₁-C₄)-alkyl, phenyl or benzyl;
Aryl is a carbocyclic aromatic radical having 6 to 14 carbon atoms;
Heterocyclyl is a heteroaromatic or heteroaliphatic ring system, where "heteroaromatic ring system" is to be understood as meaning an aryl radical where at least one CH group is replaced by N and/or at least two adjacent CH groups are replaced by S, NH or O, and "heteroaliphatic ring system" is to be understood as meaning a (C₃-C₈)-cycloalkyl radical in which at least one carbon unit is replaced by O, S or a group NR¹¹ and R¹¹ is hydrogen, (C₁-C₄)-alkyl, (C₁-C₄)-alkoxy or aryl,
where the cyclic radicals in the meaning of R⁵, R⁶ are optionally substituted by one or more radicals selected from the group consisting of halogen, cyano, nitro, amino, hydroxyl, thio, (C₁-C₄)-alkyl, (C₁-C₄)-haloalkyl, (C₃-C₈)-cycloalkyl, (C₁-C₄)-alkoxy, (C₁-C₄)-haloalkoxy, (C₁-C₄)-alkylthio, (C₁-C₄)-haloalkylthio, (C₁-C₄)-alkylamino, (C₁-C₄)-haloalkylamino and (C₁-C₄)-alkanoyl;
and its double-bond-, pure stereoisomers, enantiomers and diastereomers and mixtures thereof, N-oxides and salts suitable for use as pesticides.

2. Compound of the formula (I) according to Claim 1, where the symbols and indices are as defined below:
X is halogen, cyano, nitro, (C₁-C₄)-alkyl, (C₁-C₃)-haloalkyl, (C₁-C₄)-alkoxy or (C₁-C₃)-haloalkoxy,
m is 0 or 1,
n is 1, 2 or 3,
Z is oxygen or CH₂,
R¹ is H, halogen, (C₁-C₄)-haloalkyl, (C₁-C₄)-alkyl, (C₁-C₄)-alkoxy or (C₁-C₄)-haloalkoxy,
t is 0,1, 2 or 3,
R⁵ is identical or different
a) halogen, CN, NO₂;
b) a straight-chain or branched alkyl group having 1 to 12 carbon atoms, where one or more (CH₂) groups are optionally replaced by -O-, -S(O)-_{0,1,2}, -NH-, -NR⁶-, -CO-, -CS-, -CH=CH-, -C≡C-, aryldiyl, heterocyclyldiyl, (C₃-C₈)-cycloalkyl or (C₃-C₈)-cycloalkenyl, with the proviso that chalcogens may not be adjacent to one another, where individual hydrogen atoms are optionally replaced by halogen;
c) in the case of two radicals R⁵ located in the α-position, the radicals are also (=Y), where Y is (=O), (=S), (=NOR⁶) or (=CR₂⁶);
with the proviso that the radical(s) R⁵ together do not comprise more than one ring system having five or more members;
R⁶ is (C₁-C₄)-alkyl, phenyl or benzyl;
Aryl is a carbocyclic aromatic radical having 6 to 14 carbon atoms;
Heterocyclyl is a heteroaromatic or heteroaliphatic ring system, where "heteroaromatic ring system" is to be understood as meaning an aryl radical where at least one CH group is replaced by N and/or at least two adjacent CH groups are replaced by S, NH or O, and "heteroaliphatic ring system" is to be understood as meaning a (C₃-C₈)-cycloalkyl radical in which at least one carbon unit is replaced by O, S or a group NR¹¹ and R¹¹ is hydrogen, (C₁-C₄)-alkyl, (C₁-C₄)-alkoxy or aryl;
where the cyclic radicals in the meaning of R⁵, R⁶ are optionally substituted by one or more radicals selected from the group consisting of halogen, cyano, nitro, amino, hydroxyl, thio, (C₁-C₄)-alkyl, (C₁-C₄)-haloalkyl, (C₃-C₈)-cycloalkyl, (C₁-C₄)-alkoxy, (C₁-C₄)-haloalkoxy, (C₁-C₄)-alkylthio, (C₁-C₄)-haloalkylthio, (C₁-C₄)-alkylamino, (C₁-C₄)-haloalkylamino and (C₁-C₄)-alkanoyl, and
the isoxazoline ring being joined to the phenyl radical in the 3- or 5-position.

3. Compound according to Claim 2, where the groups R⁵ are as defined below:
R⁵ is CN, unsubstituted or substituted phenyl, unsubstituted or substituted phenoxy, (C₁-C₆)-alkyl, (C₁-C₆)-alkenyl, (C₁-C₈)-hatoalkyl, (C₁-C₆)-haloalkenyl, -(C₁-C₆)-alkanediyl-aryl, where the aryl group is unsubstituted or substituted and where one -CH₂- unit is optionally replaced by -C(O)-NR¹⁰-, NR¹⁰-(CO), NR¹⁰ or O;
R¹⁰ is H, (C₁-C₆)-alkyl, (C₁-C₆)-haloalkyl, unsubstituted or substituted phenyl, unsubstituted or substituted benzyl.

4. Compound according to one or more of Claims 1 to 3, where 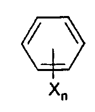 is

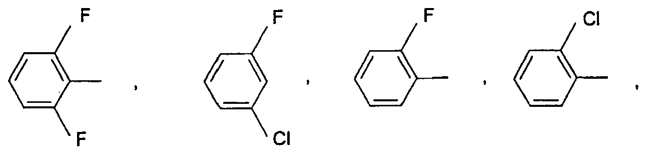

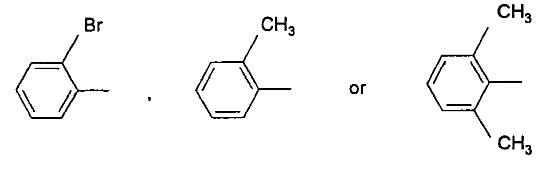

5. Compound according to one or more of Claims 1 to 4, selected from the group consisting of (I1) to (128): 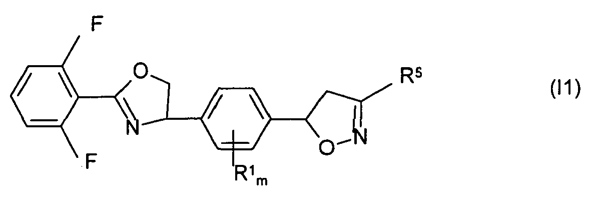 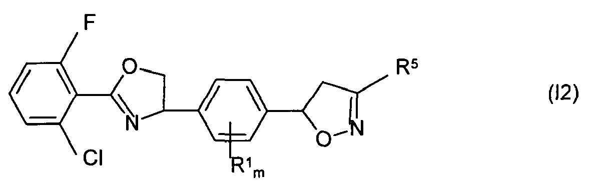 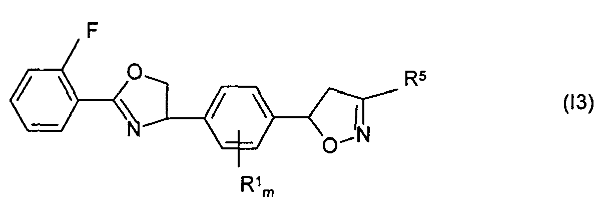 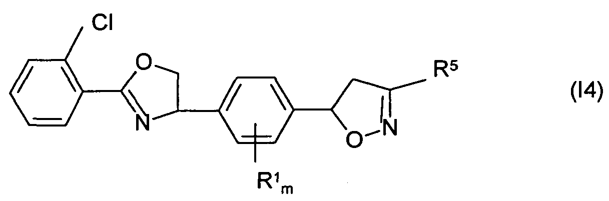 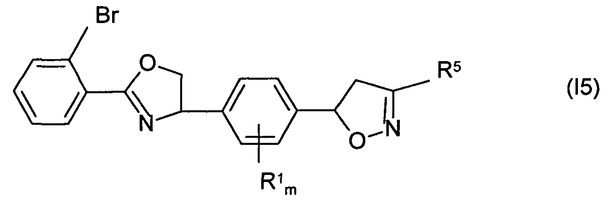 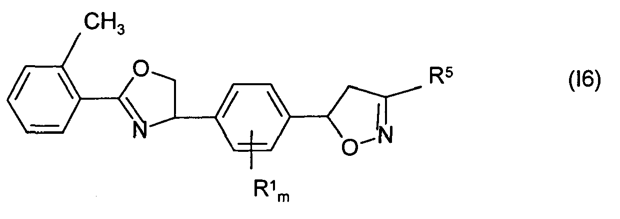 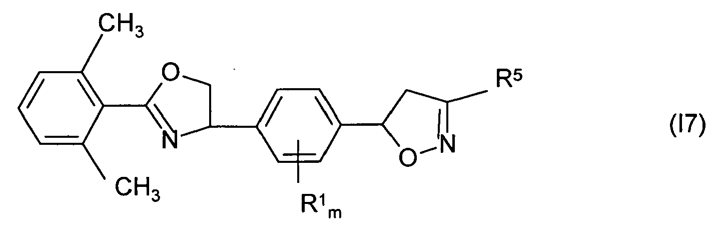 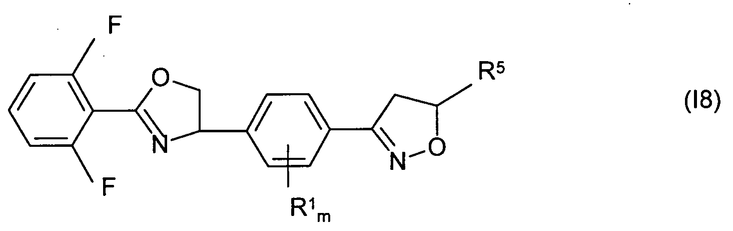 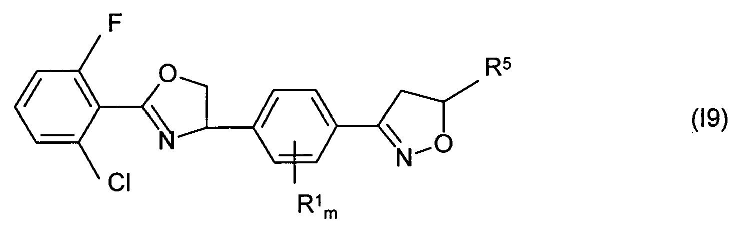 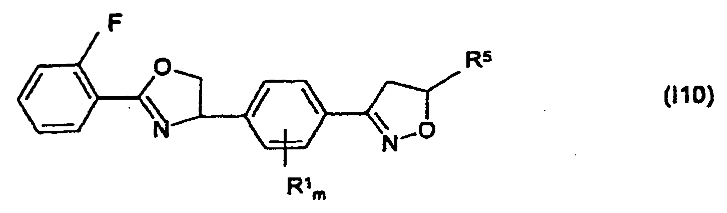 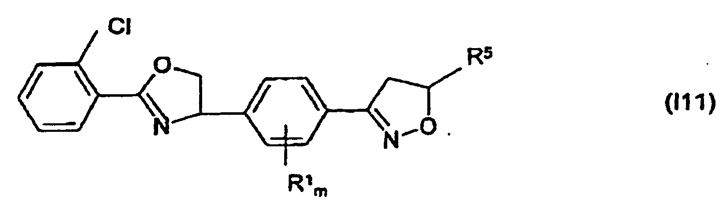 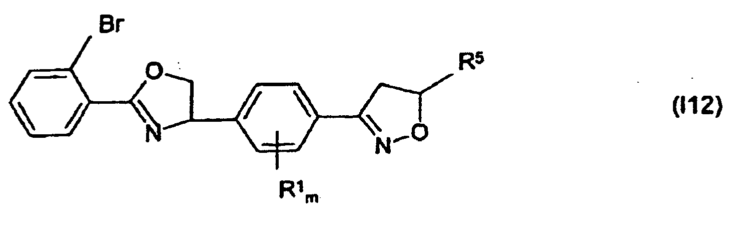 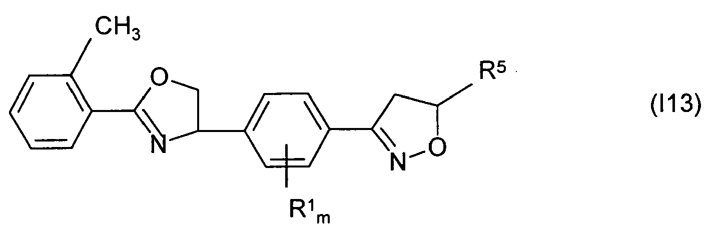 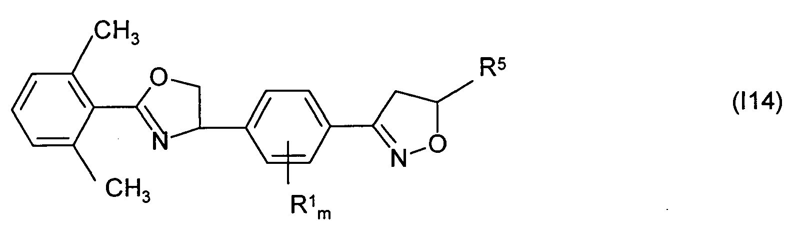 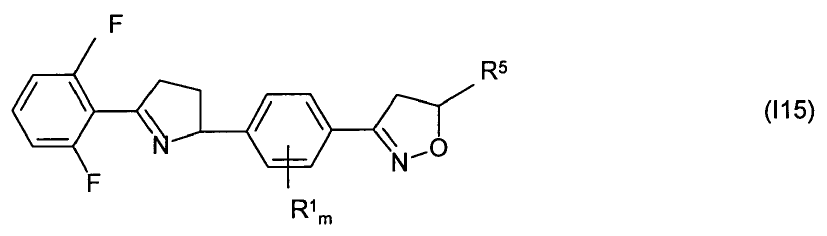 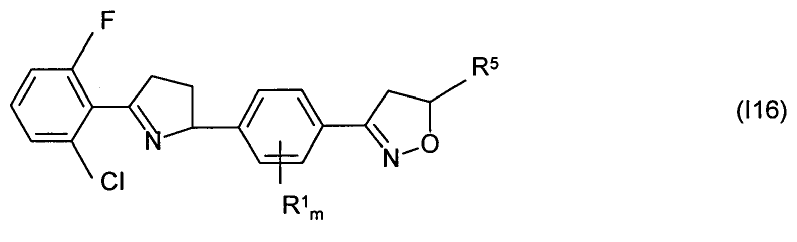 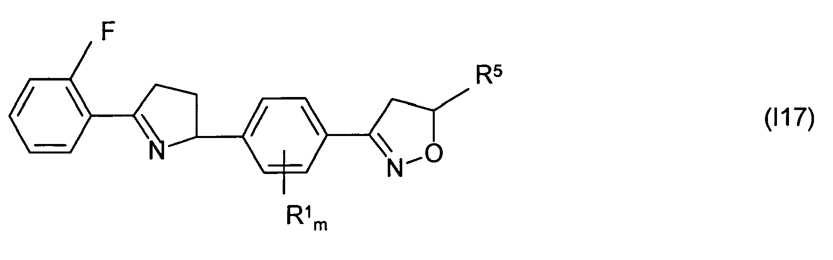 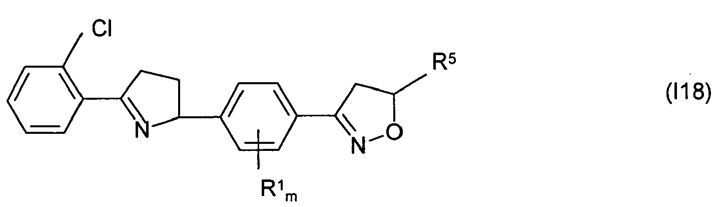 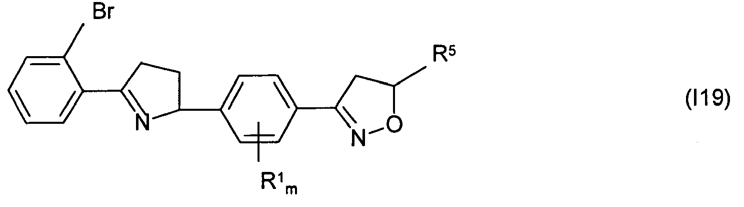 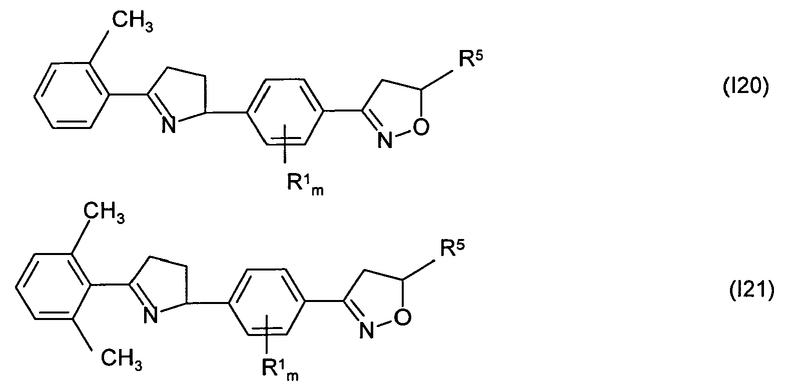 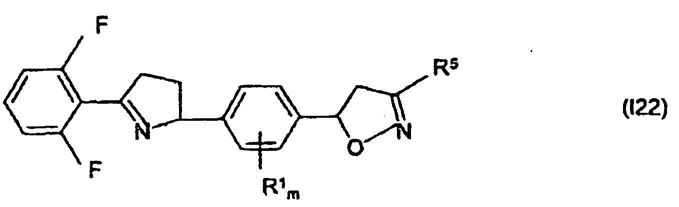 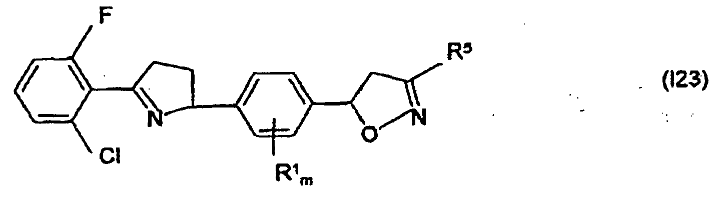 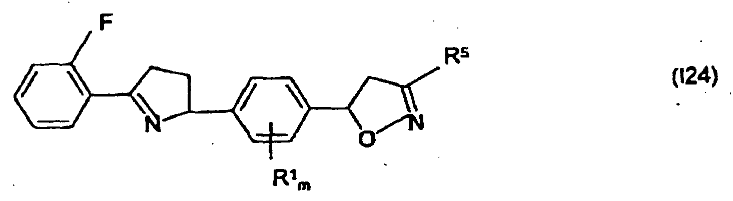 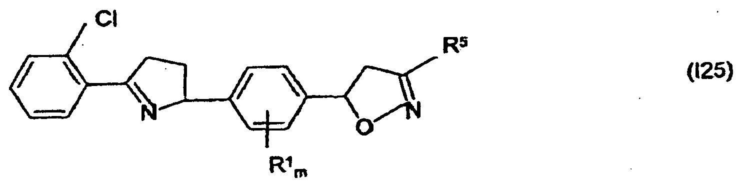 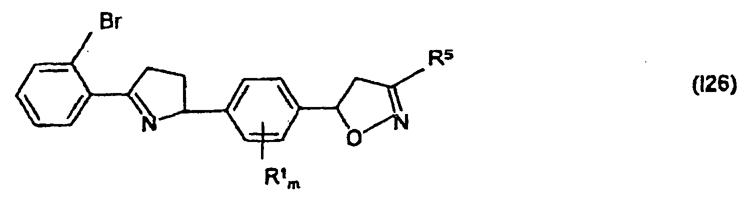 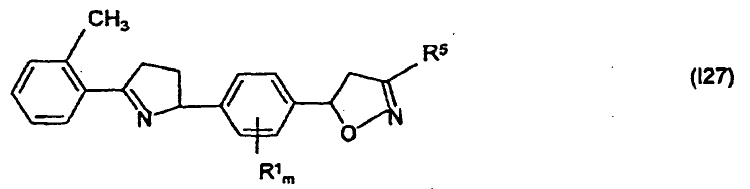 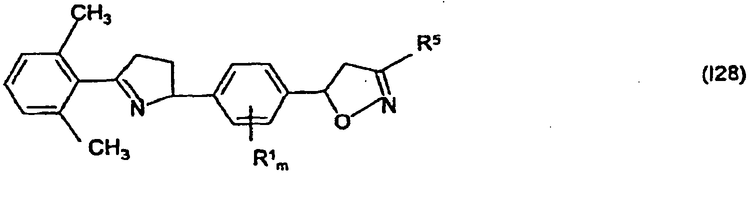 where R¹ₘ and R⁵ are as defined in claim 2.

6. Process for preparing compounds of the formula (I) according to one or more of Claims 1 to 5, where
a) to prepare compounds having a 3-isoxazolinyl radical, an oxime of the formula (II), 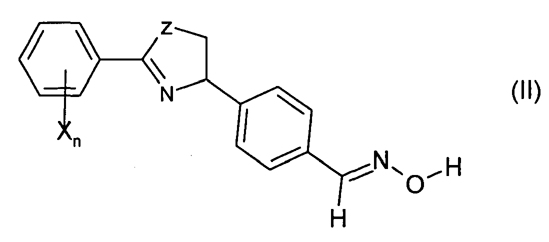 in which
X and Z are as defined for formula (I)
is reacted with a halogenating agent to give a compound of the formula (III) 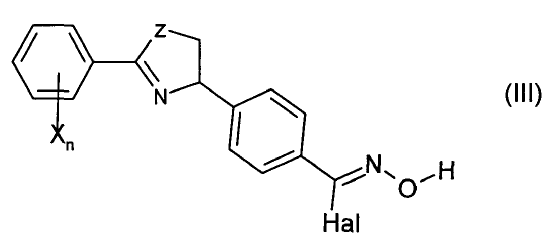 in which
Hal is halogen
and then reacted further with an olefin of the formula (IV), 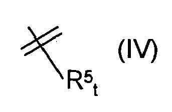 in which R⁵ and t are as defined in Claim 1;
or
b) to prepare compounds having a 5-isoxazinyl radical, an olefin of the formula (VII) 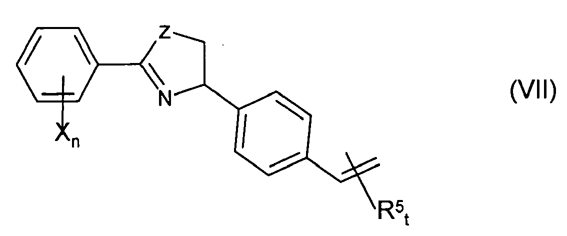 in which
Z and R⁵ₜ are as defined in Claim 2, is reacted with a halogenated oxime of the formula (VIII) 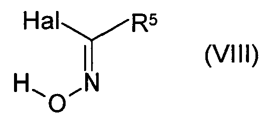 where R⁵ is as defined in Claim 2.

7. Pesticide, comprising at least one compound according to any of Claims 1 to 5 and at least one formulation auxiliary.

8. Insecticidal, acaricidal and/or nematocidal composition according to Claim 7, comprising an effective amount of at least one compound according to one or more of Claims 1 to 5 together with additives or auxiliaries conventionally used for this application.

9. Pesticide, comprising an insecticidally, acaricidally and/or nematicidally effective amount of at least one compound according to one or more of Claims 1 to 5 and at least one further active compound, together with auxiliaries and additives conventionally used for this application.

10. Composition for use in timber protection or as a preservative in sealants, in paints, in cooling lubricants for metal working or in drilling and cutting oils, comprising an effective amount of at least one compound according to any of Claims 1 to 5 together with the auxiliaries or additives conventionally used for this application.

11. Use of a compound according to one or more of Claims 1 to 5 or of a composition according to Claim 7, 8 or 9 for preparing a veterinary medicament.

12. Process for preparing a composition according to one or more of Claims 7 to 11, which comprises combining the active compound and the other additives and formulating them to give a suitable use form.

13. Use of a compound according to one or more of Claims 1 to 5 or of a composition according to one or more of Claims 7, 8 and 9 as a timber preservative or as a preservative in sealants, in paints, in cooling lubricants for metal working and/or in drilling and cutting oils.

14. Use of compounds according to one or more of Claims 1 to 5 or of a composition according to one or more of Claims 7, 8, 9 and 10 for controlling harmful insects, Acarina, molluscs and nematodes.

15. Method for controlling harmful insects, Acarina, molluscs and/or nematodes, in which an effective amount of one or more compounds according to one or more of Claims 1 to 5 or of a composition according to one or more of Claims 7, 8, 9 and 10 is brought into contact with the organisms mentioned.

16. Method for controlling harmful insects, Acarina, molluscs and/or nematodes according to Claim 15, in which an effective amount of a compound according to any of Claims 1 to 5 or of a composition according to any of Claims 7, 8 and 9 is applied to these organisms or to the plants, areas or substrates infested with them.

17. Seed, comprising or coated with an effective amount of a compound according to any of Claims 1 to 5 or of a composition according to any of Claims 7, 8 or 9.

## Revendications

1. Dérivés d'arylisoxazolines de formule (I) 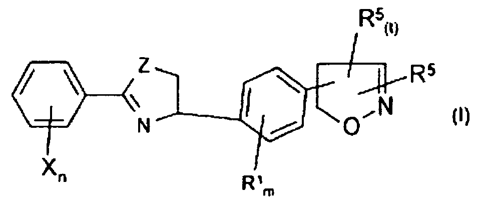 dans laquelle les symboles et les indices ont les définitions suivantes :
X a des valeurs identiques ou différentes et représente :
a) un halogène, un groupe cyano, nitro,
b) un reste alkyle en C₁ à C₄, alkoxy en C₁ à C₄, alkylthio en C₁ à C₄, alkylsulfinyle en C₁ à C₄, les restes du groupe b étant éventuellement substitués par un ou plusieurs restes du groupe halogène ;
R¹ a des valeurs identiques ou différentes et représente un halogène, un reste halogénalkyle en C₁ en C₄, alkyle en C₁ à C₉, alkoxy en C₁ à C₄, halogénalkoxy en C₁ à C₄ ou cyano ;
m a la valeur 0, 1, 2 ou 4 ;
n a la valeur 1, 2, 3, 4 ou 5 ;
Z représente l'oxygène, le soufre, un groupe CH₂ ou NR²;
R² représente CN, un reste (alkoxy en C₁ à C₄)-(alkyle en C₁ à C₄), CHO, (alkyle en C₁ à C₆) carbonyle, (alkoxy en C₁ à C₆)carbonyle ou (CW)NR³R⁹;
R³,R⁴ identiques ou différents, représentent H, un reste alkyle en C₁ à C₆;
W représente O ou S;
t a la valeur 0, 1, 2 ou 3;
R⁵ a des valeurs identiques ou différentes et représente:
a) un halogène, un groupe CN, NO₂
b) un groupe alkyle linéaire ou ramifié ayant 1 à 12 atomes de carbone, un ou plusieurs groupes CH₂ étant éventuellement remplacés par -O-, -S (O)_{0,1,2}, -NH-, -NR⁶-, -CO-, -CS-, -CH=CH-, -C≡C-, aryl-diyle, hétérocyclyl-diyle, (cycloalcane en C₃ à C₈) diyle ou (cycloalcényle en C₃ à C₈) diyle, sous réserve que des chalcogènes ne puissent pas être voisins, des atomes d'hydrogène individuels étant éventuellement remplacés par un halogène ;
c) également (=Y), dans le cas de deux restes R⁵ en position α, Y représentant (=O), (=S),(=NOR⁶) ou (=CR₂⁶) ;
sous réserve que le ou les restes R⁶ ne contiennent pas, ensemble, plus d'un système cyclique ayant cinq ou plus de cinq chaînons ;
R⁶ est un reste alkyle en C₁ à C₄, phényle ou benzyle ;
Aryle est un reste aromatique carbocyclique ayant 6 à 14 atomes de carbone ;
Hétérocyclyle est un système cyclique hétéroaromatique ou hétéroaliphatique, l'expression "système cyclique hétéroaromatique" devant être comprise comme désignant un reste aryle dans lequel au moins un groupe CH est remplacé par N et/ou au moins deux groupes CH voisins sont remplacés par S, NH ou O, et l'expression "système cyclique hétéroaliphatique" désignant un reste cycloalkyle en C₃ à C₈, dans lequel au moins un motif carboné est remplacé par O, S ou un groupe NR¹¹, et R¹¹ représente l'hydrogène, un reste alkyle en C₁ à C₄, alkoxy en C₁ à C₄ ou aryle,
les restes cycliques dans la définition de R⁵, R⁶ étant éventuellement substitués par un ou plusieurs restes du groupe halogéno, cyano, nitro, amino, hydroxy, thio, alkyle en C₁ à C₄, halogénalkyle en C₁ à C₄, cycloalkyle en C₃ à C₈, alkoxy en C₁ à C₄, halogénalkoxy en C₁ à C₉, alkylthio en C₁ à C₄, halogénalkylthio en C₁ à C₄, alkylamino en C₁ à C₄, halogénalkylamino en C₁ à C₄ et alcanoyle en C₁ à C₄ ; leurs stéréoisomères purs au niveau des doubles liaisons, leurs énantiomères et leurs diastéréoisomères ainsi que leurs mélanges, leurs N-oxydes et leurs sels convenant pour être utilisés comme pesticides.

2. Composé de formule (I) suivant la revendication 1, formule dans laquelle les symboles et les indices ont les définitions suivantes :
X est un halogène, un groupe cyano, nitro, alkyle en C₁ à C₄, halogénalkyle en C₁ à C₃, alkoxy en C₁ à C₄ ou halogénalkoxy en C₁ à C₃ ;
m a la valeur 0 ou 1 ;
n a la valeur 1, 2 ou 3 ;
Z représente l'oxygène ou CH₂ ;
R¹ représente H, un halogène, un reste halogénalkyle en C₁ à C₄, alkyle en C₁ à C₄, alkoxy en C₁ à C₄ ou halogénalkoxy en C₁ à C₄ ;
t a la valeur 0, 1, 2 ou 3 ;
R⁵ a des valeurs identiques ou différentes et représente :
a) un halogène, un groupe CN, NO₂,
b) un groupe alkyle linéaire ou ramifié ayant 1 à 12 atomes de carbone, un ou plusieurs groupes CH₂ étant éventuellement remplacés par -O-, -S(O)_{0,1,2}, -NH-, -NR⁶-, -CO-, -CS-, -CH=CH-, -C=C-, aryl-diyle, hétérocyclyldiyle, cycloalkyle en C₃ à C₈ ou cycloalcényle en C₃ à C₈, sous réserve que des chalcogènes ne puissent pas être voisins, des atomes d'hydrogène individuels étant éventuellement remplacés par un halogène ;
c) également (=Y), dans le cas de deux restes R⁵ en position α, Y désignant (=O), (=S), (=NOR⁶) ou (-CR₂⁶) ;
sous réserve que le ou les restes R⁵ ne contiennent, ensemble, pas plus d'un système cyclique à cinq ou plus de cinq chaînons ;
R⁶ est un reste alkyle en C₁ à C₄, phényle ou benzyle ;
Aryle est un reste aromatique carbocyclique ayant 6 à 14 atomes de carbone ;
Hétérocyclyle est un système cyclique hétéroaromatique ou hétéroaliphatique, l'expression "système cyclique hétéroaromatique" devant être comprise comme désignant un reste aryle dans lequel au moins un groupe CH est remplacé par N et/ou au moins deux groupes CH voisins étant remplacés par S, NH ou O, et l'expression "système cyclique hétéroaliphatique" désigne un reste cycloalkyle en C₃ à C₈ dans lequel au moins un motif carboné est remplacé par O, S ou un groupe R¹¹, et R¹¹ représente l'hydrogène, un reste alkyle en C₁ à C₄, alkoxy en C₁ à C₄ ou aryle,
les restes cycliques dans la définition de R⁵, R⁶ étant éventuellement substitués par un ou plusieurs restes du groupe halogéno, cyano, nitro, amino, hydroxy, thio, alkyle en C₁ à C₄, halogénalkyle en C₁ à C₄, cycloalkyle en C₃ à C₈, alkoxy en C₁ à C₄, halogénalkoxy en C₁ à C₄, alkylthio en C₁ à C₄, halogénalkylthio en C₁ à C₉, alkylamino en C₁ à C₉, halogénalkylamino en C₁ à C₄ et alcanoyle en C₁ à C₄, et le noyau d'isoxazoline étant lié en position 3 ou 5 au reste phényle.

3. Composé suivant la revendication 2, dans lequel les groupes R⁵ ont les définitions suivantes :
R⁵ représente CN, un reste phényle éventuellement substitué, un reste phénoxy éventuellement substitué, un reste alkyle en C₁ à C₆, alcényle en C₁ à C₆, halogénalkyle en C₁ à C₆, halogénalcényle en C₁ à C₆, - (alcanediyle en C₁ à C₆) -aryle, le groupe aryle étant éventuellement substitué et un motif -CH₂- étant éventuellement remplacé par -C(O)-NR¹⁰-, NR¹⁰-CO, NR¹⁰ ou O ;
R¹⁰ représente H, un reste alkyle en C₁ à C₆, halogénalkyle en C₁ à C₆, phényle éventuellement substitué, benzyle éventuellement substitué.

4. Composé suivant une ou plusieurs des revendications 1 à 3, dans lequel 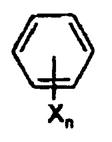 représente

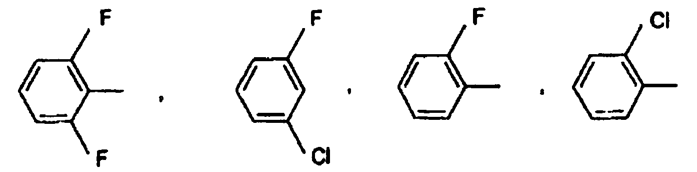

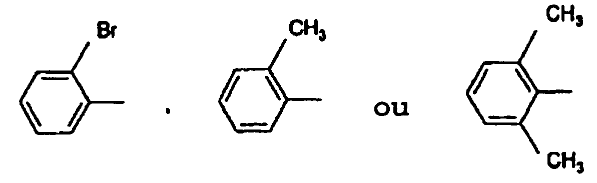

5. Composés suivant une ou plusieurs des revendications 1 à 4, du groupe des composés (I1) à (I28) : 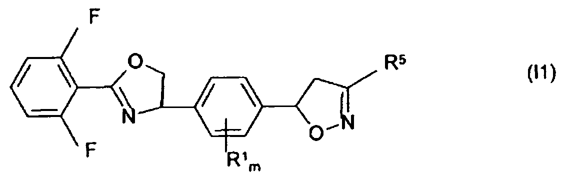 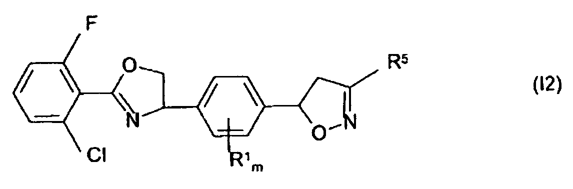 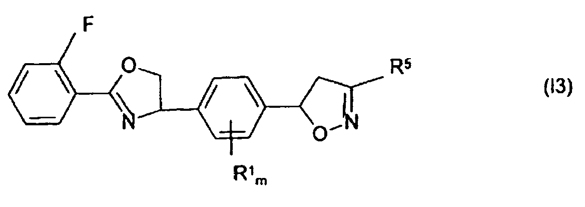 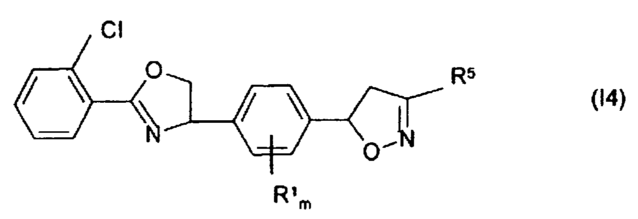 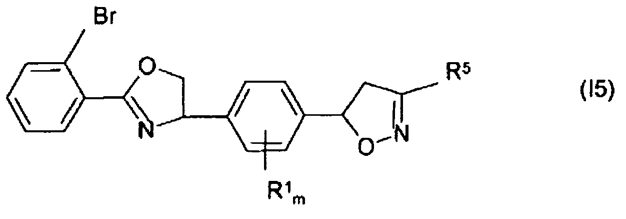 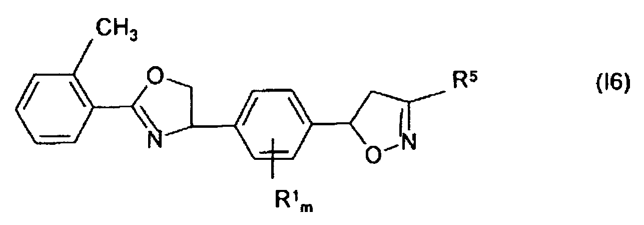 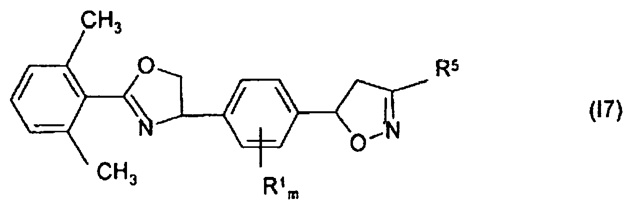 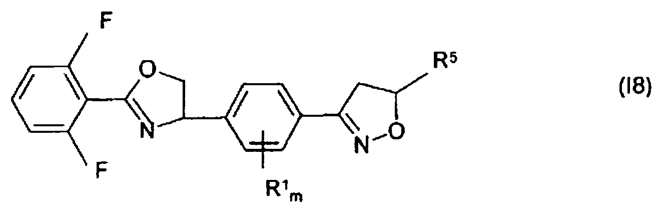 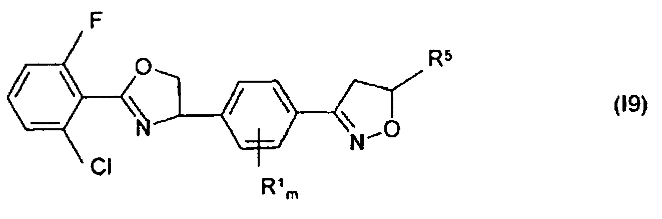 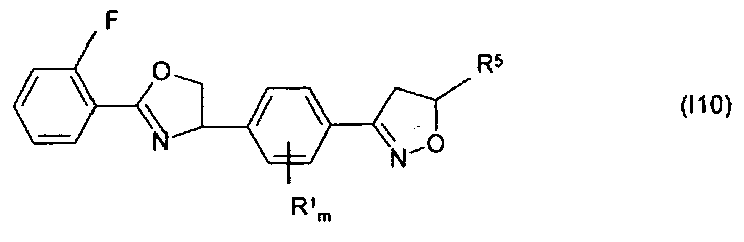 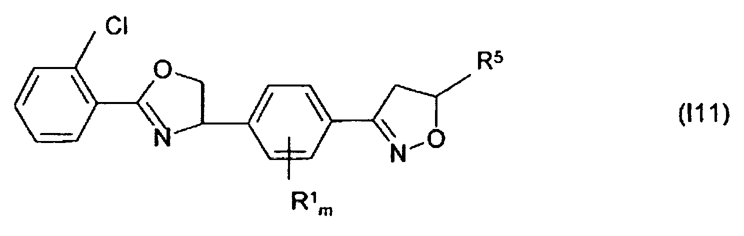 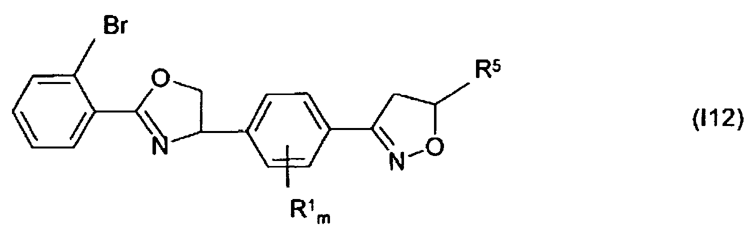 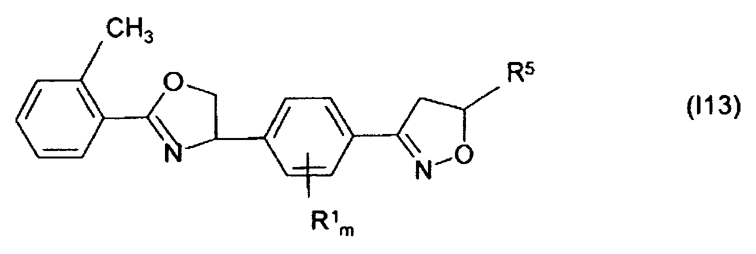 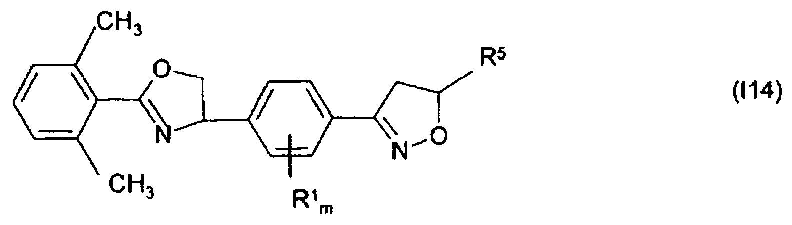 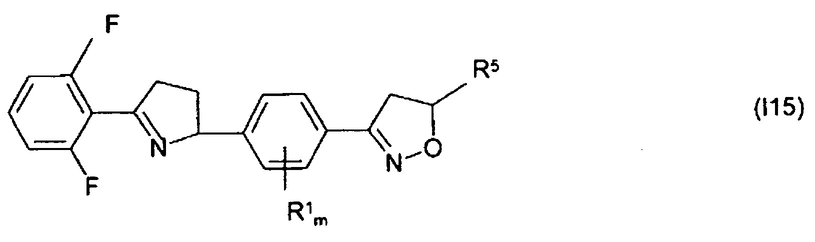 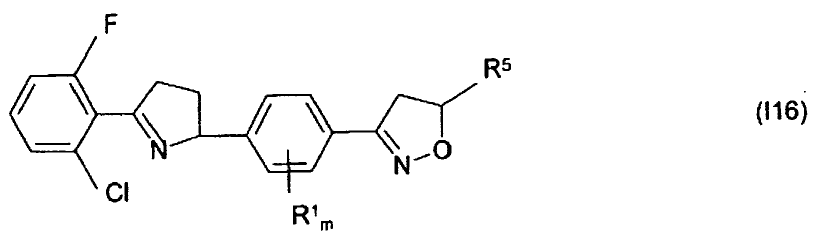 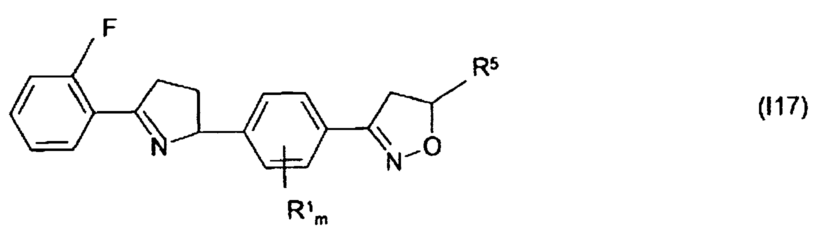 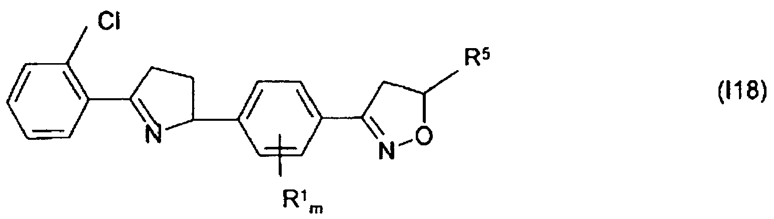 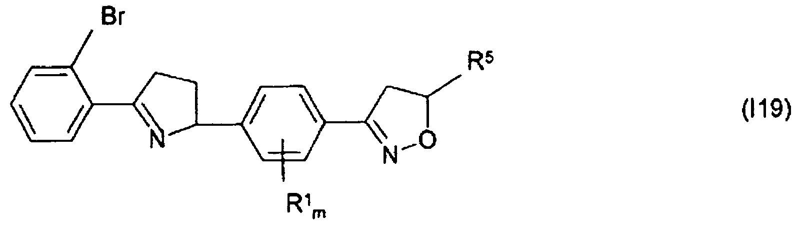 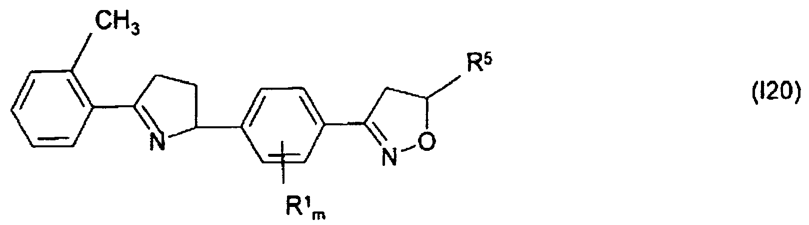 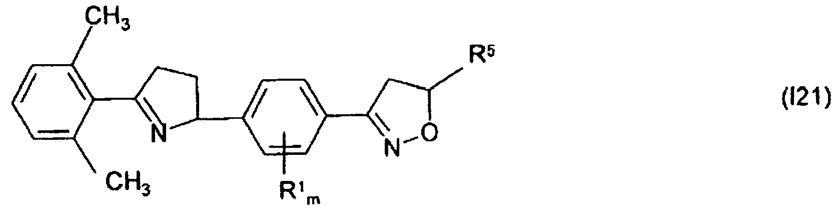 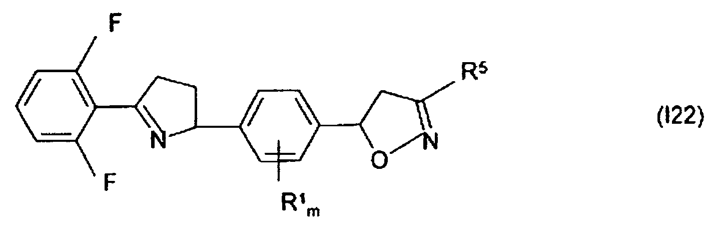 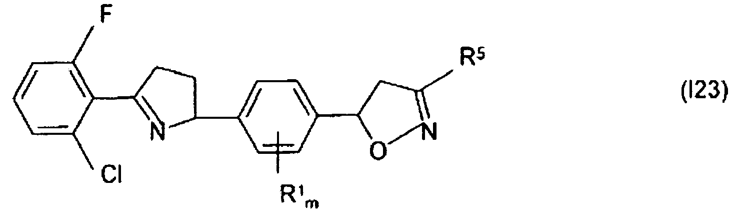 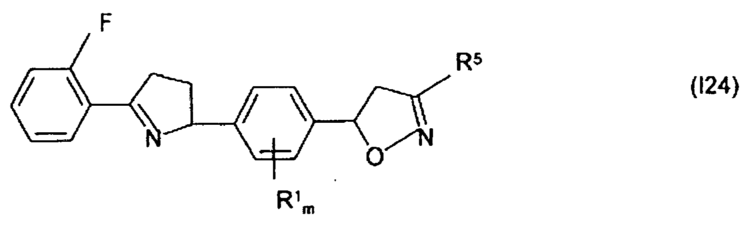 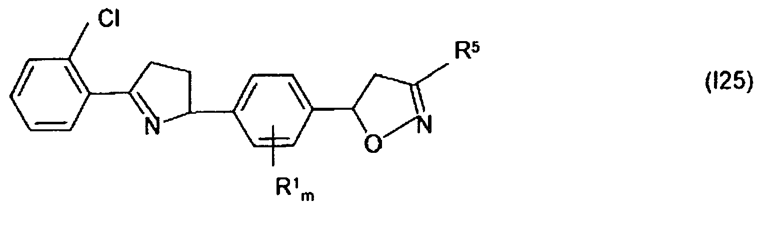 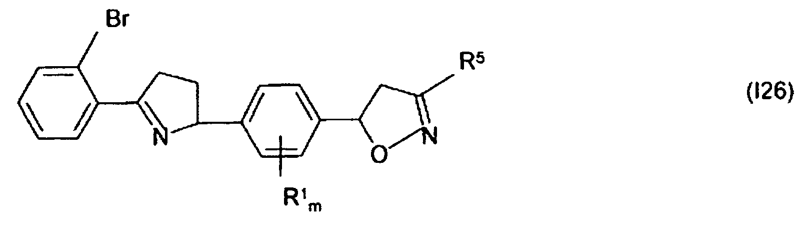 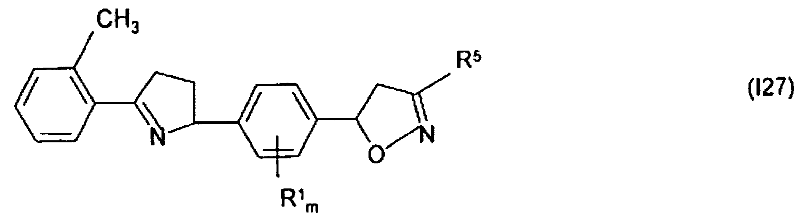 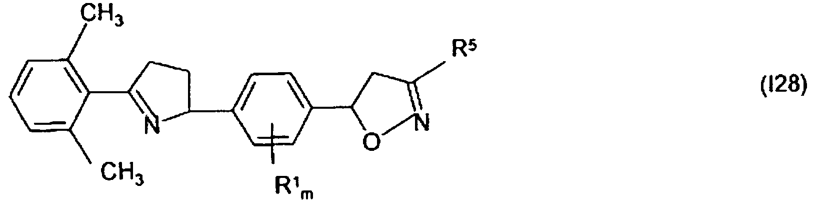 où R¹ₘ et R⁵ ont les définitions indiquées dans la revendication 2.

6. Procédé de production de composés de formule (I) suivant une ou plusieurs des revendications 1 à 5, dans lequel
a) pour produire des composés portant un reste 3-isoxazolinyle, on fait réagir une oxime de formule (II) 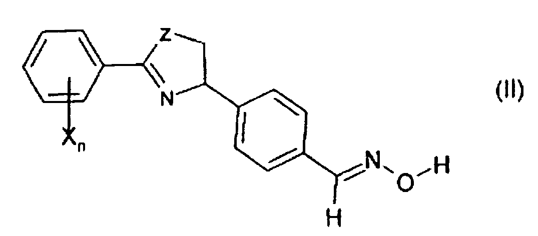 dans laquelle
X et Z ont les définitions indiquées pour la formule (I), avec un agent d'halogénation pour obtenir un composé de formule (III) 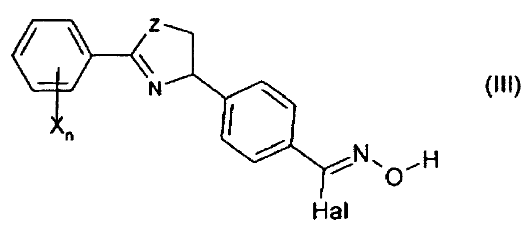 dans laquelle
Hal représente un halogène,
qu'on fait ensuite réagir avec une oléfine de formule (IV) 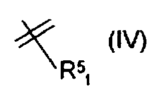 dans laquelle R⁵ et t ont les définitions indiquées dans la revendication 1 ;
ou bien
b) pour produire des composés portant un reste 5-isoxazinyle, on fait réagir une oléfine de formule (VII) 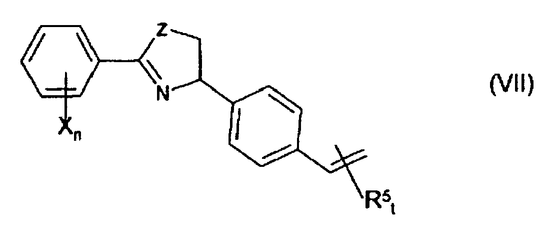 dans laquelle
Z et R⁵ₜ ont les définitions indiquées dans la revendication 2, avec une halogénoxime de formule (VIII) 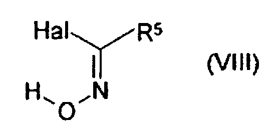 dans laquelle R⁵ a les définitions indiquées dans la revendication 2.

7. Composition pesticide, contenant au moins un composé suivant l'une des revendications 1 à 5 et au moins un auxiliaire de formulation.

8. Composition insecticide, acaricide et/ou nématicide suivant la revendication 7, contenant une quantité efficace d'au moins un composé suivant une ou plusieurs des revendications 1 à 5 conjointement avec des additifs ou substances auxiliaires d'emploi courant pour cette application.

9. Composition pesticide, contenant une quantité à effet insecticide, acaricide et/ou nématicide d'au moins un composé suivant une ou plusieurs des revendications 1 à 5 et au moins une autre substance active conjointement avec des substances auxiliaires et des additifs d'emploi courant pour cette application.

10. Composition destinée à être utilisée dans la protection du bois et comme conservateur dans des matières d'étanchéité, dans des peintures, dans des lubrifiants réfrigérants pour l'usinage des métaux ou dans des huiles d'alésage et de coupe, contenant une quantité efficace d'au moins un composé suivant l'une des revendications 1 à 5 conjointement avec des substances auxiliaires et des additifs d'emploi courant pour ces applications.

11. Utilisation d'un composé suivant une ou plusieurs des revendications 1 à 5 ou d'une composition suivant la revendication 7, 8 ou 9 pour la préparation d'un médicament à usage vétérinaire.

12. Procédé de préparation d'une composition suivant une ou plusieurs des revendications 7 à 11, dans lequel on réunit la substance active et les autres additifs et on leur fait prendre la forme d'utilisation qui convient.

13. Utilisation d'un composé suivant une ou plusieurs des revendications 1 à 5 ou d'une composition suivant une ou plusieurs des revendications 7, 8 et 9 comme moyen de protection du bois, comme conservateur dans des matières d'étanchéité, dans des peintures, dans des lubrifiants réfrigérants pour l'usinage des métaux et/ou dans des huiles d'alésage et de coupe.

14. Utilisation de composés suivant une ou plusieurs des revendications 1 à 5 ou d'une composition suivant une ou plusieurs des revendications 7, 8, 9 ou 10 pour combattre des insectes parasites, des acariens, des mollusques et des nématodes.

15. Procédé pour combattre des insectes parasites, des acariens, des mollusques et/ou des nématodes, dans lequel on met en contact les organismes mentionnés avec une quantité efficace d'un ou plusieurs composés suivant une ou plusieurs des revendications 1 à 5 ou d'une composition suivant une ou plusieurs des revendications 7, 8, 9 ou 10.

16. Procédé pour combattre des insectes parasites, des acariens, des mollusques et/ou des nématodes suivant la revendication 15, dans lequel on applique sur ces organismes ou sur les plantes, les aires ou les substrats qu'ils attaquent, une quantité efficace d'un composé suivant l'une des revendications 1 à 5 ou d'une composition suivant l'une des revendications 7, 8 ou 9.

17. Semences contenant une quantité efficace, ou enrobées d'une quantité efficace, d'un composé suivant l'une des revendications 1 à 5 ou d'une composition suivant l'une des revendications 7, 8 ou 9.
